# EUROPEAN PATENT APPLICATION

(11) **EP 4 847 088 A2**
(43) Date of publication of application: **02.12.2026**
(21) Application number: 26196626.1
(22) Date of filing: 21.12.2017
(51) Int. Cl.: A61P 19/02

(54) **ANTIBODIES THAT SPECIFICALLY BIND TO HUMAN IL-15 AND USES THEREOF**

(30) Priority: 21.12.2016 US 201662437143 P
(62) Document number(s) of the earlier application(s) in accordance with Art. 76 EPC: 25165676.5 / 4 585 261; 17883800.9 / 3 558 369
(71) Applicant: Cephalon LLC, West Chester, PA 19380 (US)
(72) Inventor: LAINE, David Jose Simon, Macquarie Park 2113 (AU); POLLARD, Matthew, Pullenvale 4069 (AU); DOYLE, Anthony Gerard, Macquarie Park 2113 (AU); POULTON, Lynn Dorothy, Macquarie Park 2113 (AU); CLARKE, Adam William, Macquarie Park 2113 (AU)
(74) Representative: Carpmaels & Ransford LLP

(57) **Abstract**

Recombinant antibodies that specifically bind to IL-15 as well as a complex of IL-15 and the IL-15 Receptor-alpha are provided. The antibodies inhibit immune cell proliferation, and are capable of use in the treatment of any autoimmune or inflammatory disease or condition where IL-15 is dysregulated, including Celiac disease.

## Description

### REFERENCE TO A SEQUENCE LISTING

This application includes a Sequence Listing entitled 2873.273PC01_SequenceListing_ST25.text, generated on December 18, 2017 with a size of 215,523 bytes. The Sequence Listing is incorporated by reference.

### FIELD

The disclosure relates generally to the field of recombinant antibody production. More particularly, the disclosure relates to recombinant antibodies that specifically bind to human IL-15, whether uncomplexed or in a complex with the IL-15 Receptor-alpha.

### BACKGROUND

Various references, including patents, published patent applications, technical articles, sequence accession numbers, and other references are cited throughout the specification. Each such reference is incorporated by reference herein, in its entirety and for all purposes.

The cytokine interleukin 15 (IL-15) is a member of IL-2 superfamily, which is secreted by a large number of cell types and tissues, including monocytes, macrophages, dendritic cells (DC), keratinocytes, fibroblasts and nerve cells. IL-15 binds to and signals through a complex composed of IL-2 receptor beta chain (CD122) and the common gamma chain (gamma-C, CD132). *In vitro,* IL-15 shares several biological activities with IL-2. *In vivo,* the specificity for IL-15 versus IL-2 is provided by unique private α-chain receptor (IL-15Rα) that completes the IL-15Rα/IL-2Rβγ heterotrimeric high-affinity receptor complex.

IL-15 has been isolated from synovial tissues from rheumatoid arthritis patients and reported to induce inflammatory cytokines and chemokines such as tumor-necrosis factor-α, IL-1β (Waldman TA (2004) Arthritis Res. Ther. 6:174-177). Blockade of IL-15 activity in a xenograft mouse model of human psoriasis resulted in a resolution of psoriasis (Villadsen LS et al. (2003) J. Clin. Invest. 112:1571-80). Increased levels of IL-15 complex in patients with T cell large granular lymphocytic leukemia, γΔ/Δ T cell lymphoma were reported (Chen J et al. (2012) Blood. 119:137-143).

Using mice deficient in IL-15, researchers have also shown that inhibiting the IL-15 signalling pathway can provide prophylactic or therapeutic benefit in several immune-mediated conditions, such as, experimental autoimmune encephalomyelitis (EAE; a model of multiple sclerosis), colitis, inflammatory bowel disease, psoriasis and arthritis.

In mouse models, overexpression of IL-15 in intestinal epithelia cells triggers a celiac-like enteropathy. In humans, the upregulation of IL-15 expression is a hallmark of celiac disease. IL-15 is overexpressed in both the lamina propria and intestinal epithelium of patients with active untreated celiac disease compared with healthy controls and gluten free diet treated celiac patients and IL-15 levels in the gut correlate with the degree of mucosal damage (Abadie V et al. (2014) Immunol. Rev. 260:221-234).

DISC0280 is a potent anti-IL-15 antibody with opposing mechanisms of action *in vitro* and *in vivo.* (Finch DK et al. (2011) Br. J. Pharmacol. 162:480-490). Disadvantageously, it was found that DISC0280 bound to the IL-15 receptor α binding site on IL-15 which allowed trans-presentation of IL-15 by the DISC0280 *in vivo,* similar to the trans-presentation by soluble IL-15 receptor α. Thus, DISC0280 acts as an agonist of IL-15 *in vivo.*

Two anti-IL-15 antibodies have been described as being able to neutralize the activity of IL-15 without competing with the binding of IL-15 to IL-15Rα. A fully human monoclonal anti-IL-15 antibody, AMG 714 (Amgen) showed improvements in disease activity in a phase I-II dose-escalation trial in patients with active rheumatoid arthritis (Baslund B et al. (2005) Arthritis Rheum. 52:2686-2692). A humanized antibody named huB-E29 has also been described to block the IL-15 activity *in vitro* and *in vivo* in a mouse model without competing with the binding of IL-15 to IL-15Rα (WO 16/001275).

Clinical trials examining new therapies for the treatment of celiac disease have as their endpoints: a) Attenuation of gluten-induced small intestinal mucosal injury as measured by the V/C ratio. The V/C is the morphometric measure of the length of the small intestinal villi with respect to the depth of the crypts taken from an intestinal biopsy sample. (b) Attenuation of gluten-induced small intestinal mucosal inflammation as measured by the enumeration of intraepithelial lymphocytes (IELs) in histological sections. (c) Attenuation of gluten-induced serum antibodies such as anti-gliadin antibodies and autoantibodies against transglutaminase. Currently no therapeutic has been shown to be efficacious in treating celiac disease as measured by the aforementioned endpoints. This disclosure features antibodies that attenuate gluten-induced small intestinal mucosal injury (improved V/C ratio), attenuate gluten-induced small intestinal mucosal inflammation (reduced IEL counts) and attenuate gluten-induced serum antibodies (reduced anti-gliadin antibodies) as measured in a rhesus macaque model of celiac disease. Antibodies of this disclosure present a new treatment for patients with celiac disease and other inflammatory diseases in which IL-15 is involved.

### SUMMARY

In a first aspect, the disclosure features antibodies comprising a variable heavy chain and a variable light chain, which antibodies specifically binds to an epitope comprising the Gln 108 residue of human IL-15 (e.g., wherein the IL-15 is complexed with IL-15Rα). In some embodiments, human IL-15 comprises the amino acid sequence of SEQ ID NO: 511. In some embodiments, the epitope may further comprise the Ser 7 and Asn 112 residues of human IL-15 (e.g., wherein the IL-15 is complexed with IL-15Rα). In some embodiments, the antibody preferably has an affinity for the epitope comprising a KD of less than about 1.8 x 10⁻⁹ M as determined by surface plasmon resonance. In some embodiments, the KD may be less than about 1.0 x 10⁻⁹ M. In some embodiments, the antibody preferably has an affinity for the epitope comprising a KD of less than about 2 x 10⁻¹⁰ M as determined by surface plasmon resonance. In some embodiments, the KD may be from about 1.6 x 10⁻¹⁰ M to about 1.8 x 10⁻¹⁰ M as determined by surface plasmon resonance. In some embodiments, the antibodies may inhibit proliferation of Natural Killer (NK) cells, *e.g*., NK-92 cells, at an IC₅₀ of less than about 900 pM in an NK cell proliferation assay, including from about 0.1 pM to about 900 pM. In some embodiments, the antibodies may inhibit proliferation of NK cells at an IC₅₀ of from about 1 pM to about 60 pM in an NK cell proliferation assay. In some embodiments, the antibodies may inhibit proliferation of NK cells at an IC₅₀ of from about 5 pM to about 35 pM in an NK cell proliferation assay. The antibodies may inhibit proliferation of NK cells at an IC₅₀ of from about 5 pM to about 25 pM in an NK cell proliferation assay. The antibodies can be capable of neutralizing IL-15. The antibodies can be capable of decreasing circulating NK cells.

In another aspect, the disclosure features antibodies that specifically bind to human IL-15, and that comprise an HCDR1 comprising the amino acid sequence of SEQ ID NO: 16, an HCDR2 comprising the amino acid sequence of SEQ ID NO: 17, an HCDR3 comprising the amino acid sequence of SEQ ID NO: 20, an LCDR1 comprising the amino acid sequence of SEQ ID NO: 25, an LCDR2 comprising the amino acid sequence of SEQ ID NO: 28, and an LCDR3 comprising the amino acid sequence of SEQ ID NO: 29. The human IL-15 may be complexed with the IL-15 Receptor-alpha. The antibodies are IL-15 antagonists. Polynucleotides encoding such antibodies are further provided.

In some embodiments, the antibodies (e.g, antibodies comprising HCDR1, HCDR2, HCDR3, LCDR1, LCDR2, and LCDR3 comprising the amino acid sequences of SEQ ID NOs: 16, 17, 20, 25, 28, and 29, respectively) may comprise an HCDR2 comprising the amino acid sequence of SEQ ID NO: 18. In some embodiments, the antibodies (e.g, antibodies comprising HCDR1, HCDR2, HCDR3, LCDR1, LCDR2, and LCDR3 comprising the amino acid sequences of SEQ ID NOs: 16, 17 or 18, 20, 25, 28, and 29, respectively) may comprise an LCDR1 comprising the amino acid sequence of SEQ ID NO: 27 and an LCDR3 comprising the amino acid sequence of SEQ ID NO: 31. In some embodiments, the antibodies may comprise a heavy chain variable region comprising the amino acid sequence of SEQ ID NO: 454 and a light chain variable region comprising the amino acid sequence of SEQ ID NO: 8, and/or may comprise a light chain comprising the amino acid sequence of SEQ ID NO: 9. In some embodiments, the antibodies (e.g, antibodies comprising HCDR1, HCDR2, HCDR3, LCDR1, LCDR2, and LCDR3 comprising the amino acid sequences of SEQ ID NOs: 16, 17 or 18, 20, 25, 28, and 29, respectively) may comprise a heavy chain FR3 comprising the amino acid sequence of SEQ ID NO: 12, or SEQ ID NO :13. Polynucleotides encoding such antibodies are further provided.

In some embodiments, the antibodies (e.g, antibodies comprising HCDR1, HCDR2, HCDR3, LCDR1, LCDR2, and LCDR3 comprising the amino acid sequences of SEQ ID NOs: 16, 17 or 18, 20, 25, 28, and 29, respectively) may comprise an LCDR1 comprising the amino acid sequence of SEQ ID NO: 26 and an LCDR3 comprising the amino acid sequence of SEQ ID NO: 31.

In some embodiments, the antibodies (e.g, antibodies comprising HCDR1, HCDR2, HCDR3, LCDR1, LCDR2, and LCDR3 comprising the amino acid sequences of SEQ ID NOs: 16, 17 or 18, 20, 25 or 26, 28, and 29 or 31, respectively) may comprise a heavy chain variable region comprising the amino acid sequence of SEQ ID NO: 4 and a light chain variable region comprising the amino acid sequence of SEQ ID NO: 455, and/or may comprise a light chain comprising the amino acid sequence of SEQ ID NO: 456. Polynucleotides encoding such antibodies are further provided.

In some embodiments, the antibodies (e.g, antibodies comprising HCDR1, HCDR2, HCDR3, LCDR1, LCDR2, and LCDR3 comprising the amino acid sequences of SEQ ID NOs: 16, 17 or 18, 20, 25, 28, and 29, respectively) may comprise an LCDR1 comprising the amino acid sequence of SEQ ID NO: 27 and an LCDR3 comprising the amino acid sequence of SEQ ID NO: 30. In some embodiments, the antibodies (e.g, antibodies comprising HCDR1, HCDR2, HCDR3, LCDR1, LCDR2, and LCDR3 comprising the amino acid sequences of SEQ ID NOs: 16, 17 or 18, 20, 27, 28, and 30, respectively) may comprise a heavy chain variable region comprising the amino acid sequence of SEQ ID NO: 4 and a light chain variable region comprising the amino acid sequence of SEQ ID NO: 457, and/or may comprise a light chain comprising the amino acid sequence of SEQ ID NO: 458. Polynucleotides encoding such antibodies are further provided.

In some embodiments, the antibodies (e.g, antibodies comprising HCDR1, HCDR2, HCDR3, LCDR1, LCDR2, and LCDR3 comprising the amino acid sequences of SEQ ID NOs: 16, 17 or 18, 20, 25, 28, and 29, respectively) may comprise an LCDR1 comprising the amino acid sequence of SEQ ID NO: 27, and an LCDR3 comprising the amino acid sequence of SEQ ID NO: 29, wherein Xaa5 of SEQ ID NO: 29 is Phe (e.g., SEQ ID NO: 519). In some embodiments, the antibodies (e.g, antibodies comprising HCDR1, HCDR2, HCDR3, LCDR1, LCDR2, and LCDR3 comprising the amino acid sequences of SEQ ID NOs: 16, 17 or 18, 20, 27, 28, and 519, respectively) may comprise a heavy chain variable region comprising the amino acid sequence of SEQ ID NO: 4 and a light chain variable region comprising the amino acid sequence of SEQ ID NO: 459, and/or may comprise a light chain comprising the amino acid sequence of SEQ ID NO: 460. Polynucleotides encoding such antibodies are further provided.

In some embodiments, the antibodies (e.g, antibodies comprising HCDR1, HCDR2, HCDR3, LCDR1, LCDR2, and LCDR3 comprising the amino acid sequences of SEQ ID NOs: 16, 17 or 18, 20, 25, 28, and 29, respectively, and optionally an HFR3 comprising the amino acid sequence of SEQ ID NO:12 or 13) may comprise an LCDR1 comprising the amino acid sequence of SEQ ID NO: 26 and an LCDR3 comprising the amino acid sequence of SEQ ID NO: 30. In some embodiments, (e.g, antibodies comprising HCDR1, HCDR2, HCDR3, LCDR1, LCDR2, and LCDR3 comprising the amino acid sequences of SEQ ID NOs: 16, 17 or 18, 20, 26, 28, and 30, respectively) the antibodies may comprise an HCDR2 comprising the amino acid sequence of SEQ ID NO: 18, and a heavy chain FR3 comprising the amino acid sequence of SEQ ID NO: 13. In some embodiments, the antibodies (e.g, antibodies comprising HCDR1, HCDR2, HCDR3, LCDR1, LCDR2, and LCDR3 comprising the amino acid sequences of SEQ ID NOs: 16, 17 or 18, 20, 26, 28, and 30, respectively, and optionally an HFR3 comprising the amino acid sequence of SEQ ID NO:12 or 13) may comprise a heavy chain variable region comprising the amino acid sequence of SEQ ID NO: 4. In some embodiments, the antibodies (e.g., antibodies comprising HCDR1, HCDR2, HCDR3, LCDR1, LCDR2, and LCDR3 comprising the amino acid sequences of SEQ ID NOs: 16, 17 or 18, 20, 26, 28, and 30, respectively, and optionally an HFR3 comprising the amino acid sequence of SEQ ID NO:12 or 13 or antibodies comprising a heavy chain variable region comprising the amino acid sequence of SEQ ID NO:4) may comprise a light chain variable region comprising the amino acid sequence of SEQ ID NO: 5, and/or may comprise a light chain comprising the amino acid sequence of SEQ ID NO: 6. Polynucleotides encoding such antibodies are further provided.

In some embodiments, the antibodies (e.g., antibodies comprising HCDR1, HCDR2, HCDR3, LCDR1, LCDR2, and LCDR3 comprising the amino acid sequences of SEQ ID NOs: 16, 17 20, 25, 28, and 29, respectively, and optionally an HFR3 comprising the amino acid sequence of SEQ ID NO:12) may comprise an HCDR2 comprising the amino acid sequence of SEQ ID NO: 19, an LCDR1 comprising the amino acid sequence of SEQ ID NO: 27, an LCDR3 comprising the amino acid sequence of SEQ ID NO: 31, and a heavy chain FR3 comprising the amino acid sequence of SEQ ID NO: 14. In some embodiments, the antibodies (e.g., antibodies comprising HCR1, HCDR2, HCDR3, LCDR1, LCDR2, and LCDR3 comprising the amino acid sequences of SEQ ID NOs: 16, 19, 20, 27, 28, and 31, and an HFR3 comprising the amino acid sequence of SEQ ID NO:14) may comprise a heavy chain variable region comprising the amino acid sequence of SEQ ID NO: 7 and a light chain variable region comprising the amino acid sequence of SEQ ID NO: 8, and/or may comprise a light chain comprising the amino acid sequence of SEQ ID NO: 9. Polynucleotides encoding such antibodies are further provided.

In another aspect, the disclosure features antibodies that specifically bind to human IL-15 (e.g., IL-15 complexed with IL-15Rα), and that comprise a heavy chain variable region comprising the amino acid sequence of SEQ ID NO: 1 and a light chain variable region comprising the amino acid sequence of SEQ ID NO: 2. In some embodiments, the antibodies may comprise a heavy chain variable region comprising the amino acid sequence of SEQ ID NO: 4 and a light chain variable region comprising the amino acid sequence of SEQ ID NO: 5. In some embodiments, the antibodies may comprise a heavy chain variable region comprising the amino acid sequence of SEQ ID NO: 454 and a light chain variable region comprising the amino acid sequence of SEQ ID NO: 8. In some embodiments, the antibodies may comprise a heavy chain variable region comprising the amino acid sequence of SEQ ID NO: 4 and a light chain variable region comprising the amino acid sequence of SEQ ID NO: 455. In some embodiments, the antibodies may comprise a heavy chain variable region comprising the amino acid sequence of SEQ ID NO: 4 and a light chain variable region comprising the amino acid sequence of SEQ ID NO: 457. In some embodiments, the antibodies may comprise a heavy chain variable region comprising the amino acid sequence of SEQ ID NO: 4 and a light chain variable region comprising the amino acid sequence of SEQ ID NO: 459. Polynucleotides encoding such antibodies are further provided.

In another aspect, the disclosure features antibodies that specifically bind to human IL-15 (e.g., IL-15 complexed with IL-15Rα), and that comprises a heavy chain variable region comprising the amino acid sequence of SEQ ID NO:4 and a light chain variable region comprising the amino acid sequence of SEQ ID NO:8; a heavy chain variable region comprising the amino acid sequence of SEQ ID NO:4 and a light chain variable region comprising the amino acid sequence of SEQ ID NO:503; a heavy chain variable region comprising the amino acid sequence of SEQ ID NO:4 and a light chain variable region comprising the amino acid sequence of SEQ ID NO:505; a heavy chain variable region comprising the amino acid sequence of SEQ ID NO:4 and a light chain variable region comprising the amino acid sequence of SEQ ID NO:507; a heavy chain variable region comprising the amino acid sequence of SEQ ID NO:4 and a light chain variable region comprising the amino acid sequence of SEQ ID NO:509; a heavy chain variable region comprising the amino acid sequence of SEQ ID NO:4 and a light chain variable region comprising the amino acid sequence of SEQ ID NO:510; a heavy chain variable region comprising the amino acid sequence of SEQ ID NO:454 and a light chain variable region comprising the amino acid sequence of SEQ ID NO:455; a heavy chain variable region comprising the amino acid sequence of SEQ ID NO:454 and a light chain variable region comprising the amino acid sequence of SEQ ID NO:503; a heavy chain variable region comprising the amino acid sequence of SEQ ID NO:454 and a light chain variable region comprising the amino acid sequence of SEQ ID NO:457; a heavy chain variable region comprising the amino acid sequence of SEQ ID NO:454 and a light chain variable region comprising the amino acid sequence of SEQ ID NO:505; a heavy chain variable region comprising the amino acid sequence of SEQ ID NO:454 and a light chain variable region comprising the amino acid sequence of SEQ ID NO:506; a heavy chain variable region comprising the amino acid sequence of SEQ ID NO:454 and a light chain variable region comprising the amino acid sequence of SEQ ID NO:507; a heavy chain variable region comprising the amino acid sequence of SEQ ID NO:454 and a light chain variable region comprising the amino acid sequence of SEQ ID NO:5; a heavy chain variable region comprising the amino acid sequence of SEQ ID NO:454 and a light chain variable region comprising the amino acid sequence of SEQ ID NO:509; or a heavy chain variable region comprising the amino acid sequence of SEQ ID NO:454 and a light chain variable region comprising the amino acid sequence of SEQ ID NO:510. Polynucleotides encoding such antibodies are further provided.

The consensus sequence for the antibody VH is SEQ ID NO: 1, and encompasses the VH sequence of SEQ ID NO: 4 and SEQ ID NO: 454. The consensus sequence for the antibody VL is SEQ ID NO: 2, and encompasses the VL sequence of SEQ ID NO: 5, SEQ ID NO: 8, SEQ ID NO: 455, SEQ ID NO: 457, and SEQ ID NO: 459. The consensus sequence for the antibody L chain is SEQ ID NO: 3, and encompasses the L chain sequence of SEQ ID NO: 6, SEQ ID NO: 9, SEQ ID NO: 456, SEQ ID NO: 458, and SEQ ID NO: 460. The consensus sequence for the antibody VH FR3 is SEQ ID NO: 12, and encompasses the VH FR3 sequence of SEQ ID NO: 13 and SEQ ID NO: 14. The consensus sequence for the antibody VH CDR1 is SEQ ID NO: 17, and encompasses the VH sequence of SEQ ID NO: 18 and SEQ ID NO: 19. The consensus sequence for the antibody VL CDR1 is SEQ ID NO: 25, and encompasses the VH sequence of SEQ ID NO: 26 and SEQ ID NO: 27. The consensus sequence for the antibody VL CDR3 is SEQ ID NO: 29, and encompasses the VH sequence of SEQ ID NO: 30 and SEQ ID NO: 31 and SEQ ID NO:519.

Any of the antibodies that bind to IL-15 (e.g., human IL-15 complexed with IL-15Rα) as described or exemplified herein, including those of any of the preceding paragraphs, may comprise an IgG constant domain. In some embodiments, the IgG constant domain may comprise an IgG1 constant domain. In some embodiments, the IgG1 constant domain may comprise SEQ ID NO: 32, SEQ ID NO: 33, SEQ ID NO: 34, SEQ ID NO: 35, SEQ ID NO: 36, SEQ ID NO: 37, SEQ ID NO: 38 or SEQ ID NO: 39. In some embodiments, the IgG constant domain may comprise an IgG2 constant domain. In some embodiments, the IgG2 constant domain may comprise SEQ ID NO: 40, SEQ ID NO: 41, SEQ ID NO: 42 or SEQ ID NO: 43. In some embodiments, the IgG constant domain may comprise an IgG4 constant domain. In some embodiments, the IgG4 constant domain may comprise SEQ ID NO: 44, SEQ ID NO: 45, SEQ ID NO: 46, SEQ ID NO: 47, SEQ ID NO: 48, SEQ ID NO: 49, SEQ ID NO: 50 or SEQ ID NO: 51.

Any of the antibodies that bind to IL-15 (e.g., human IL-15 complexed with IL-15Rα) as described or exemplified herein, including those of any of the preceding paragraphs, may be formulated as a composition with a carrier or excipient. The carrier may comprise a pharmaceutically acceptable carrier.

In some embodiments, a method of treating Celiac disease comprises administering an antibody that binds to IL-15 (e.g., human IL-15 complexed with IL-15Rα) to a subject in need thereof. Administration of the IL-15 antibody can repair the mucosa of the small intestine in the subject. Administration of the IL-15 antibody can increase the mean villous height vs. crypt depths (V/C) ratio in the subject. Administration of the IL-15 antibody can increase the height of small intestinal villi in the subject. Administration of the IL-15 antibody can decrease anti-gliadin antibodies in the subject. Administration of the IL-15 antibody can repair gluten-induced small intestinal mucosal injury in a subject.

Any of the antibodies that bind to IL-15 (e.g., human IL-15 complexed with IL-15Rα) as described or exemplified herein, including those of any of the preceding paragraphs, may be administered as part of a treatment regimen to a subject in need thereof. Thus, in another aspect, the disclosure features methods for treating a subject in need thereof with an IL-15 antibody. The subject is preferably a human being. The antibodies may be administered as part of a treatment regimen to treat any autoimmune or inflammatory disease or condition where IL-15 is dysregulated, in particular, where IL-15 is upregulated.

In some detailed embodiments, the method may be for treating Celiac disease, and comprise administering to the subject any antibody as described or exemplified herein, including those of any of the preceding paragraphs, that binds to IL-15 (e.g. human IL-15 complexed with IL-15Rα), which antibody may be in a composition, which may include a pharmaceutically acceptable carrier or excipient. In some embodiments, a method for repairing the mucosa of a small intestine in a subject having gluten sensitivity, gluten allergy, or Celiac disease comprises administering to the subject any antibody as described or exemplified herein, including those of any of the preceding paragraphs, that binds to IL-15 (e.g. human IL-15 complexed with IL-15Rα), which antibody may be in a composition, which may include a pharmaceutically acceptable carrier or excipient. In some embodiments, a method for increasing the mean villous height vs. crypt depths (V/C) ratio in a subject having gluten sensitivity, gluten allergy, or Celiac disease comprises administering to the subject any antibody as described or exemplified herein, including those of any of the preceding paragraphs, that binds to IL-15 (e.g. human IL-15 complexed with IL-15Rα), which antibody may be in a composition, which may include a pharmaceutically acceptable carrier or excipient. In some embodiments, a method for increasing the height of small intestinal villi in a subject having gluten sensitivity, gluten allergy, or Celiac disease comprises administering to the subject any antibody as described or exemplified herein, including those of any of the preceding paragraphs, that binds to IL-15 (e.g. human IL-15 complexed with IL-15Rα), which antibody may be in a composition, which may include a pharmaceutically acceptable carrier or excipient. In some embodiments, a method for decreasing anti-gliadin antibodies in a subject in need thereof comprises administering to the subject any antibody as described or exemplified herein, including those of any of the preceding paragraphs, that binds to IL-15 (e.g. human IL-15 complexed with IL-15Rα), which antibody may be in a composition, which may include a pharmaceutically acceptable carrier or excipient. In some embodiments, a method for repairing gluten-induced small intestinal mucosal injury comprises administering to the subject any antibody as described or exemplified herein, including those of any of the preceding paragraphs, that binds to IL-15 (e.g. human IL-15 complexed with IL-15Rα), which antibody may be in a composition, which may include a pharmaceutically acceptable carrier or excipient. In some embodiments, the subject has gluten sensitivity, a gluten allergy, or Celiac disease. In some embodiments, the method may be for treating refractory Celiac disease, and comprise administering to the subject any antibody as described or exemplified herein, including those of any of the preceding paragraphs, that binds to IL-15 (e.g., human IL-15 complexed with IL-15Rα), which antibody may be in a composition, which may include a pharmaceutically acceptable carrier or excipient. In some embodiments, the method may be for treating rheumatoid arthritis, and comprise administering to the subject any antibody as described or exemplified herein, including those of any of the preceding paragraphs, that binds to IL-15 (e.g., human IL-15 complexed with IL-15Rα), which antibody may be in a composition, which may include a pharmaceutically acceptable carrier or excipient. In some embodiments, the method may be for treating psoriasis, and comprise administering to the subject any antibody as described or exemplified herein, including those of any of the preceding paragraphs, that binds to IL-15 (e.g., human IL-15 complexed with IL-15Rα), which antibody may be in a composition, which may include a pharmaceutically acceptable carrier or excipient. In some embodiments, the method may be for treating inflammatory bowel disease, and comprise administering to the subject any antibody as described or exemplified herein, including those of any of the preceding paragraphs, that binds to IL-15 (e.g., human IL-15 complexed with IL-15Rα), which antibody may be in a composition, which may include a pharmaceutically acceptable carrier or excipient. In some embodiments, the method may be for treating type 1 diabetes, and comprise administering to the subject any antibody as described or exemplified herein, including those of any of the preceding paragraphs, that binds to IL-15 (e.g., human IL-15 complexed with IL-15Rα), which antibody may be in a composition, which may include a pharmaceutically acceptable carrier or excipient. In some embodiments, the method may be for treating alopecia areata, and comprise administering to the subject any antibody as described or exemplified herein, including those of any of the preceding paragraphs, that binds to IL-15 (e.g., human IL-15 complexed with IL-15Rα), which antibody may be in a composition, which may include a pharmaceutically acceptable carrier or excipient. In some embodiments, the method may be for treating T cell large granular lymphocytic leukemia, and comprise administering to the subject any antibody as described or exemplified herein, including those of any of the preceding paragraphs, that binds to IL-15 (e.g., human IL-15 complexed with the IL-15 Receptor-alpha), which antibody may be in a composition, which may include a pharmaceutically acceptable carrier or excipient. In some embodiments, the method may be for treating or inhibiting symptoms of gluten exposure, for example, gluten exposure in a patient who has a gluten sensitivity or allergy, and comprise administering to the subject any antibody as described or exemplified herein, including those of any of the preceding paragraphs, that binds to IL-15 (e.g., human IL-15 complexed with IL-15Rα), which antibody may be in a composition, which may include a pharmaceutically acceptable carrier or excipient. The one or more symptoms of gluten exposure may include one or more of muscle pain, body pain, joint pain, fatigue, bloating, gas, nausea, cramps, constipation, diarrhea, skin rash, headache, migraine headache, depression, anxiety, brain fog, and/or irritability. See, Biesiekierski JR (2015) United European Gastroenterol. J. 3:160-165.

Any of the antibodies that bind to IL-15 (e.g., human IL-15 complexed with IL-15Rα) as described or exemplified herein, including those of any of the preceding paragraphs, may be used in the manufacture of a medicament. Any such antibodies may be used to treat any autoimmune or inflammatory disease or condition where IL-15 is dysregulated. In some embodiments, the antibodies may be used for treating Celiac disease or in the manufacture of a medicament for treating Celiac disease. The antibodies may be used for treating refractory Celiac disease or in the manufacture of a medicament for treating refractory Celiac disease. In some embodiments, the antibodies may be used for treating rheumatoid arthritis or in the manufacture of a medicament for treating rheumatoid arthritis. In some embodiments, the antibodies may be used for treating psoriasis or in the manufacture of a medicament for treating psoriasis. In some embodiments, the antibodies may be used for treating inflammatory bowel disease or in the manufacture of a medicament for treating inflammatory bowel disease. In some embodiments, the antibodies may be used for treating type 1 diabetes or in the manufacture of a medicament for treating type 1 diabetes. In some embodiments, the antibodies may be used for treating alopecia areata or in the manufacture of a medicament for treating alopecia areata. In some embodiments, the antibodies may be used for treating T cell large granular lymphocytic leukemia or in the manufacture of a medicament for treating T cell large granular lymphocytic leukemia.

Any of the antibodies that bind to IL-15 (e.g., human IL-15 complexed with IL-15Rα) as described or exemplified herein, including those of any of the preceding paragraphs, may be used in an *in vitro* method for detecting IL-15 (optionally complexed with IL-15Rα) in a tissue sample isolated from a subject, the method comprising contacting the antibody with a tissue sample isolated from a subject to form an antibody-IL-15 complex (optionally further complexed with the IL-15 Receptor-alpha), and detecting the complex in the tissue sample. Any of the antibodies that bind to IL-15 as described or exemplified herein, including those of any of the preceding paragraphs, may be used in an *in vitro* method for detecting the IL-15 complex with the IL-15 Receptor-alpha in a tissue sample isolated from a subject, comprising contacting the antibody with a tissue sample isolated from a subject to form an antibody-antigen complex of the antibody with IL-15 and IL-15 receptor α complex, and detecting the antibody-antigen complex in the tissue sample.

In another aspect, the disclosure further features transformed cells that express any of the antibodies that bind to IL-15 (e.g., human IL-15 complexed with IL-15Rα) as described or exemplified herein, including those of any of the preceding paragraphs. In some embodiments, the transformed cell may be a mammalian cell. In some embodiments, the mammalian cell may be a Chinese Hamster Ovary cell.

In another aspect, the disclosure further features polynucleotides that encode any of the antibodies that bind to IL-15 (e.g., human IL-15 complexed with IL-15Rα) as described or exemplified herein, including those of any of the preceding paragraphs. In some embodiments, a polynucleotide encoding an antibody heavy chain variable region comprises the nucleic acid sequence of SEQ ID NO: 517. In some embodiments, a polynucleotide encoding an antibody light chain variable region comprises the nucleic acid sequence of SEQ ID NO: 518. Vectors comprising these polynucleotides are also provided. Cells comprising these polynucleotides or vectors are also provided. Cells comprising a polynucleotide comprising a nucleic acid that encodes the variable heavy chain of an antibody that binds to IL-15(e.g., human IL-15 complexed with IL-15Rα) as described or exemplified herein, including those of any of the preceding paragraphs and a nucleic acid that encodes the variable light chain of the antibody are also provided. The nucleic acid that encodes the variable heavy chain and the nucleic acid that encodes the variable light chain can be on the same vector or on different vectors.

### BRIEF DESCRIPTION OF THE DRAWINGS

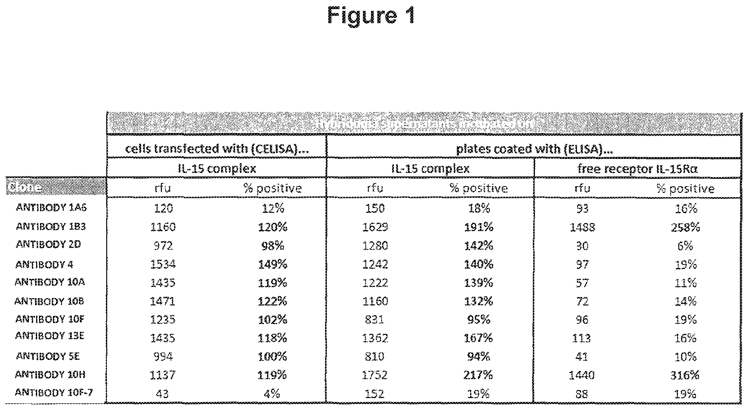

Figure 1 shows binding of anti-IL-15 antibodies to the human IL-15 complex with the IL-15 Receptor-alpha, or uncomplexed IL-15Rα. The binding of representative hybridoma supernatants to uncomplexed recombinant human IL-15Rα or recombinant IL-15 complex with the IL-15 Receptor-alpha was determined by cell ELISA (cELISA) and ELISA. Results are expressed at relative fluorescence units.

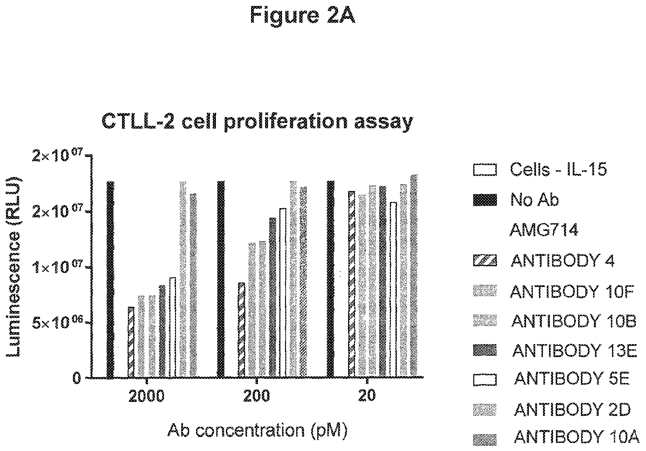

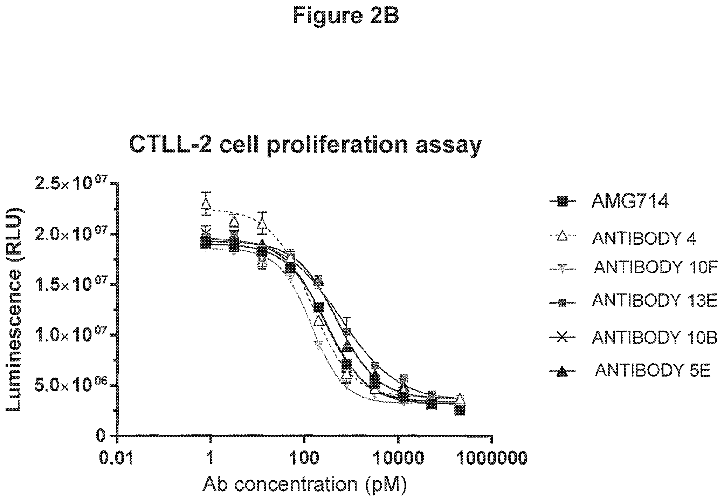

Figures 2A and 2B show dose response inhibition of IL-15 mediated CTLL-2 proliferation by anti-IL-15 antibodies. Figure 2A shows the inhibition of IL-15 mediated CTLL-2 proliferation by representative anti-IL-15 antibodies diluted at 2000, 200 and 20 pM, and Figure 2B shows inhibition in a full dose response for 72 hours. Results are expressed as relative luminescence units.

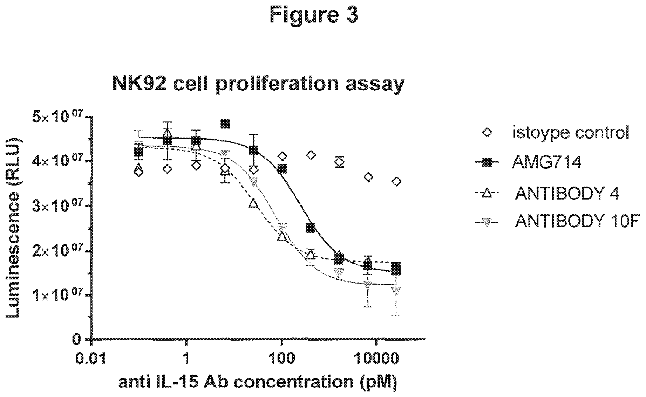

Figure 3 shows inhibition of IL-15 mediated NK-92 proliferation by representative anti-IL-15 antibodies, AMG714 or isotype control (anti-KLH C3 IgG1). The readout was taken at 72 hours and is expressed as relative luminescence units. Results are expressed as mean ± error 3 replicates.

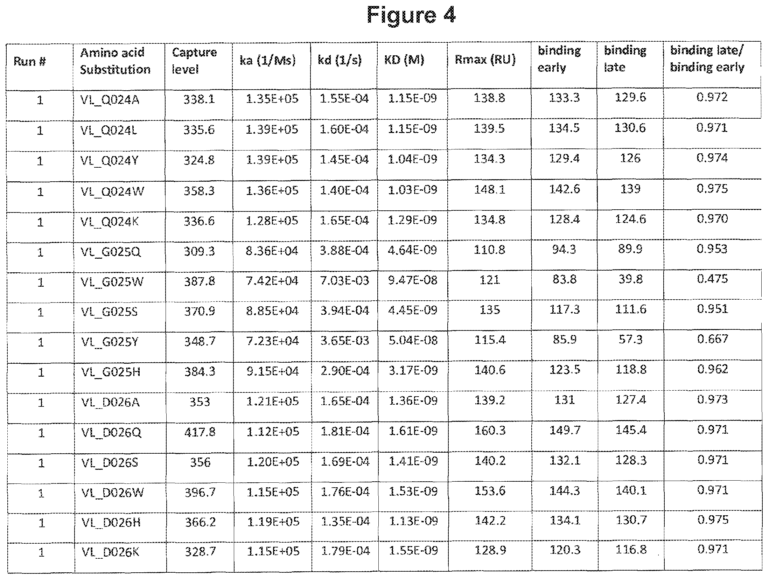

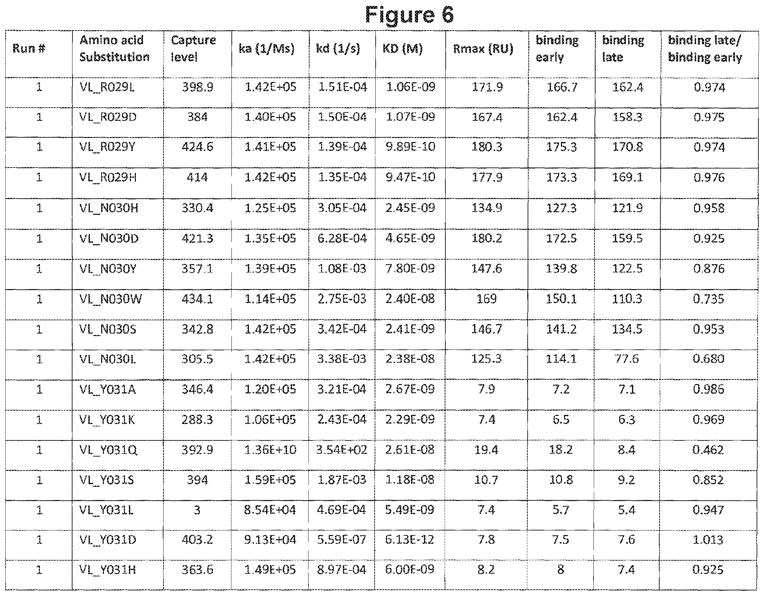

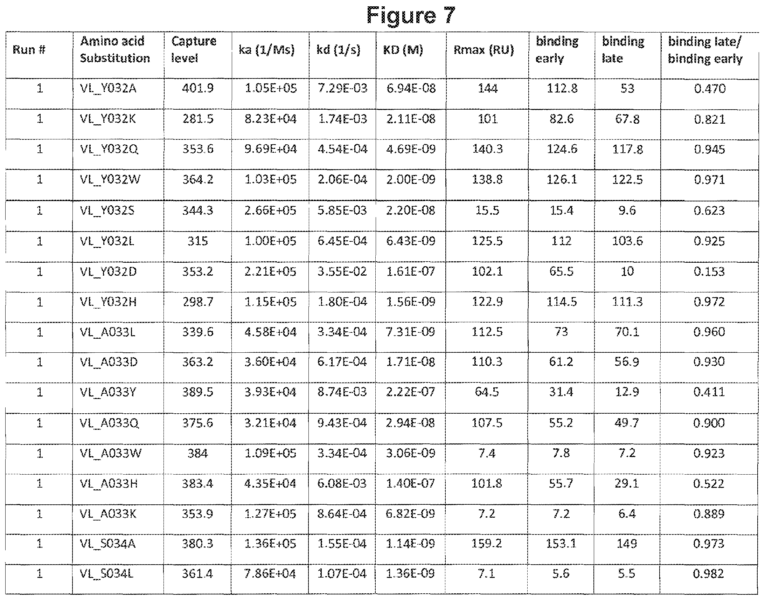

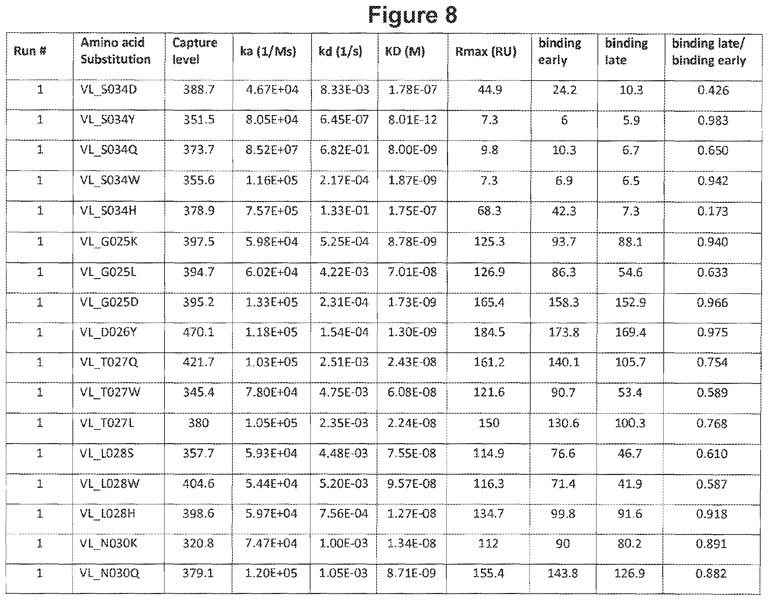

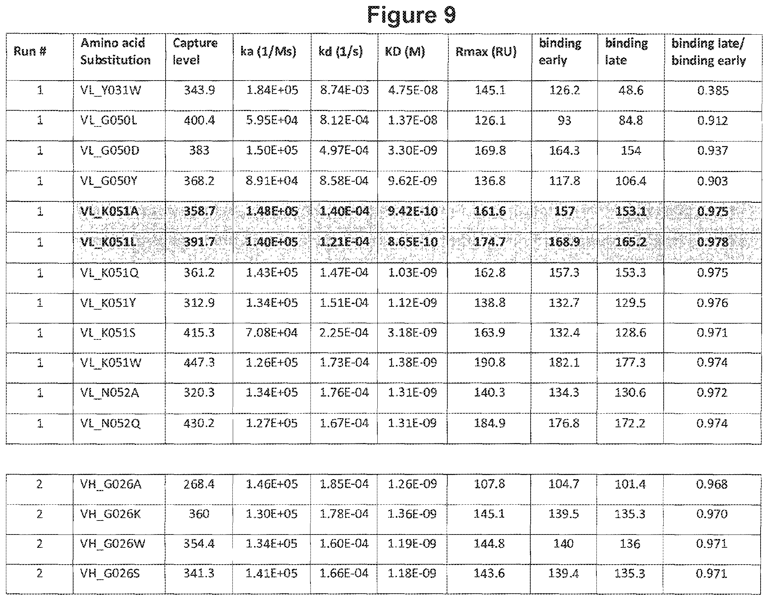

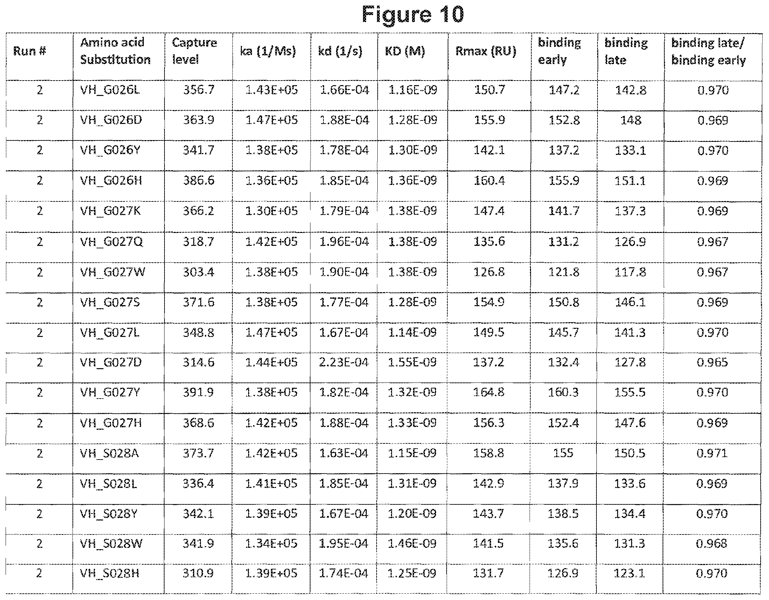

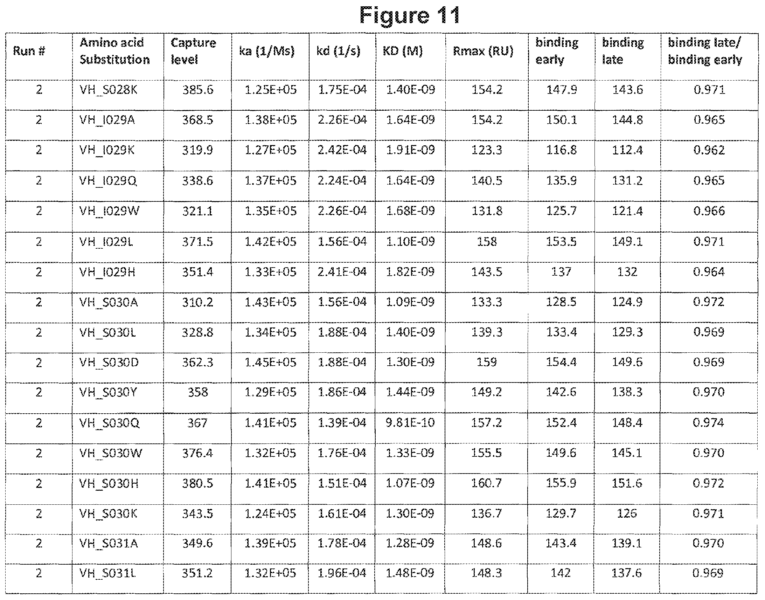

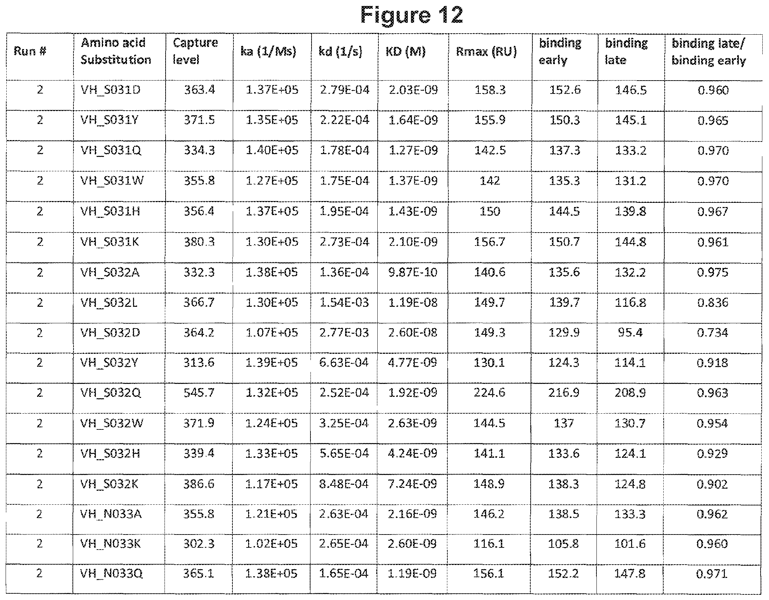

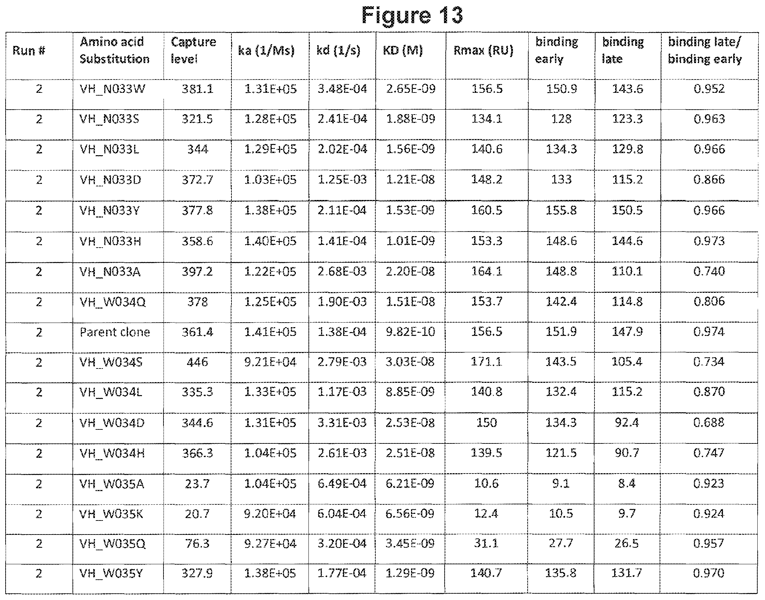

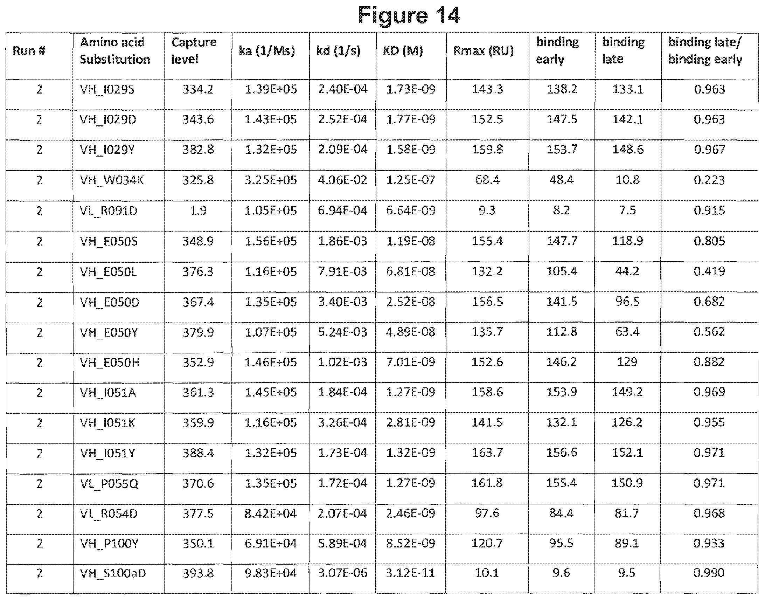

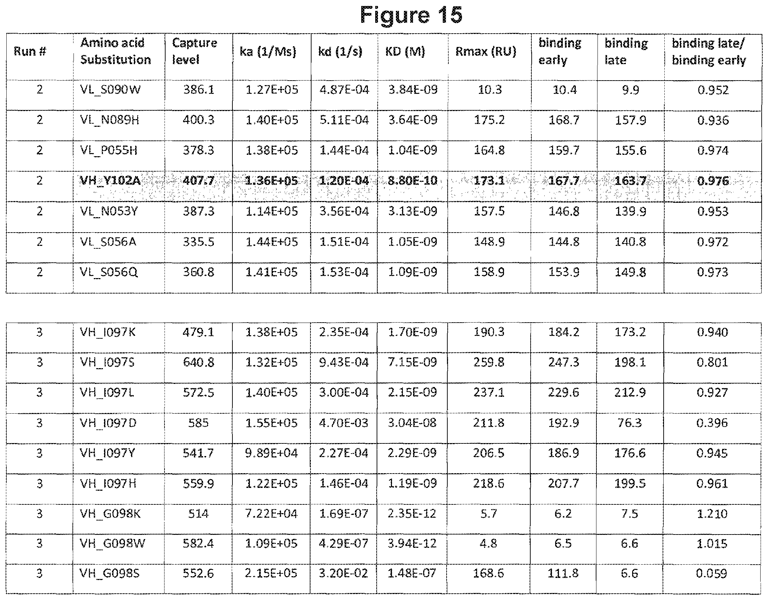

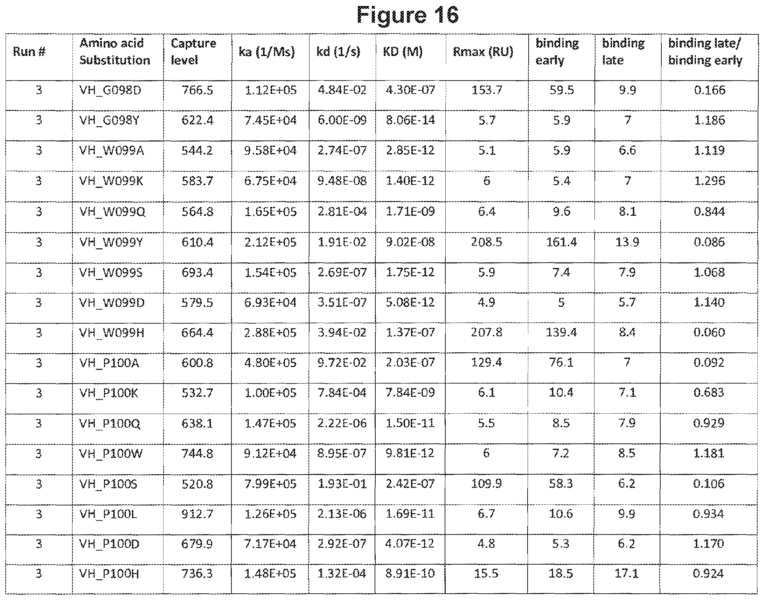

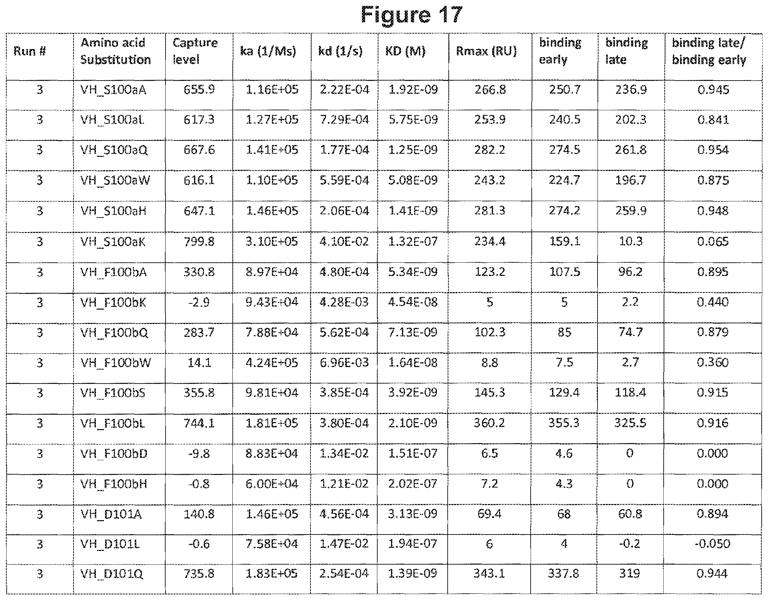

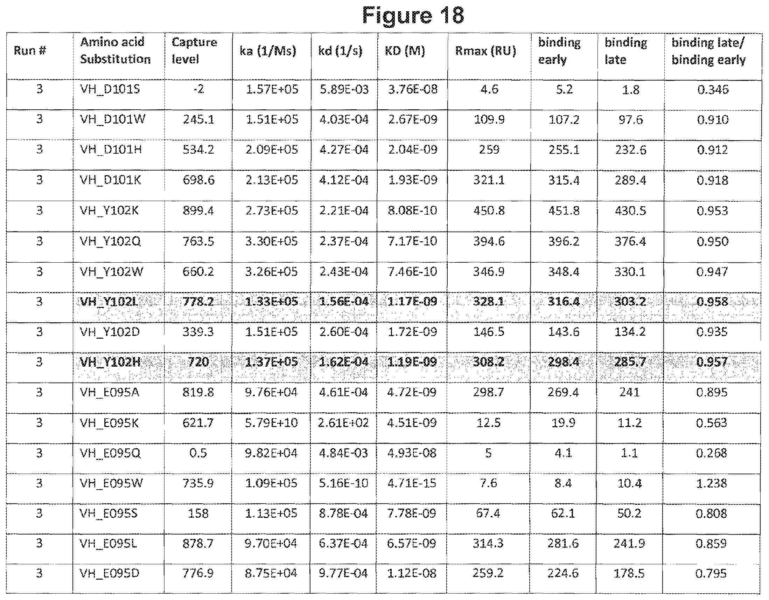

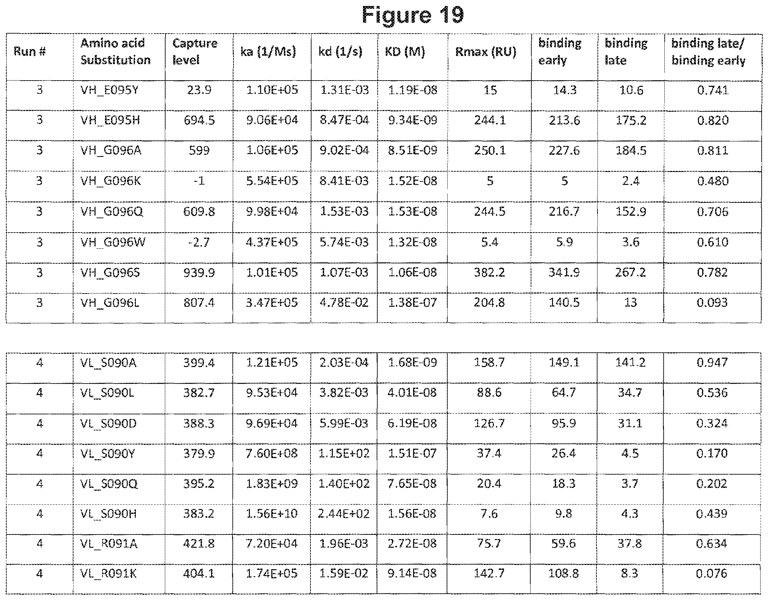

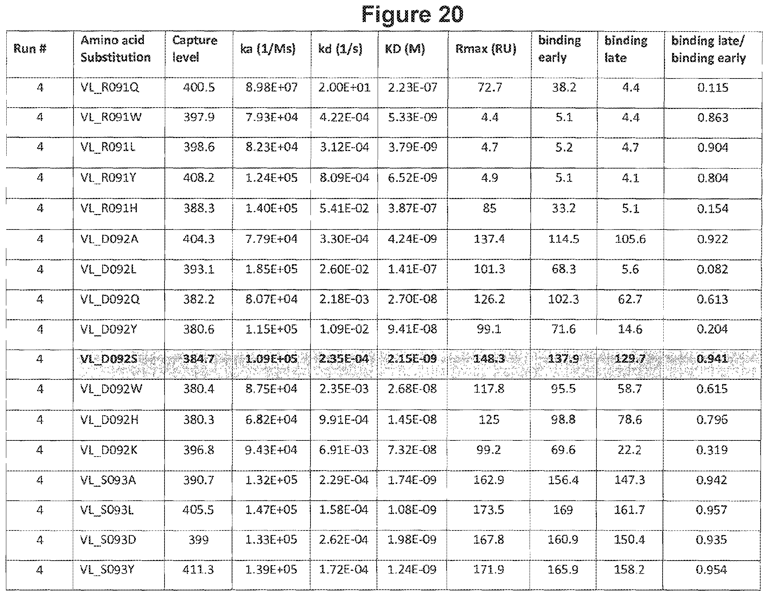

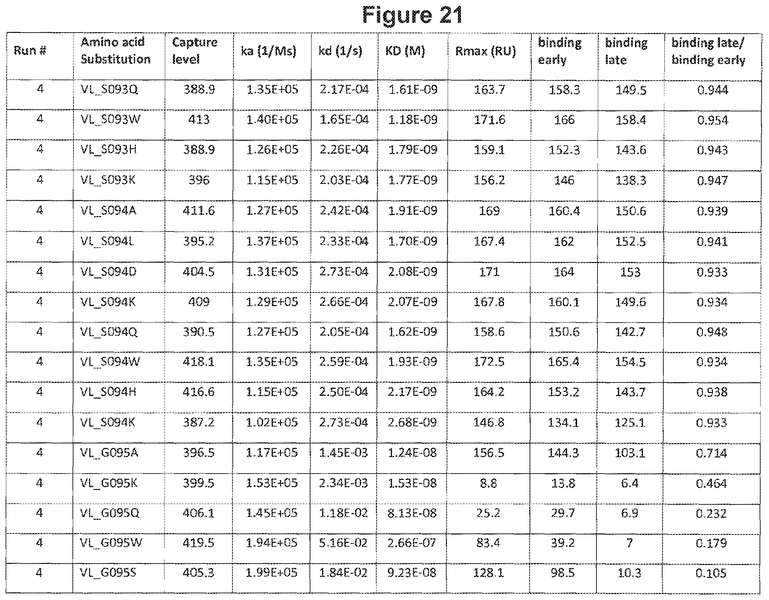

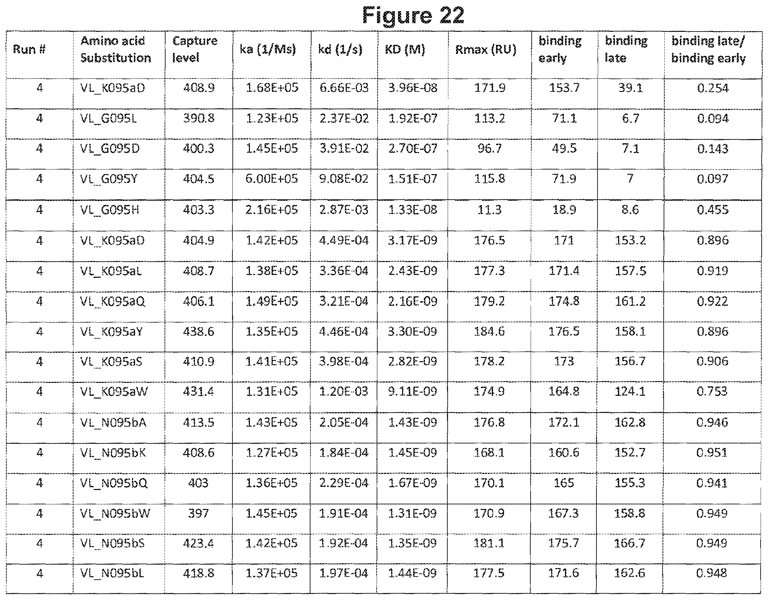

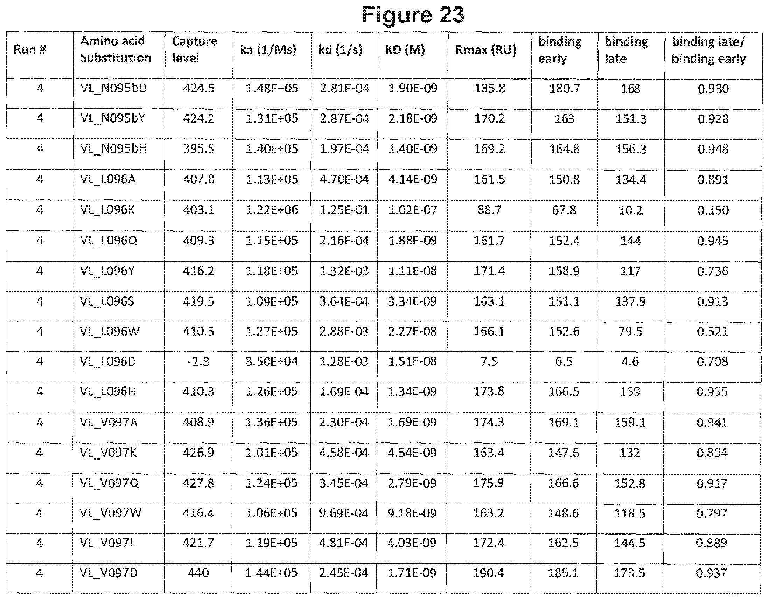

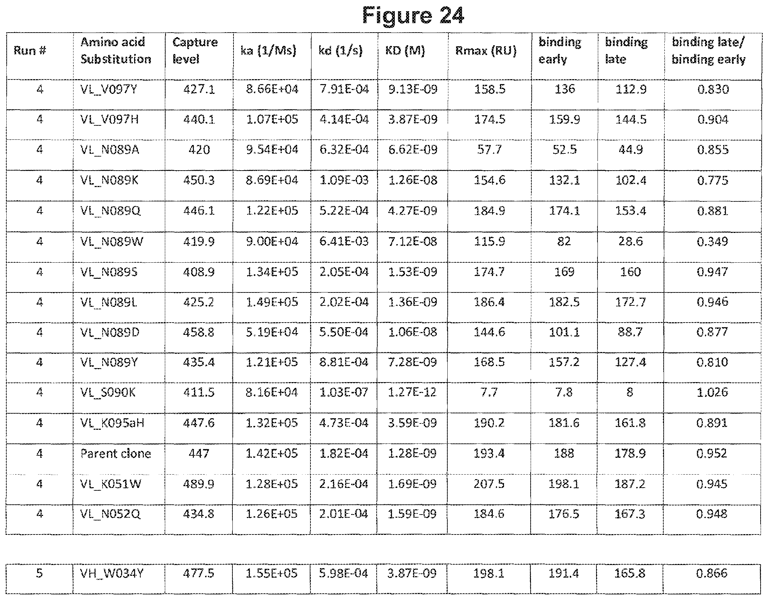

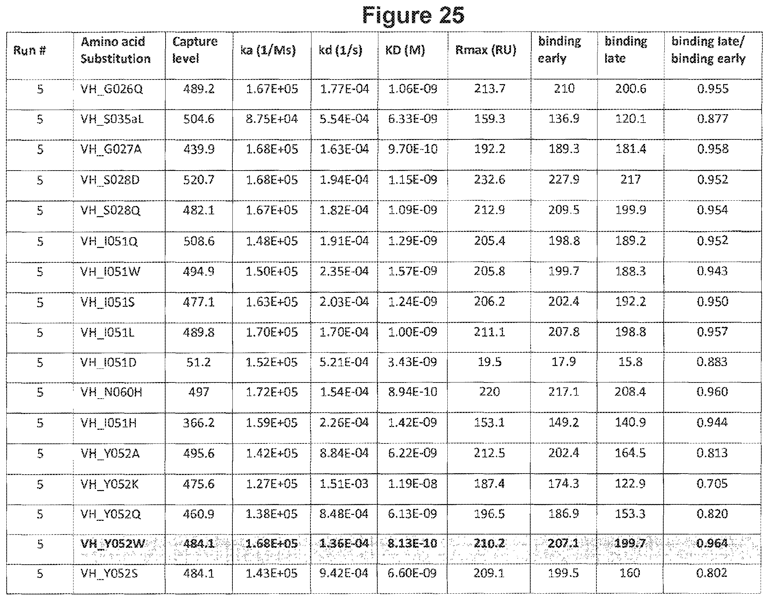

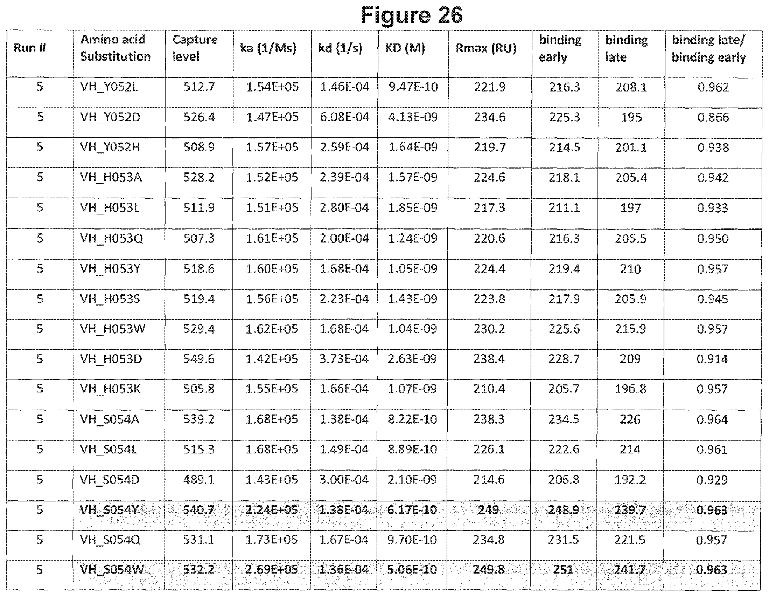

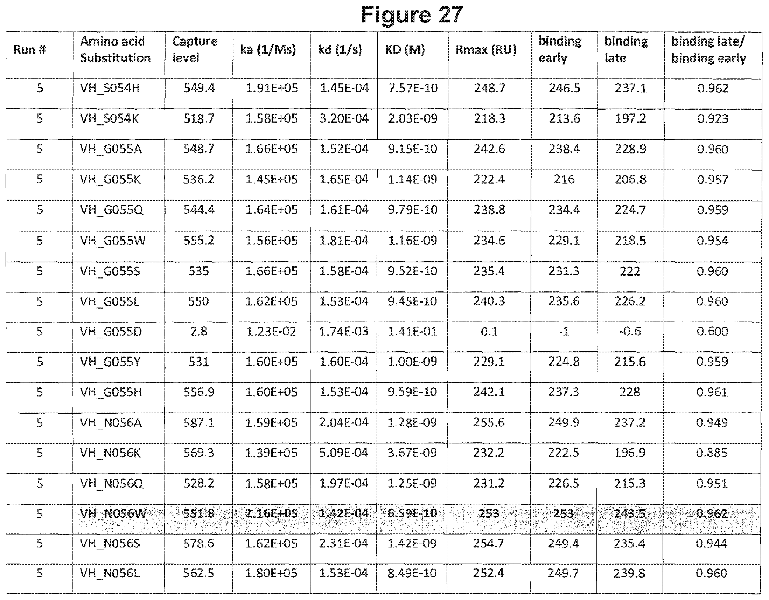

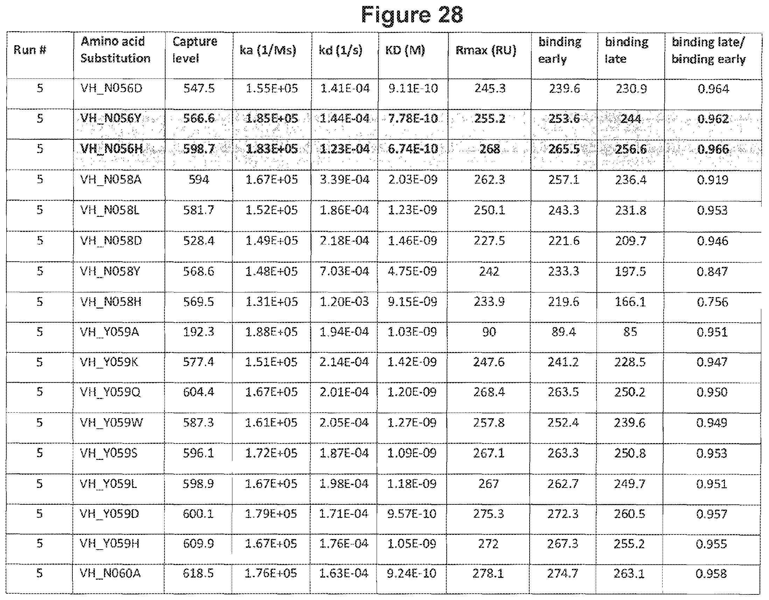

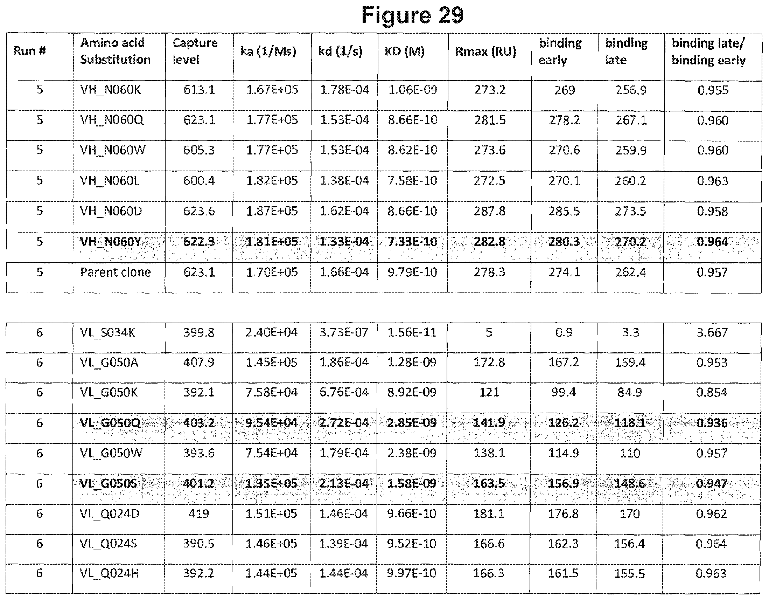

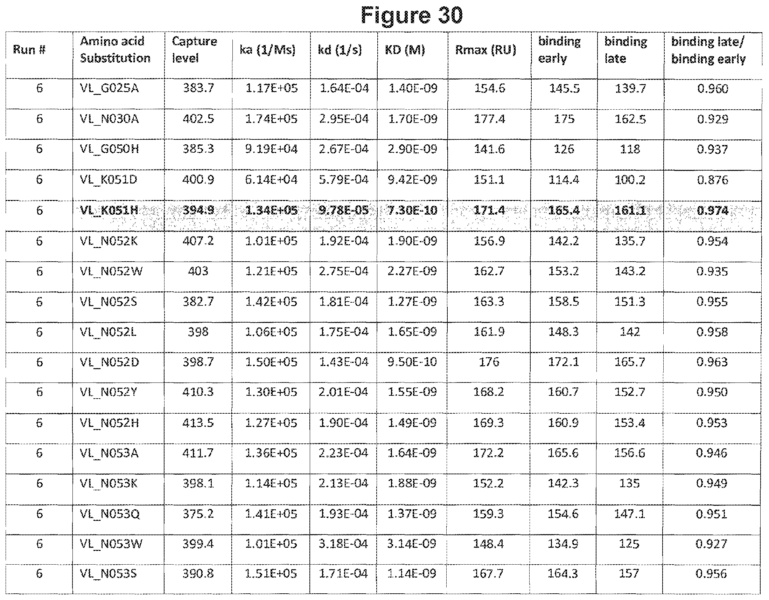

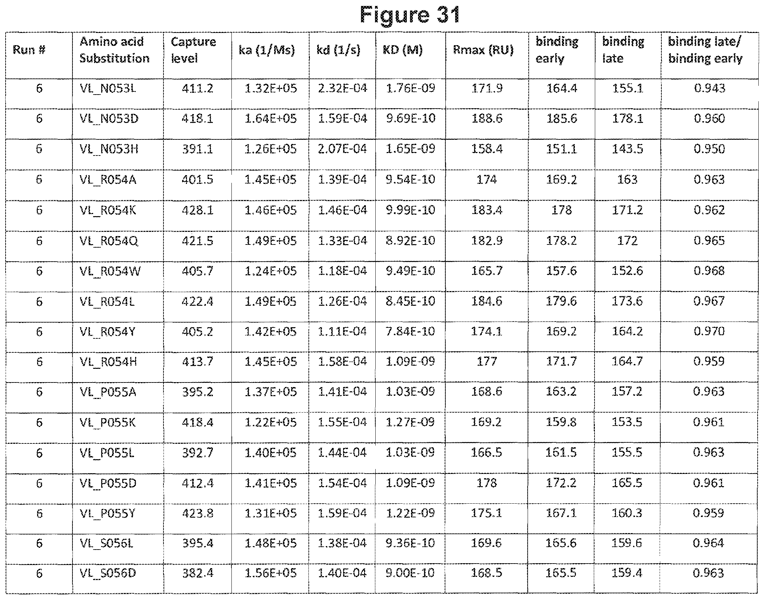

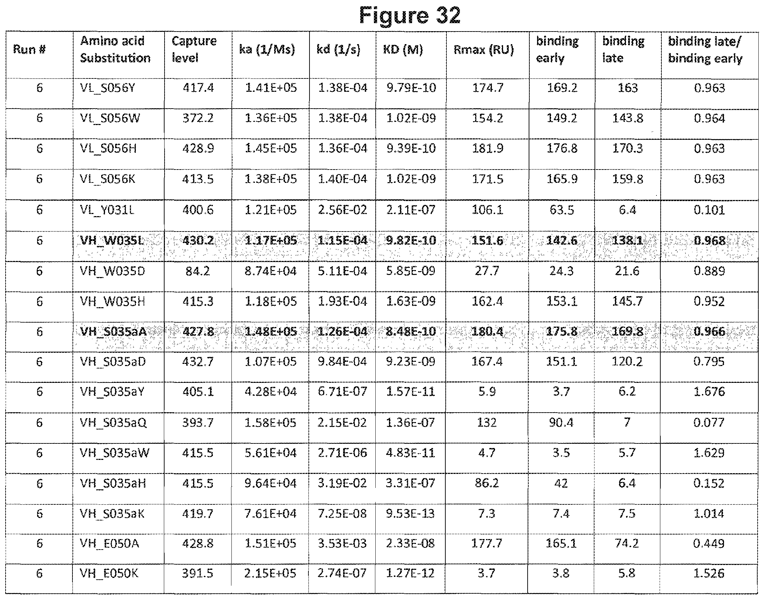

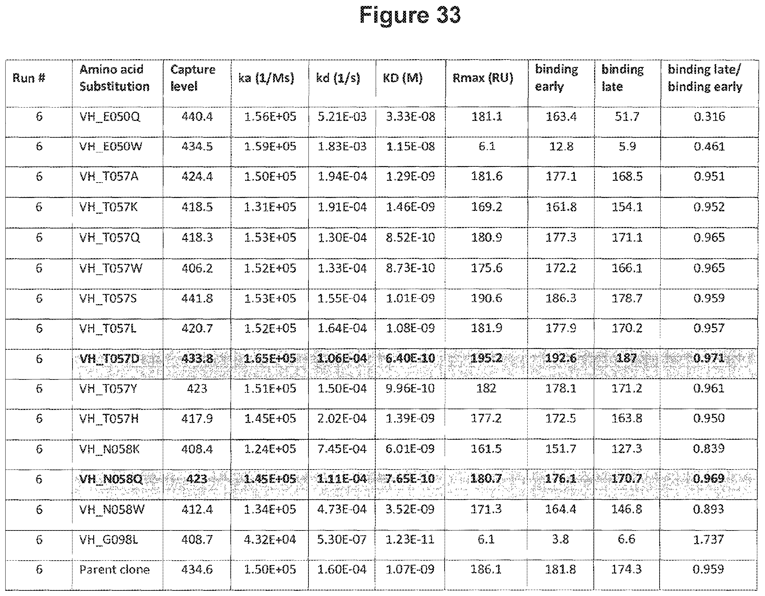

Figures 4-33 show BIACORE^{®} profiles of anti-IL-15 variants detailing antibody capture levels, single point affinity measurements and sequence changes relative to the parental antibody, Antibody 4.

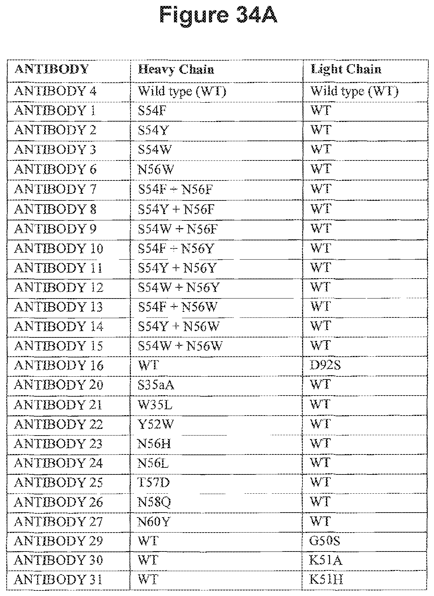

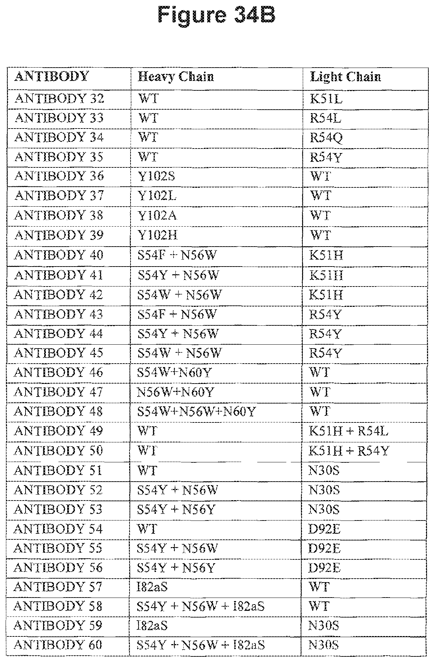

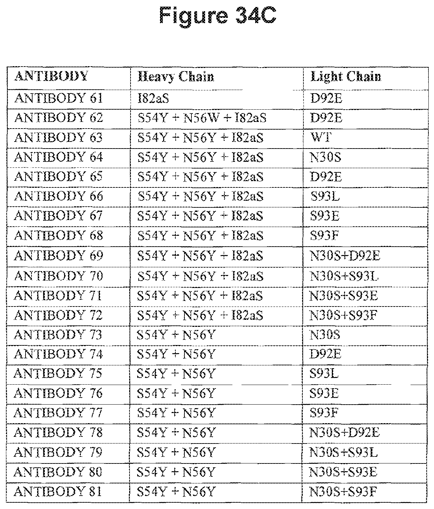

Figures 34A, 34B, and 34C show anti-IL-15 antibody variants, detailing their heavy and light chain amino acid substitutions relative to the parent antibody, Antibody 4.

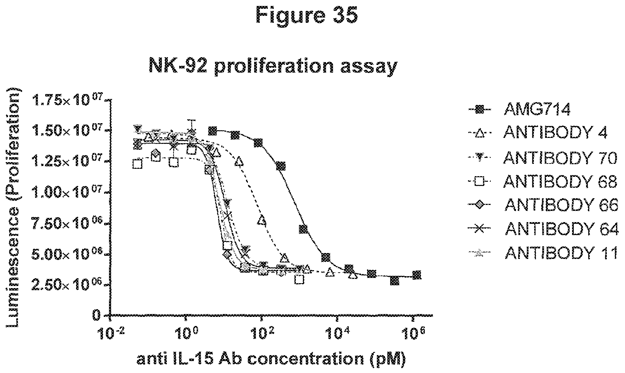

Figure 35 shows Antibody 4 variants with improved inhibition of IL-15-mediated NK-92 proliferation relative to the parent antibody, Antibody 4, and other anti-IL-15 antibodies. Readout was taken after 72 hours and is expressed as relative luminescence units.

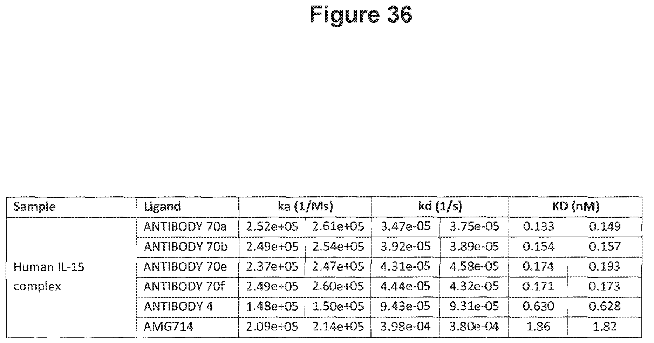

Figure 36 shows the binding kinetics of Antibody 4 variants and AMG714 binding to IL-15 complexed with the IL-15 Receptor-alpha. Binding kinetics was determined using surface plasmon resonance on a Biacore T200 (GE Healthcare) system. Antibody 4 variants bound the IL-15 complex with higher affinity than AMG714.

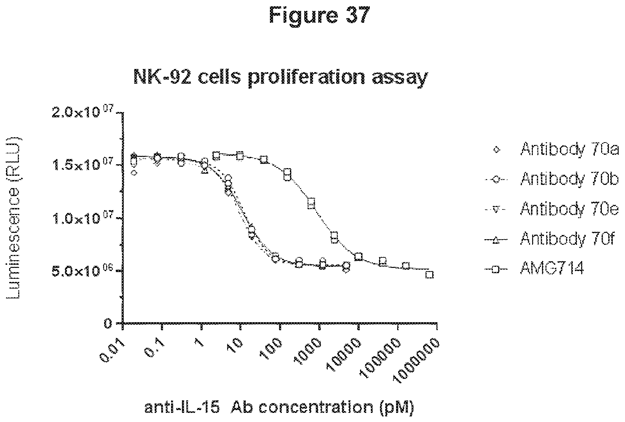

Figure 37 shows a comparison of anti-IL-15 antibodies in a NK-92 cell based assay. The inhibition of 25 pM of IL-15 complex mediated NK-92 proliferation by anti-IL-15 antibodies for 48 hours is expressed as relative luminescence units. The Antibody 70 variants have similar potency to each other and have a lower IC-50 value than AMG-714.

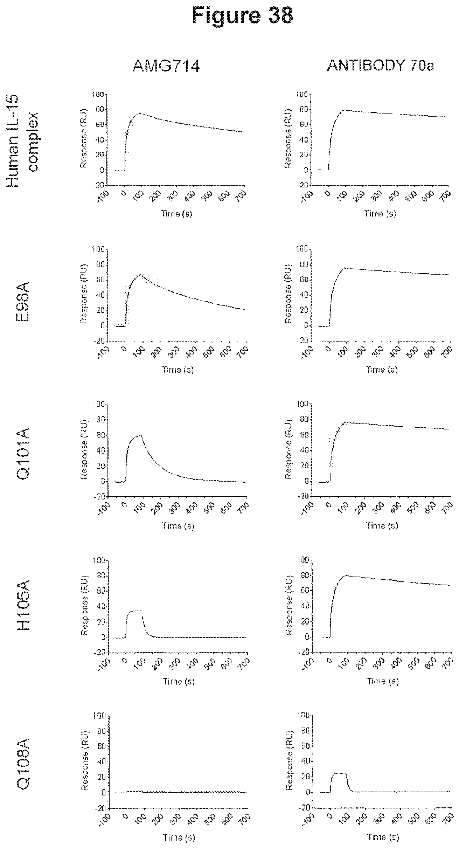

Figure 38 shows surface-exposed residues on IL-15 were converted to alanine by site-directed mutagenesis and were co-expressed with human IL-15Rα in EXPI293^{®} F cells. Binding of anti-IL-15 antibodies to purified IL-15 variants was assessed using surface plasmon resonance on a BICAORE^{®} T200 (GE Healthcare) system. AMG714 had significantly reduced or no binding to E98A, Q101A, H105A or Q108A as characterized by rapid dissociation or a faster dissociation rate. Antibody 70a had low binding to Q108A as characterized by a reduced association rate and a rapid dissociation.

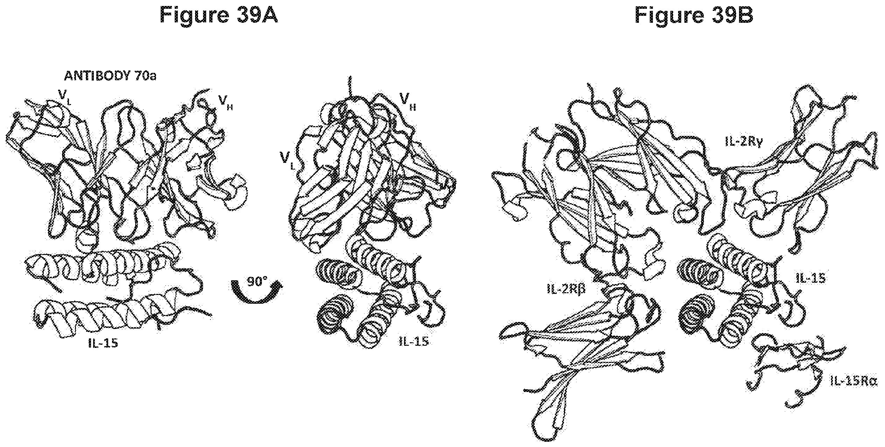

Figures 39A and 39B show the crystal structures of Antibody 70a.FAb/IL-15 complex and the quaternary IL-15 receptor complex. (Figure 39A) Cartoon representation of the variable region of Antibody 70a.FAb binding human IL-15, front and side views. (Figure 39B) The quaternary structure of the functional IL-15 complex. Cartoon representation of human IL-15 bound to IL-15Rα, IL-2Rβ and IL-2Ry (pdb code, 4GS7). The Antibody 70a.FAb disrupts the binding of IL-15 to IL-2Rβ and IL-2Ry. The Antibody 70a FAb binds to IL-15 distal to IL-15Rα and is able to bind the IL-15/ IL-15Rα complex.

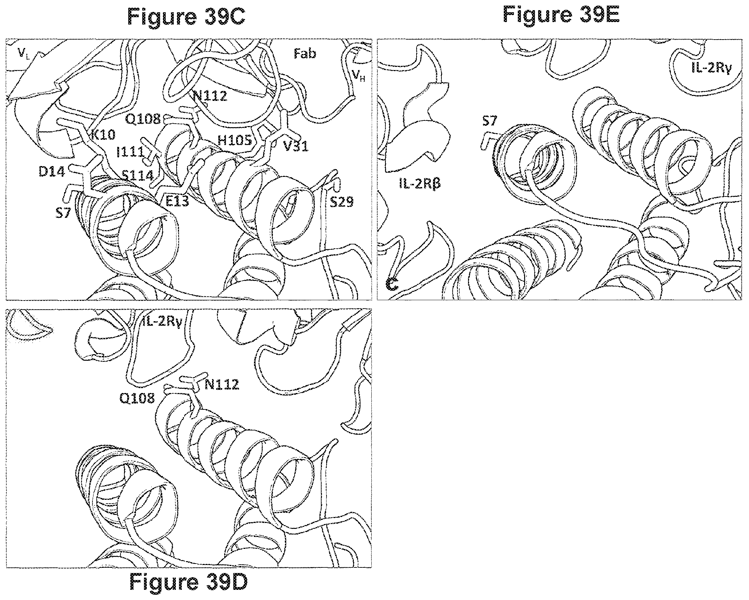

Figures 39C, 39D, and 39E show key binding residues of IL-15 interacting with Antibody 70a, IL-2Ry and IL-2Rβ. Only the IL-15 residues that contact respective partner proteins via hydrogen bonding are depicted and numbered. (Figure 39C) Residues used by IL-15 for interactions with Antibody 70a FAb (Figure 39D) Selected IL-15 residues that mediate hydrogen bonding with IL-2Ry including Q108, N112. (Figure 39E) The IL-15 residue, S7, makes a hydrogen bond with IL-2Rβ.

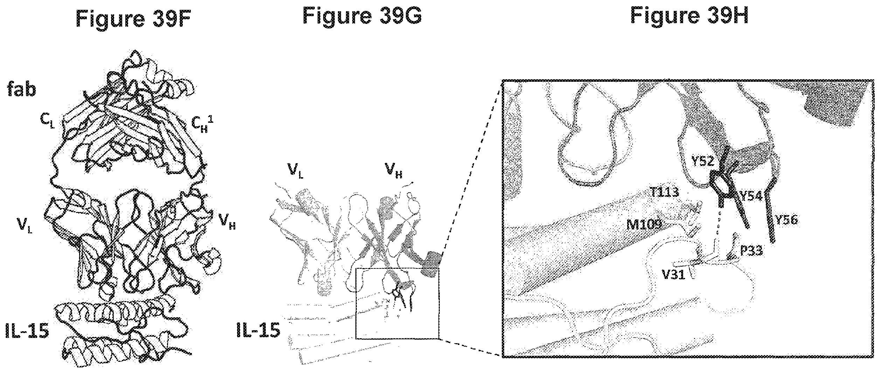

Figures 39F, 39G, and 39H show the crystal structure of human IL-15 with Antibody 70a. (Figure 39F) Cartoon representation showing the antibody FAb binding to human IL-15. (Figure 39G) A triple tyrosine motif comprising Y52/54/56 in CDRH2 is a key binding determinant of the antibody with human IL-15. (Figure 39H) A close-up of the YYY motif from Antibody 70a mediating interactions with human IL-15. This motif veils and protects hydrophobic residues around helix 4 of IL-15 preventing solvation. Side chains from IL-15 and CDRH2 of residues involved in this interaction are indicated in white and black sticks, respectively.

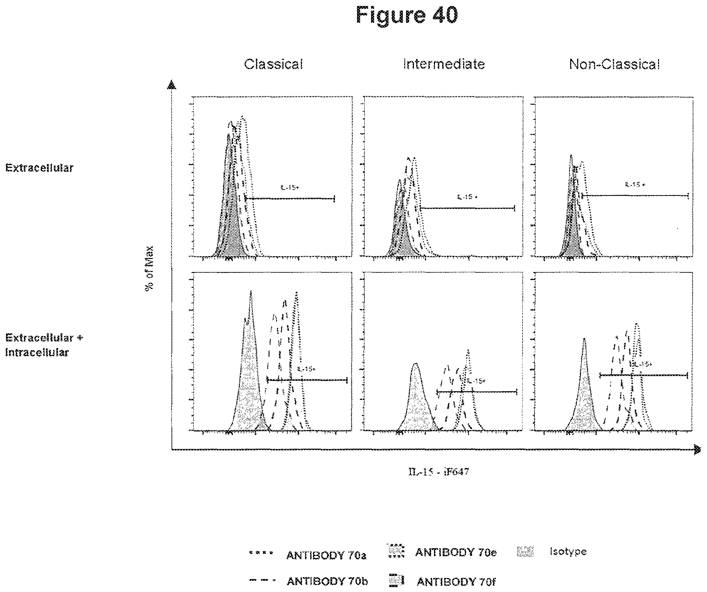

Figure 40 shows binding of Antibody 70 variants to human IL-15. Extracellular and intracellular IL-15 binding by anti-IL-15 mAbs was assessed on human monocyte subsets: classical, intermediate and non-classical monocytes. Anti-KLH C3 IgG1 isotype control was included in the analysis (filled). Representative Donor A data shown.

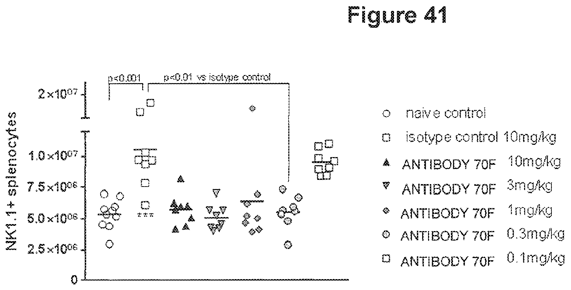

Figure 41 shows inhibition of IL-15 activity in mice by an exemplary anti-IL-15 antibody. The results presented are an enumeration of circulating NK cells in the spleen of mice injected with vehicle control or IL-15/II-15Rα-Fc complex followed by exemplary anti-IL-15 antibody or an Anti-KLH C3 IgG1 isotype control. Results are expressed as mean ± standard deviation of 8 animals per group.

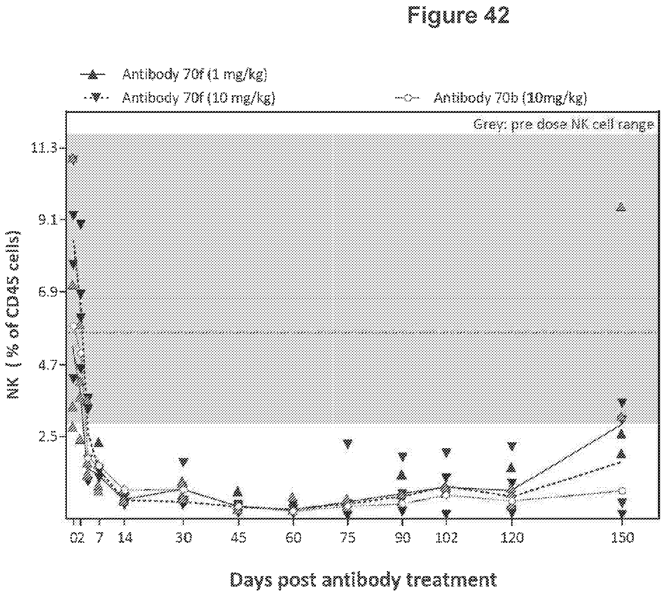

Figure 42 shows inhibition of circulating median NK cell numbers in cynomolgus monkeys by exemplary anti-IL-15 antibodies. Enumeration of circulating median NK cells in cynomolgus monkeys injected with exemplary anti-IL-15 antibodies tested at 10 mg/kg or 1 mg/kg. Circulating median numbers of NK cells were quantified by expression of the NK cell marker CD159a (NKG2A) and CD16. Results are expressed as individual timepoints for each monkey with the solid line indicating the median NK cell numbers per group (n=4).

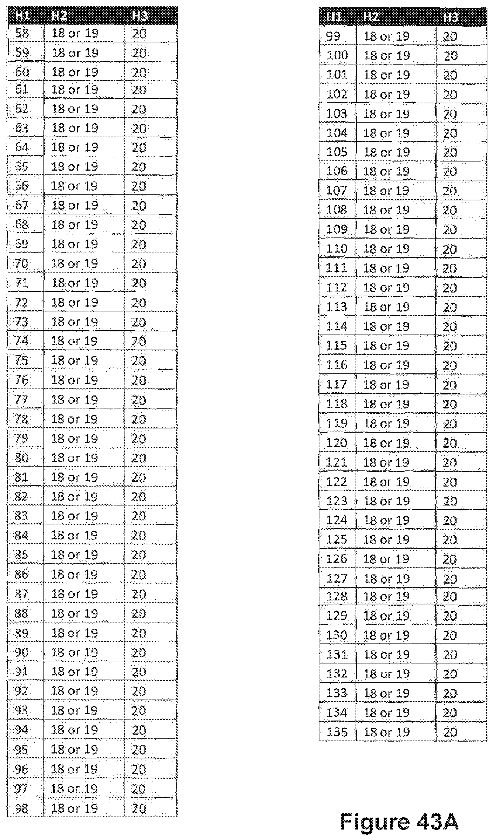

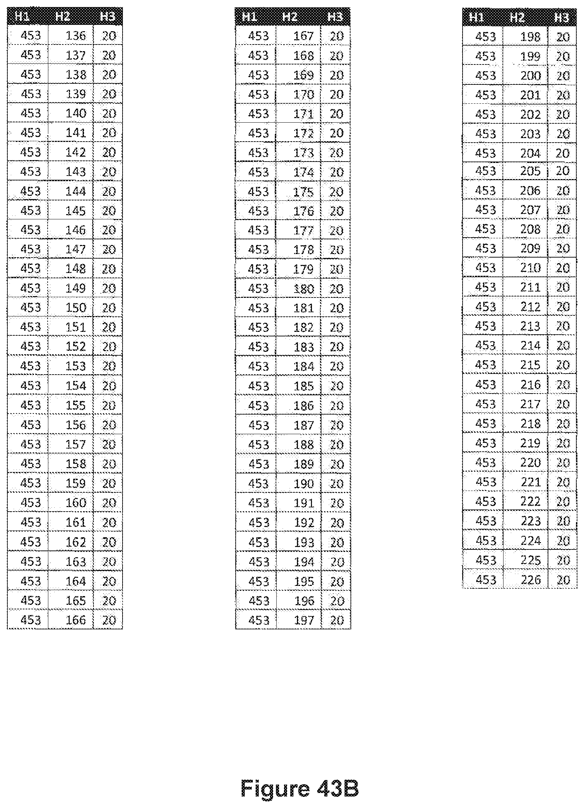

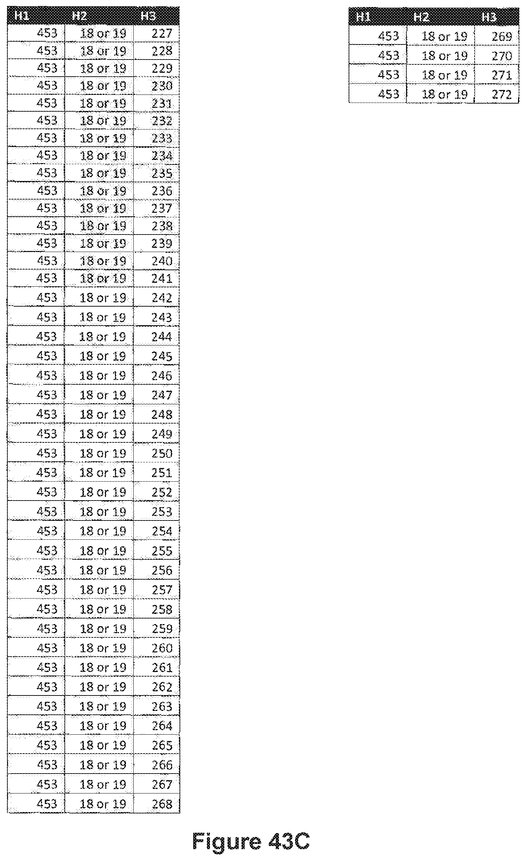

Figures 43A, 43B, and 43C show various HCDR1, HCDR2, and HCDR3 combinations.

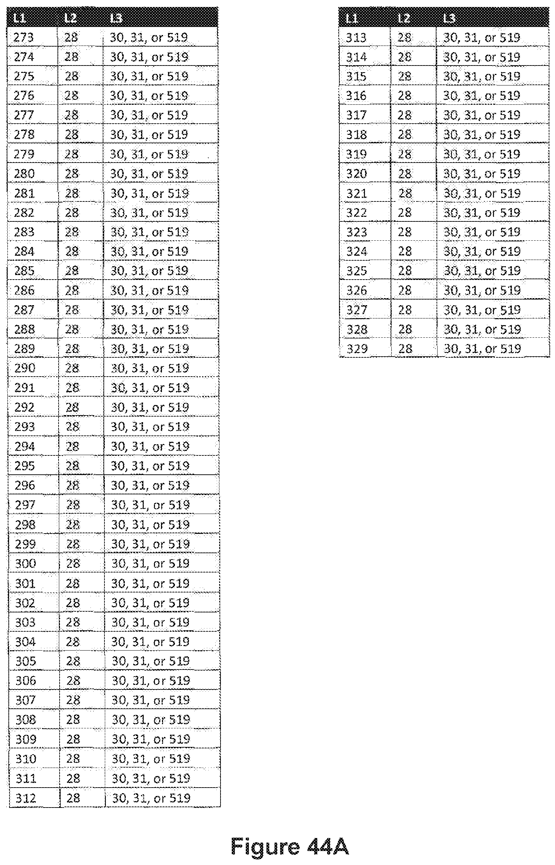

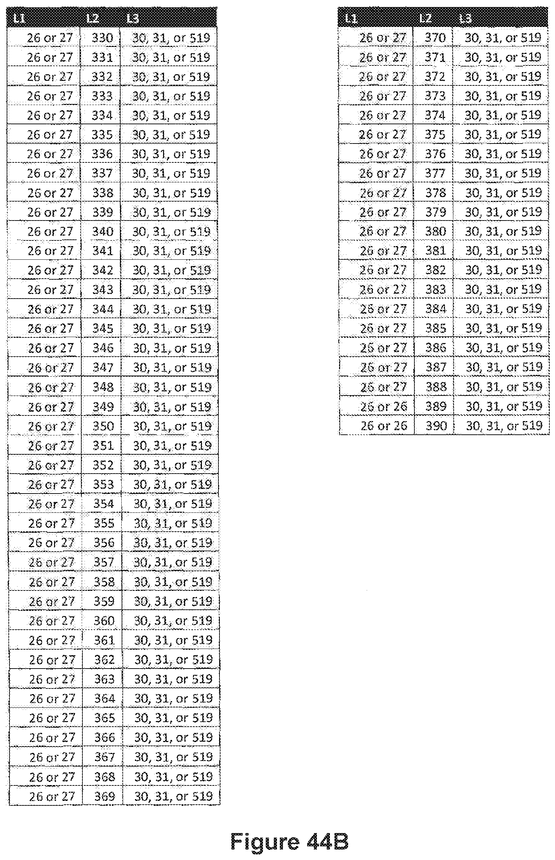

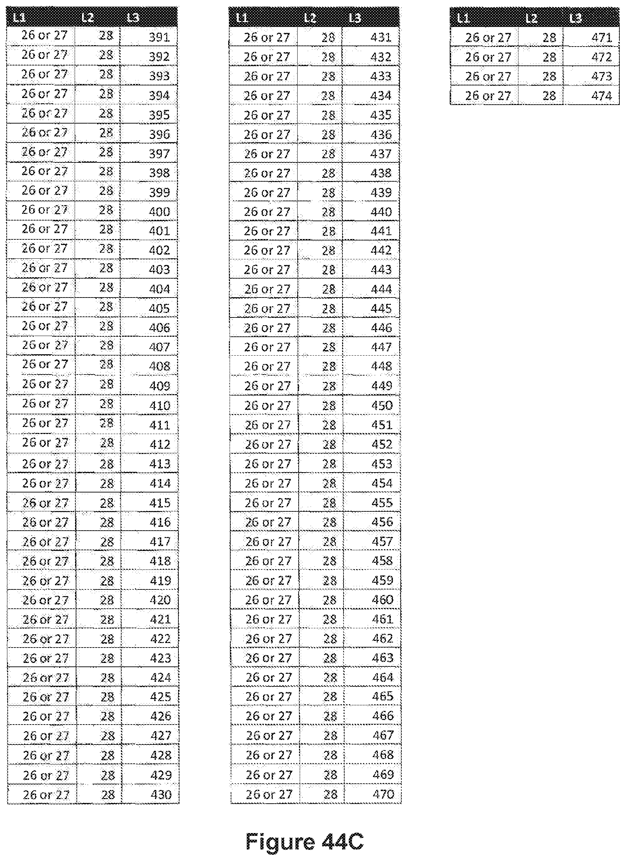

Figure 44A, 44B, and 44C show various LCDR1, LCDR2, and LCDR2 combinations.

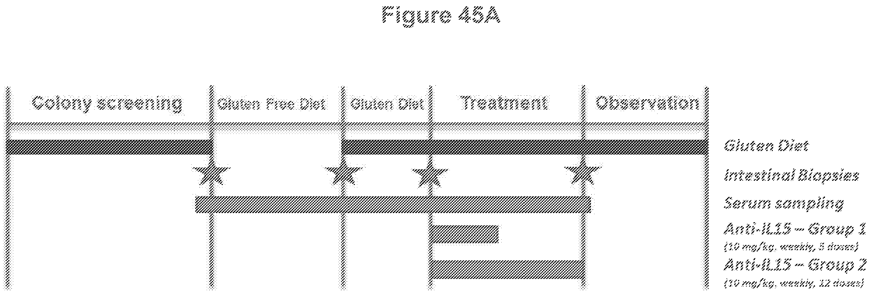

Figure 45A shows the study design of the rhesus macaque celiac disease model indicating stages, endpoints and treatment with Anti-IL15 antibody in two groups.

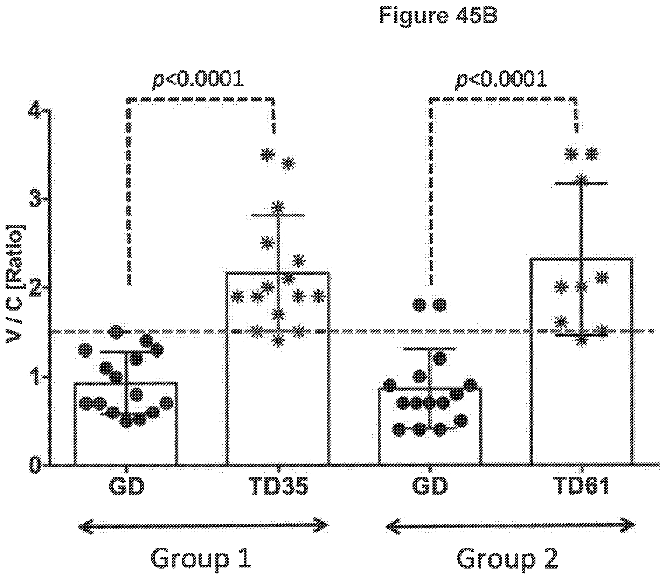

Figure 45B shows the attenuation of gluten-induced small intestinal mucosal injury by Anti-IL15 treatment as measured by the ratio between small intestinal villous heights and crypt depths (V/C). Intestinal jejunum wedge biopsies collected from two groups of macaques at time points corresponding to 6 months of GD diet, 35 days of aIL-15 treatment in group 1 macaques (TD35), and 61 days of treatment in group 2 macaques (TD61) were used to determine the V/C ratios.

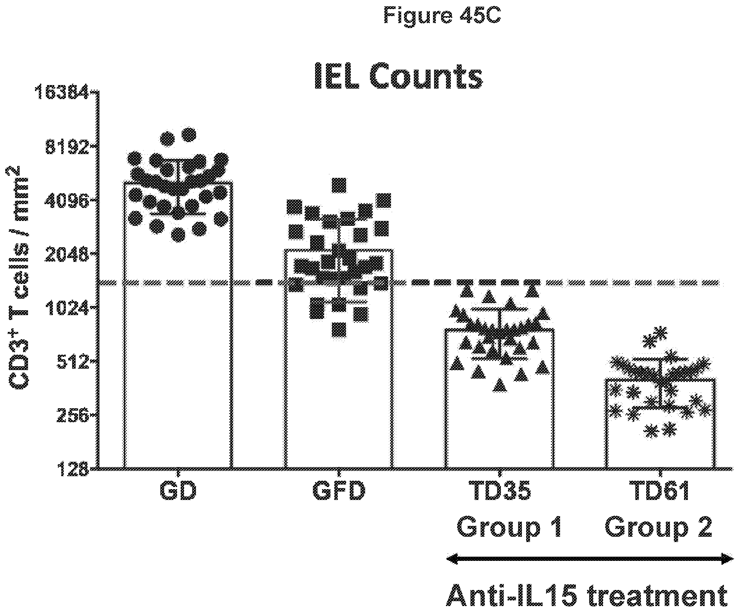

Figure 45C shows the attenuation of gluten-induced small intestinal mucosal inflammation by Anti-IL15 treatment as measured by the enumeration of intraepithelial lymphocytes (IELs) in histological sections. Time points reflect 6 months of GD diet, 3 months of GFD diet, 35 days post Anti-IL15 treatment in group 1 macaques (TD35), and 61 days of treatment in group 2 macaques (TD61). Dashed blue lines indicate healthy control baselines.

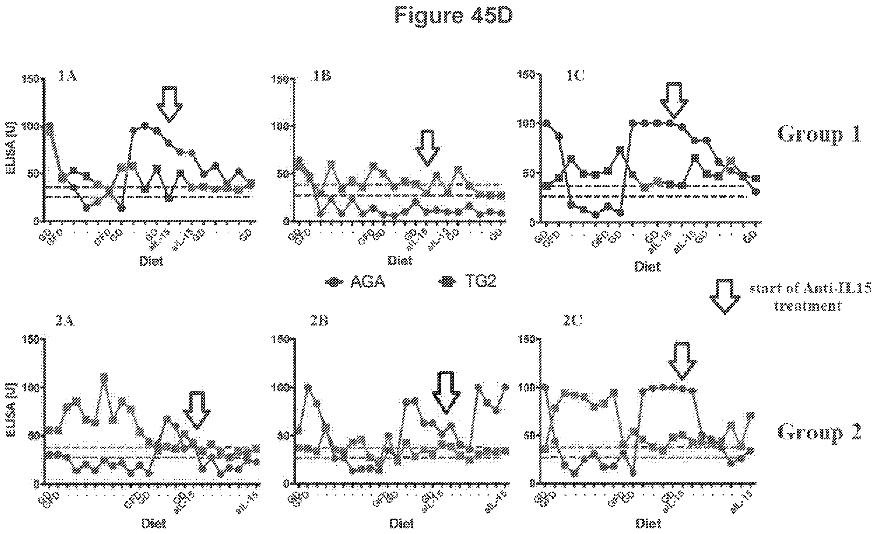

Figure 45D shows the attenuation of gluten-induced serum antibodies (anti-gliadin antibodies) by Anti-IL15 treatment. AGA is anti-gliadin antibodies; TG2 is anti-transglutaminase 2 autoantibodies. Distances between time points correspond to two-weeks intervals. Negative base line levels are indicated by dashed lines. Start of Anti-IL15 treatment is indicated by an arrow.

### DETAILED DESCRIPTION

Various terms relating to aspects of disclosure are used throughout the specification and claims. Such terms are to be given their ordinary meaning in the art, unless otherwise indicated. Other specifically defined terms are to be construed in a manner consistent with the definition provided herein.

As used herein, the singular forms "a," "an," and "the" include plural referents unless expressly stated otherwise.

The terms "subject" and "patient" are used interchangeably and include any animal. Mammals are preferred, including companion mammals *(e.g.,* cat, dog), farm mammals (*e.g.,* pig, horse, cow), rodents (e.g., mice, rabbits, rats, guinea pigs), and non-human primates. Human beings are highly preferred.

As used herein, "IL-15 complex" refers to the interaction between IL-15 and the IL-15 Receptor alpha (IL-15Rα).

"Specificity" in the context of antibody-antigen interactions is not necessarily an absolute designation but may constitute a relative term signifying the degree of selectivity of an antibody for an antigen. Specificity of an antibody for an antigen mediated by the variable regions of the antibody, and usually by the complementarity determining regions (CDRs) of the antibody.

The disclosure provides recombinantly produced antibodies that specifically bind to free (uncomplexed) human interleukin 15 (IL-15), as well as IL-15 that has bound to the IL-15 receptor alpha (IL-15 Rα) - the IL-15 complex. The antibodies bind to their antigen with high affinity, and significantly reduce IL-15-mediated proliferation of immune cells. The antibodies antagonize IL-15.

In preferred aspects, the antibodies bind to an epitope on human IL-15 (e.g. human IL-15 complexed with IL-15Rα) that includes at least the glutamine at position 108. The epitope may further include one or more of the serine at position 7 and the asparagine at position 112 of human IL-15 (e.g. human IL-15 complexed with IL-15Rα).

The epitope for a given antibody/antigen interaction can be elucidated using a variety of experimental epitope mapping methods. The experimental methods include mutagenesis (including alanine scanning), X-ray crystallography and various other methods that are well known in the art.

An epitope for the interaction between the antigen and the antibody may include the spatial coordinates defining the atomic contacts present in the antigen-antibody interaction. The epitope may be characterized by the spatial coordinates defining the atomic contacts between the antigen and antibody. The epitope may be characterized by the amino acid residues defined by a specific criterion, *e.g.,* by distance between atoms (*e.g.,* non-hydrogen atoms).

In the context of an X-ray derived crystal structure defined by spatial coordinates of a complex between an antibody, e.g., a FAb fragment, and its antigen, the term epitope includes IL-15 residues characterized by having water-mediated hydrogen bonds between atom pairs; hydrogen bonds of heteroatoms between 2.5- 3.5 Å; or a hydrogen bond corresponding to a donor/acceptor atom within an aromatic ring. Alternatively, a given IL-15 amino acid residue is considered to be part of an epitope if it participates in hydrophobic interaction or van der Waals interactions between atom pairs.

The epitope can also more generically include amino acid residues for which substitution by another amino acid will alter the characteristics of the interaction between the antibody and antigen (e.g., using alanine scanning). Alanine scanning mutagenesis experiments can be performed using a mutant IL-15 in which various residues of the IL-15 polypeptide have been replaced with alanine. By assessing binding of the antibody to the mutant IL-15, the importance of the particular IL-15 residues to antibody binding can be assessed. However, if burial of a nonpolar side chain occurs during the binding of antigen and antibody and results in the packing of the side chain against the antigen, then an alanine mutation at this position might not have a large impact on binding. It may be that although an alanine mutant results in reduced binding by the antibody, this does not mean that the residue is making contact, rather that the local three dimensional structure of the IL-15 could be perturbed by the introduction of an alanine. Further structural analysis of the complex, such as by X-ray crystallography, may be needed to assess contact residues between antibody and antigen.

The anti-IL-15 antibodies are preferably capable of inhibiting, reducing, or preventing the proliferation immune cells, such as natural killer (NK) cells and CD8⁺ T cells. In some aspects, the anti-IL-15 antibodies inhibit proliferation at an IC₅₀ of less than about 900 pM in an NK proliferation assay. In some aspects, the anti-IL-15 antibodies inhibit proliferation at an IC₅₀ of greater than 0 pM and less than about 900 pM in an NK proliferation assay. The anti-IL-15 antibodies may inhibit proliferation at an IC₅₀ of from about 1 pM to about 500 pM in an NK proliferation assay. The anti-IL-15 antibodies may inhibit proliferation at an IC₅₀ of from about 1 pM to about 250 pM in an NK proliferation assay. The anti-IL-15 antibodies may inhibit proliferation at an IC₅₀ of from about 1 pM to about 200 pM in an NK proliferation assay. The anti-IL-15 antibodies may inhibit proliferation at an IC₅₀ of from about 1 pM to about 150 pM in an NK proliferation assay. The anti-IL-15 antibodies may inhibit proliferation at an IC₅₀ of from about 1 pM to about 100 pM in an NK proliferation assay. The anti-IL-15 antibodies preferably inhibit proliferation at an IC₅₀ of from about 1 pM to about 60 pM in an NK proliferation assay. The anti-IL-15 antibodies preferably inhibit proliferation at an IC₅₀ of from about 5 pM to about 35 pM in an NK proliferation assay. The anti-IL-15 antibodies preferably inhibit proliferation at an IC₅₀ of from about 5 pM to about 30 pM in an NK proliferation assay.

As part of a suitable NK proliferation assay, cells such as CTLL-2 cells, may be cultured and induced to proliferate using a suitable concentration of a complex of IL-15 and the IL-15 Receptor alpha. Thus, a CTLL-2 proliferation assay may be used to determine the IC₅₀ of antibodies for proliferation inhibition. Any of the anti-IL-15 antibodies described or exemplified herein are added to the cell culture, and then the cells are incubated for a suitable period of time, including 48 hours, and assessed thereafter for proliferation or inhibition of proliferation owing to the presence of the antibodies, including by way of a cell viability assay.

As described or exemplified herein, amino acid positions assigned to CDRs and FRs may be according to Kabat Sequences of Proteins of Immunological Interest, National Institutes of Health, Bethesda, Md., 1987 and 1991 (also referred to herein as the Kabat numbering system). In addition, the amino acid positions assigned to CDRs and FRs may be according to the Enhanced Chothia Numbering Scheme (www.bioinfo.org.uk/mdex.html).

According to the numbering system of Kabat, VH FRs and CDRs may be positioned as follows: residues 1-30 (FR1), 31-35 (CDR1), 36-49 (FR2), 50-65 (CDR2), 66-94 (FR3), 95-102 (CDR3) and 103-113 (FR4), and VL FRs and CDRs are positioned as follows: residues 1-23 (FR1), 24-34 (CDR1), 35-49 (FR2), 50-56 (CDR2), 57-88 (FR3), 89-97 (CDR3) and 98-107 (FR4). In some instances, variable regions may increase in length and according to the Kabat numbering system some amino acids may be designated by a number followed by a letter. This specification is not limited to FRs and CDRs as defined by the Kabat numbering system, but includes all numbering systems, including the canonical numbering system or of Chothia et al. (1987) J. Mol. Biol. 196:901-17; Chothia et al. (1989) Nature 342:877-83; and/or Al-Lazikani et al. (1997) J. Mol. Biol. 273:927-48; the numbering system of Honnegher et al. (2001) J. Mol. Biol., 309:657-70; or the IMGT system discussed in Giudicelli et al. (1997) Nucleic Acids Res. 25:206-11. In preferred aspects, the CDRs are defined according to the Kabat numbering system.

In some particular aspects, for any of the heavy chain CDR2 subdomains described herein, according to the Kabat numbering system, the five C-terminal amino acids may not participate directly in antigen binding and, accordingly, it will be understood that any one or more of these five C-terminal amino acids may be substituted with another naturally-occurring amino acid without substantially adversely affecting antigen binding. In some aspects, for any of the light chain CDR1 subdomains described herein, according to the Kabat numbering system, the four N-terminal amino acids may not participate directly in antigen binding and, accordingly, it will be understood that any one or more of these four amino acids may be substituted with another naturally-occurring amino acid without substantially adversely affecting antigen binding. For example, as described by Padlan et al. (1995) FASEB J. 9:133-139, the five C terminal amino acids of heavy chain CDR2 and/or the four N-terminal amino acids of light chain CDR1 may not participate in antigen binding. In some aspects, both the heavy chain CDR2 and the light chain CDR1 do not directly participate in antigen binding.

In some aspects, the antibodies specifically bind to IL-15 (e.g. human IL-15 complexed with IL-15Rα), and comprise a heavy chain variable region CDR1 comprising the amino acid sequence of SEQ ID NO: 16, a heavy chain variable region CDR2 comprising the amino acid sequence of SEQ ID NO: 17 and a heavy chain variable region CDR3 comprising the amino acid sequence of SEQ ID NO: 20. In some preferred aspects, the antibodies comprise a heavy chain variable region CDR2 comprising the amino acid sequence of SEQ ID NO: 19, and in some preferred aspects, the antibodies comprise a heavy chain variable region CDR2 comprising the amino acid sequence of SEQ ID NO: 18. The antibodies may comprise a heavy chain variable region FR3 comprising the amino acid sequence of SEQ ID NO: 12, SEQ ID NO: 13, or SEQ ID NO: 14. The antibodies may further comprise a light chain variable region or a light chain. The light chain variable region may comprise the amino acid sequence of SEQ ID NO: 2, SEQ ID NO: 5, SEQ ID NO: 8, SEQ ID NO: 455, SEQ ID NO: 457, or SEQ ID NO: 459. The light chain variable region may comprise the amino acid sequence of SEQ ID NO: 503, SEQ ID NO:505, SEQ ID NO:506, SEQ ID NO:507, SEQ ID NO:509, or SEQ ID NO:510. The light chain may comprise the amino acid sequence of SEQ ID NO: 3, SEQ ID NO: 6, SEQ ID NO: 9, SEQ ID NO: 456, SEQ ID NO: 458, or SEQ ID NO: 460.

The heavy chain variable region CDR1 may comprise the amino acid sequence of any one of SEQ ID NO: 453 or SEQ ID NO: 52 through SEQ ID NO: 135. The heavy chain variable region CDR2 may comprise the amino acid sequence of any one of SEQ ID NO: 136 through SEQ ID NO: 226. The heavy chain variable region CDR3 may comprise the amino acid sequence of any one of SEQ ID NO: 227 through SEQ ID NO: 272. Suitable combinations of heavy chain variable region CDR1, CDR2, and CDR3 domains are shown in Figures 43A through 43C. Antibodies comprising such heavy chain variable region CDR1, CDR2, or CDR3 domains, or antibodies comprising the combinations of heavy chain variable region CDR1, CDR2, and CDR3 domains shown in Figures 43A through 43C may further comprise a light chain variable region or a light chain. The light chain variable region may comprise the amino acid sequence of SEQ ID NO: 2, SEQ ID NO: 5, SEQ ID NO: 8, SEQ ID NO: 455, SEQ ID NO: 457, or SEQ ID NO: 459. The light chain variable region may comprise the amino acid sequence of SEQ ID NO: 503, SEQ ID NO:505, SEQ ID NO:506, SEQ ID NO:507, SEQ ID NO:509, or SEQ ID NO:510. The light chain may comprise the amino acid sequence of SEQ ID NO: 3, SEQ ID NO: 6, SEQ ID NO: 9, SEQ ID NO: 456, SEQ ID NO: 458, or SEQ ID NO: 460.

In some aspects, the antibodies specifically bind to IL-15 (e.g. human IL-15 complexed with IL-15Rα), and comprise a light chain variable region CDR1 comprising the amino acid sequence of SEQ ID NO: 25, a light chain variable region CDR2 comprising the amino acid sequence of SEQ ID NO: 28, and a light chain variable region CDR3 comprising the amino acid sequence of SEQ ID NO: 29. In some preferred aspects, the antibodies comprise a light chain variable region CDR1 comprising the amino acid sequence of SEQ ID NO: 27, and in some preferred aspects, the antibodies comprise a light chain variable region CDR1 comprising the amino acid sequence of SEQ ID NO: 26. In some preferred aspects, the antibodies comprise a light chain variable region CDR3 comprising the amino acid sequence of SEQ ID NO: 31, and in some preferred aspects, the antibodies comprise a light chain variable region CDR3 comprising the amino acid sequence of SEQ ID NO: 30. The antibodies may further comprise a heavy chain variable region. The heavy chain variable region may comprise the amino acid sequence of SEQ ID NO: 1, SEQ ID NO: 4, SEQ ID NO: 7, or SEQ ID NO: 454.

The light chain variable region CDR1 may comprise the amino acid sequence of any one of SEQ ID NO: 273 through SEQ ID NO: 329. The light chain variable region CDR2 may comprise the amino acid sequence of any one of SEQ ID NO: 330 through SEQ ID NO: 390. The light chain variable region CDR3 may comprise the amino acid sequence of any one of SEQ ID NO: 391 through SEQ ID NO: 452. Suitable combinations of light chain variable region CDR1, CDR2, and CDR3 domains are shown in Figures 44A through 44C. Antibodies comprising such light chain variable region CDR1, CDR2, or CDR3 domains, or antibodies comprising the combinations of light chain variable region CDR1, CDR2, and CDR3 domains shown in Figures 44A through 44C may further comprise a heavy chain variable region. The heavy chain variable region may comprise the amino acid sequence of SEQ ID NO: 1, SEQ ID NO: 4, SEQ ID NO: 7, or SEQ ID NO: 454.

In some aspects, the antibodies specifically bind to IL-15 (e.g. human IL-15 complexed with IL-15Rα), and comprise a heavy chain variable region CDR1 comprising the amino acid sequence of SEQ ID NO: 16, a heavy chain variable region CDR2 comprising the amino acid sequence of SEQ ID NO: 17, a heavy chain variable region CDR3 comprising the amino acid sequence of SEQ ID NO: 20, a light chain variable region CDR1 comprising the amino acid sequence of SEQ ID NO: 25, a light chain variable region CDR2 comprising the amino acid sequence of SEQ ID NO: 28, and a light chain variable region CDR3 comprising the amino acid sequence of SEQ ID NO: 29. The antibodies may further comprise a heavy chain variable region FR3 comprising the amino acid sequence of SEQ ID NO: 12, SEQ ID NO: 13, or SEQ ID NO: 14.

In some preferred aspects, the antibodies comprise a heavy chain variable region CDR1 comprising the amino acid sequence of SEQ ID NO: 16, a heavy chain variable region CDR2 comprising the amino acid sequence of SEQ ID NO: 18, a heavy chain variable region CDR3 comprising the amino acid sequence of SEQ ID NO: 20, a light chain variable region CDR1 comprising the amino acid sequence of SEQ ID NO: 26, a light chain variable region CDR2 comprising the amino acid sequence of SEQ ID NO: 28, and a light chain variable region CDR3 comprising the amino acid sequence of SEQ ID NO: 30. The antibodies may further comprise a heavy chain variable region FR3 comprising the amino acid sequence of SEQ ID NO: 12, or SEQ ID NO: 13.

In some aspects, the antibodies specifically bind to IL-15 (e.g. human IL-15 complexed with IL-15Rα), and comprise a heavy chain variable region CDR1 comprising the amino acid sequence of SEQ ID NO: 16, a heavy chain variable region CDR2 comprising the amino acid sequence of SEQ ID NO: 19, a heavy chain variable region CDR3 comprising the amino acid sequence of SEQ ID NO: 20, a light chain variable region CDR1 comprising the amino acid sequence of SEQ ID NO: 27, a light chain variable region CDR2 comprising the amino acid sequence of SEQ ID NO: 28, and a light chain variable region CDR3 comprising the amino acid sequence of SEQ ID NO: 31. The antibodies may further comprise a heavy chain variable region FR3 comprising the amino acid sequence of SEQ ID NO: 12, SEQ ID NO: 13, or SEQ ID NO: 14.

In some aspects, the antibodies specifically bind to IL-15 (e.g. human IL-15 complexed with IL-15Rα), and comprise a heavy chain variable region CDR1 comprising the amino acid sequence of SEQ ID NO: 16, a heavy chain variable region CDR2 comprising the amino acid sequence of SEQ ID NO: 18, a heavy chain variable region CDR3 comprising the amino acid sequence of SEQ ID NO: 20, a light chain variable region CDR1 comprising the amino acid sequence of SEQ ID NO: 27, a light chain variable region CDR2 comprising the amino acid sequence of SEQ ID NO: 28, and a light chain variable region CDR3 comprising the amino acid sequence of SEQ ID NO: 31. The antibodies may further comprise a heavy chain variable region FR3 comprising the amino acid sequence of SEQ ID NO: 12 or SEQ ID NO: 14.

In some aspects, the antibodies specifically bind to IL-15 (e.g. human IL-15 complexed with IL-15Rα), and comprise a heavy chain variable region CDR1 comprising the amino acid sequence of SEQ ID NO: 16, a heavy chain variable region CDR2 comprising the amino acid sequence of SEQ ID NO: 18, a heavy chain variable region CDR3 comprising the amino acid sequence of SEQ ID NO: 20, a light chain variable region CDR1 comprising the amino acid sequence of SEQ ID NO: 26, a light chain variable region CDR2 comprising the amino acid sequence of SEQ ID NO: 28, and a light chain variable region CDR3 comprising the amino acid sequence of SEQ ID NO: 31. The antibodies may further comprise a heavy chain variable region FR3 comprising the amino acid sequence of SEQ ID NO: 12 or SEQ ID NO: 13.

In some aspects, the antibodies specifically bind to IL-15 (e.g. human IL-15 complexed with IL-15Rα), and comprise a heavy chain variable region CDR1 comprising the amino acid sequence of SEQ ID NO: 16, a heavy chain variable region CDR2 comprising the amino acid sequence of SEQ ID NO: 18, a heavy chain variable region CDR3 comprising the amino acid sequence of SEQ ID NO: 20, a light chain variable region CDR1 comprising the amino acid sequence of SEQ ID NO: 27, a light chain variable region CDR2 comprising the amino acid sequence of SEQ ID NO: 28, and a light chain variable region CDR3 comprising the amino acid sequence of SEQ ID NO: 30. The antibodies may further comprise a heavy chain variable region FR3 comprising the amino acid sequence of SEQ ID NO: 12 or SEQ ID NO: 13.

In some aspects, the antibodies specifically bind to IL-15 (e.g. human IL-15 complexed with IL-15Rα), and comprise a heavy chain variable region CDR1 comprising the amino acid sequence of SEQ ID NO: 16, a heavy chain variable region CDR2 comprising the amino acid sequence of SEQ ID NO: 18, a heavy chain variable region CDR3 comprising the amino acid sequence of SEQ ID NO: 20, a light chain variable region CDR1 comprising the amino acid sequence of SEQ ID NO: 27 , a light chain variable region CDR2 comprising the amino acid sequence of SEQ ID NO: 28, and a light chain variable region CDR3 comprising the amino acid sequence of SEQ ID NO: 29 wherein Xaa5 of SEQ ID NO: 29 is F (SEQ ID NO: 519). The antibodies may further comprise a heavy chain variable region FR3 comprising the amino acid sequence of SEQ ID NO: 12 or SEQ ID NO: 13.

In some aspects, the antibodies specifically bind to IL-15 (e.g. human IL-15 complexed with IL-15Rα), and comprise a heavy chain variable region comprising a CDR2 comprising the amino acid sequence of SEQ ID NO: 18, and a light chain variable region or a light chain. The light chain variable region may comprise the amino acid sequence of SEQ ID NO: 2, SEQ ID NO: 5, SEQ ID NO: 8, SEQ ID NO: 455, SEQ ID NO: 457, or SEQ ID NO: 459. The light chain variable region may comprise the amino acid sequence of SEQ ID NO: 503, SEQ ID NO:505, SEQ ID NO:506, SEQ ID NO:507, SEQ ID NO:509, or SEQ ID NO:510. The light chain may comprise the amino acid sequence of SEQ ID NO: 6, SEQ ID NO: 9, SEQ ID NO: 456, SEQ ID NO: 458, or SEQ ID NO: 460.

The antibodies may specifically bind to IL-15 (e.g. human IL-15 complexed with IL-15Rα), and comprise, or further comprise a heavy chain variable region comprising the subdomains comprising the amino acid sequence shown in the following table:

| | FR1 | H1 | FR2 | H2 | FR3 | H3 | FR4 |
|---|---|---|---|---|---|---|---|
| SEQ ID NO: | 10 | 16 | 11 | 19 | 14 | 20 | 15 |
| SEQ ID NO: | 10 | 16 | 11 | 18 | 14 | 20 | 15 |
| SEQ ID NO: | 10 | 16 | 11 | 18 | 13 | 20 | 15 |
| SEQ ID NO: | 10 | 16 | 11 | 18 | 13 | 20 | 15 |
| SEQ ID NO: | 10 | 16 | 11 | 18 | 13 | 20 | 15 |
| SEQ ID NO: | 10 | 16 | 11 | 18 | 13 | 20 | 15 |

The antibodies may specifically bind to IL-15 (e.g. human IL-15 complexed with IL-15Rα), and comprise, or further comprise a light chain variable region comprising the subdomains comprising the amino acid sequence shown in the following table:

| | FR1 | L1 | FR2 | L2 | FR3 | L3 | FR4 |
|---|---|---|---|---|---|---|---|
| SEQ ID NO: | 21 | 27 | 22 | 28 | 23 | 31 | 24 |
| SEQ ID NO: | 21 | 27 | 22 | 28 | 23 | 31 | 24 |
| SEQ ID NO: | 21 | 26 | 22 | 28 | 23 | 31 | 24 |
| SEQ ID NO: | 21 | 27 | 22 | 28 | 23 | 30 | 24 |
| SEQ ID NO: | 21 | 27 | 22 | 28 | 23 | 519 | 24 |
| SEQ ID NO: | 21 | 26 | 22 | 28 | 23 | 30 | 24 |

In some aspects, the antibodies specifically bind to IL-15 (e.g. human IL-15 complexed with IL-15Rα), and comprise a heavy chain variable region comprising the amino acid sequence of SEQ ID NO: 1. In some preferred aspects, the antibodies comprise a heavy chain variable region comprising the amino acid sequence of SEQ ID NO: 4. In some preferred aspects, the antibodies comprise a heavy chain variable region comprising the amino acid sequence of SEQ ID NO: 7. In some preferred aspects, the antibodies comprise a heavy chain variable region comprising the amino acid sequence of SEQ ID NO: 454. The antibodies may further comprise a light chain variable region or a light chain. The light chain variable region may comprise the amino acid sequence of SEQ ID NO: 2, SEQ ID NO: 5, SEQ ID NO: 8, SEQ ID NO: 454, SEQ ID NO: 457, or SEQ ID NO: 459. The light chain variable region may comprise the amino acid sequence of SEQ ID NO: 503, SEQ ID NO:505, SEQ ID NO:506, SEQ ID NO:507, SEQ ID NO:509, or SEQ ID NO:510. The light chain may comprise the amino acid sequence of SEQ ID NO: 3, SEQ ID NO: 6, SEQ ID NO: 9, SEQ ID NO: 456, SEQ ID NO: 458, or SEQ ID NO: 460.

In some aspects, the antibodies specifically bind to IL-15 (e.g. human IL-15 complexed with IL-15Rα), and comprise a light chain variable region comprising the amino acid sequence of SEQ ID NO: 2. In some preferred aspects, the antibodies comprise a light chain variable region comprising the amino acid sequence of SEQ ID NO: 5. In some preferred aspects, the antibodies comprise a light chain variable region comprising the amino acid sequence of SEQ ID NO: 8. In some preferred aspects, the antibodies comprise a light chain variable region comprising the amino acid sequence of SEQ ID NO: 455. In some preferred aspects, the antibodies comprise a light chain variable region comprising the amino acid sequence of SEQ ID NO: 457. In some preferred aspects, the antibodies comprise a light chain variable region comprising the amino acid sequence of SEQ ID NO: 459. In some preferred aspects, the antibodies comprise a light chain variable region comprising the amino acid sequence of SEQ ID NO: 503. In some preferred aspects, the antibodies comprise a light chain variable region comprising the amino acid sequence of SEQ ID NO: 505. In some preferred aspects, the antibodies comprise a light chain variable region comprising the amino acid sequence of SEQ ID NO: 506. In some preferred aspects, the antibodies comprise a light chain variable region comprising the amino acid sequence of SEQ ID NO: 507. In some preferred aspects, the antibodies comprise a light chain variable region comprising the amino acid sequence of SEQ ID NO: 509. In some preferred aspects, the antibodies comprise a light chain variable region comprising the amino acid sequence of SEQ ID NO: 510. The antibodies may further comprise a heavy chain variable region. The heavy chain variable region may comprise the amino acid sequence of SEQ ID NO: 1, SEQ ID NO: 4, SEQ ID NO: 7, or SEQ ID NO: 454.

In some aspects, the antibodies specifically bind to IL-15 (e.g. human IL-15 complexed with IL-15Rα), and comprise a light chain comprising the amino acid sequence of SEQ ID NO: 3. In some preferred aspects, the antibodies comprise a light chain comprising the amino acid sequence of SEQ ID NO: 6. In some preferred aspects, the antibodies comprise a light chain comprising the amino acid sequence of SEQ ID NO: 9. In some preferred aspects, the antibodies comprise a light chain comprising the amino acid sequence of SEQ ID NO: 456. In some preferred aspects, the antibodies comprise a light chain comprising the amino acid sequence of SEQ ID NO: 458. In some preferred aspects, the antibodies comprise a light chain comprising the amino acid sequence of SEQ ID NO: 460. The antibodies may further comprise a heavy chain variable region. The heavy chain variable region may comprise the amino acid sequence of SEQ ID NO: 1, SEQ ID NO: 4, SEQ ID NO: 7, or SEQ ID NO: 454.

The antibodies may comprise a heavy chain variable region comprising the amino acid sequence of SEQ ID NO: 1, and a light chain variable region comprising the amino acid sequence of SEQ ID NO: 2. The antibodies may comprise a heavy chain variable region comprising the amino acid sequence of SEQ ID NO: 1, and a light chain variable region comprising the amino acid sequence of SEQ ID NO: 5. The antibodies may comprise a heavy chain variable region comprising the amino acid sequence of SEQ ID NO: 1, and a light chain variable region comprising the amino acid sequence of SEQ ID NO: 8. The antibodies may comprise a heavy chain variable region comprising the amino acid sequence of SEQ ID NO: 1, and a light chain variable region comprising the amino acid sequence of SEQ ID NO: 455. The antibodies may comprise a heavy chain variable region comprising the amino acid sequence of SEQ ID NO: 1, and a light chain variable region comprising the amino acid sequence of SEQ ID NO: 457. The antibodies may comprise a heavy chain variable region comprising the amino acid sequence of SEQ ID NO: 1, and a light chain variable region comprising the amino acid sequence of SEQ ID NO: 459. Antibodies comprising such heavy chain variable region and light chain variable region pairs preferably specifically bind to IL-15 (e.g. human IL-15 complexed with IL-15Rα).

The antibodies may comprise a heavy chain variable region comprising the amino acid sequence of SEQ ID NO: 4, and a light chain variable region comprising the amino acid sequence of SEQ ID NO: 2. The antibodies may comprise a heavy chain variable region comprising the amino acid sequence of SEQ ID NO: 4, and a light chain variable region comprising the amino acid sequence of SEQ ID NO: 5. The antibodies may comprise a heavy chain variable region comprising the amino acid sequence of SEQ ID NO: 4, and a light chain variable region comprising the amino acid sequence of SEQ ID NO: 8. The antibodies may comprise a heavy chain variable region comprising the amino acid sequence of SEQ ID NO: 4, and a light chain variable region comprising the amino acid sequence of SEQ ID NO: 455. The antibodies may comprise a heavy chain variable region comprising the amino acid sequence of SEQ ID NO: 4, and a light chain variable region comprising the amino acid sequence of SEQ ID NO: 457. The antibodies may comprise a heavy chain variable region comprising the amino acid sequence of SEQ ID NO: 4, and a light chain variable region comprising the amino acid sequence of SEQ ID NO: 459. The antibodies may comprise a heavy chain variable region comprising the amino acid sequence of SEQ ID NO: 4, and a light chain variable region comprising the amino acid sequence of SEQ ID NO: 503. The antibodies may comprise a heavy chain variable region comprising the amino acid sequence of SEQ ID NO: 4, and a light chain variable region comprising the amino acid sequence of SEQ ID NO: 505. The antibodies may comprise a heavy chain variable region comprising the amino acid sequence of SEQ ID NO: 4, and a light chain variable region comprising the amino acid sequence of SEQ ID NO: 507. The antibodies may comprise a heavy chain variable region comprising the amino acid sequence of SEQ ID NO: 4, and a light chain variable region comprising the amino acid sequence of SEQ ID NO: 509. The antibodies may comprise a heavy chain variable region comprising the amino acid sequence of SEQ ID NO: 4, and a light chain variable region comprising the amino acid sequence of SEQ ID NO: 510. The antibodies may comprise a heavy chain variable region comprising the amino acid sequence of SEQ ID NO: 454, and a light chain variable region comprising the amino acid sequence of SEQ ID NO: 8. The antibodies may comprise a heavy chain variable region comprising the amino acid sequence of SEQ ID NO: 454, and a light chain variable region comprising the amino acid sequence of SEQ ID NO: 455. The antibodies may comprise a heavy chain variable region comprising the amino acid sequence of SEQ ID NO: 454, and a light chain variable region comprising the amino acid sequence of SEQ ID NO: 503. The antibodies may comprise a heavy chain variable region comprising the amino acid sequence of SEQ ID NO: 454, and a light chain variable region comprising the amino acid sequence of SEQ ID NO: 457. The antibodies may comprise a heavy chain variable region comprising the amino acid sequence of SEQ ID NO: 454, and a light chain variable region comprising the amino acid sequence of SEQ ID NO: 505. The antibodies may comprise a heavy chain variable region comprising the amino acid sequence of SEQ ID NO: 454, and a light chain variable region comprising the amino acid sequence of SEQ ID NO: 506. The antibodies may comprise a heavy chain variable region comprising the amino acid sequence of SEQ ID NO: 454, and a light chain variable region comprising the amino acid sequence of SEQ ID NO: 507. The antibodies may comprise a heavy chain variable region comprising the amino acid sequence of SEQ ID NO: 454, and a light chain variable region comprising the amino acid sequence of SEQ ID NO: 5. The antibodies may comprise a heavy chain variable region comprising the amino acid sequence of SEQ ID NO: 454, and a light chain variable region comprising the amino acid sequence of SEQ ID NO: 509. The antibodies may comprise a heavy chain variable region comprising the amino acid sequence of SEQ ID NO: 454, and a light chain variable region comprising the amino acid sequence of SEQ ID NO: 510. The antibodies may comprise a heavy chain variable region comprising the amino acid sequence of SEQ ID NO: 7, and a light chain variable region comprising the amino acid sequence of SEQ ID NO: 8. Antibodies comprising such heavy chain variable region and light chain variable region pairs preferably specifically bind to IL-15 (e.g. human IL-15 complexed with IL-15Rα).

The antibodies may comprise a heavy chain variable region comprising the amino acid sequence of SEQ ID NO: 1, and a light chain comprising the amino acid sequence of SEQ ID NO: 3. The antibodies may comprise a heavy chain variable region comprising the amino acid sequence of SEQ ID NO: 1, and a light chain comprising the amino acid sequence of SEQ ID NO: 6. The antibodies may comprise a heavy chain variable region comprising the amino acid sequence of SEQ ID NO: 1, and a light chain comprising the amino acid sequence of SEQ ID NO: 9. The antibodies may comprise a heavy chain variable region comprising the amino acid sequence of SEQ ID NO: 1, and a light chain comprising the amino acid sequence of SEQ ID NO: 456. The antibodies may comprise a heavy chain variable region comprising the amino acid sequence of SEQ ID NO: 1, and a light chain comprising the amino acid sequence of SEQ ID NO: 458. The antibodies may comprise a heavy chain variable region comprising the amino acid sequence of SEQ ID NO: 1, and a light chain comprising the amino acid sequence of SEQ ID NO: 460. Antibodies comprising such heavy chain variable region and light chain pairs preferably specifically bind to IL-15 (e.g. human IL-15 complexed with IL-15Rα).

The antibodies may comprise a heavy chain variable region comprising the amino acid sequence of SEQ ID NO: 4, and a light chain comprising the amino acid sequence of SEQ ID NO: 3. The antibodies may comprise a heavy chain variable region comprising the amino acid sequence of SEQ ID NO: 4, and a light chain comprising the amino acid sequence of SEQ ID NO: 6. The antibodies may comprise a heavy chain variable region comprising the amino acid sequence of SEQ ID NO: 4, and a light chain comprising the amino acid sequence of SEQ ID NO: 9. The antibodies may comprise a heavy chain variable region comprising the amino acid sequence of SEQ ID NO: 4, and a light chain comprising the amino acid sequence of SEQ ID NO: 456. The antibodies may comprise a heavy chain variable region comprising the amino acid sequence of SEQ ID NO: 4, and a light chain comprising the amino acid sequence of SEQ ID NO: 458. The antibodies may comprise a heavy chain variable region comprising the amino acid sequence of SEQ ID NO: 4, and a light chain comprising the amino acid sequence of SEQ ID NO: 460. The antibodies may comprise a heavy chain variable region comprising the amino acid sequence of SEQ ID NO: 454, and a light chain comprising the amino acid sequence of SEQ ID NO: 9. Antibodies comprising such heavy chain variable region and light chain pairs preferably specifically bind to IL-15 (e.g. human IL-15 complexed with IL-15Rα).

In some aspects, the antibodies specifically bind to IL-15 (e.g. human IL-15 complexed with IL-15Rα), and comprise a heavy chain variable region comprising the amino acid sequence of any one of, SEQ ID NO: 461, SEQ ID NO: 462, SEQ ID NO: 463, SEQ ID NO: 464, SEQ ID NO: 465, SEQ ID NO: 466, SEQ ID NO: 467, SEQ ID NO: 468, SEQ ID NO: 469, SEQ ID NO: 470, SEQ ID NO: 471, SEQ ID NO: 472, SEQ ID NO: 473, SEQ ID NO: 474, SEQ ID NO: 475, SEQ ID NO: 476, SEQ ID NO: 477, SEQ ID NO: 478, SEQ ID NO: 479, SEQ ID NO: 480, SEQ ID NO: 481, SEQ ID NO: 482, SEQ ID NO: 483, SEQ ID NO: 484, SEQ ID NO: 485, SEQ ID NO: 486, SEQ ID NO: 487, SEQ ID NO: 488, SEQ ID NO: 489, or SEQ ID NO: 490, and a light chain variable region or a light chain. In some aspects, the antibodies specifically bind to IL-15 (e.g. human IL-15 complexed with IL-15Rα), and comprise a heavy chain variable region comprising the amino acid sequence of any one of SEQ ID NO: 461, SEQ ID NO: 462, SEQ ID NO: 463, SEQ ID NO: 464, SEQ ID NO: 465, SEQ ID NO: 466, SEQ ID NO: 467, SEQ ID NO: 468, SEQ ID NO: 469, SEQ ID NO: 470, SEQ ID NO: 471, SEQ ID NO: 472, SEQ ID NO: 473, SEQ ID NO: 474, SEQ ID NO: 475, SEQ ID NO: 476, SEQ ID NO: 477, SEQ ID NO: 478, SEQ ID NO: 479, SEQ ID NO: 480, SEQ ID NO: 481, SEQ ID NO: 482, SEQ ID NO: 483, SEQ ID NO: 484, SEQ ID NO: 485, SEQ ID NO: 486, SEQ ID NO: 487, SEQ ID NO: 488, SEQ ID NO: 489, or SEQ ID NO: 490, and a light chain variable region or a light chain, and the light chain variable region may be any one of SEQ ID NO: 491, SEQ ID NO: 492, SEQ ID NO: 493, SEQ ID NO: 494, SEQ ID NO: 495, SEQ ID NO: 496, SEQ ID NO: 497, SEQ ID NO: 498, or SEQ ID NO: 499. In some aspects, the antibodies specifically bind to IL-15 (e.g. human IL-15 complexed with IL-15Rα), and comprise a light chain variable region comprising the amino acid sequence of any one of SEQ ID NO: 491, SEQ ID NO: 492, SEQ ID NO: 493, SEQ ID NO: 494, SEQ ID NO: 495, SEQ ID NO: 496, SEQ ID NO: 497, SEQ ID NO: 498, or SEQ ID NO: 499, and a heavy chain variable region. Antibodies comprising such heavy chain variable region and light chain pairs preferably specifically bind to IL-15 (e.g. human IL-15 complexed with IL-15Rα).

In some aspects, the antibodies specifically bind to IL-15 (e.g. human IL-15 complexed with IL-15Rα), and comprise a heavy chain variable region comprising the amino acid sequence of any one of SEQ ID NO:7, SEQ ID NO:454, and SEQ ID NO:4, and a light chain variable region or a light chain. In some aspects, the antibodies specifically bind to IL-15 (e.g. human IL-15 complexed with IL-15Rα), and comprise a heavy chain variable region comprising the amino acid sequence of any one of SEQ ID NO:7, SEQ ID NO:454, and SEQ ID NO:4, and a light chain variable region or a light chain, and the light chain variable region can be any one of SEQ ID NO:5, SEQ ID NO:8, SEQ ID NO:455, SEQ ID NO:457, SEQ ID NO:459, SEQ ID NO:503, SEQ ID NO:505, SEQ ID NO:506, SEQ ID NO:507, SEQ ID NO:509, or SEQ ID NO:510. In some aspects, the antibodies specifically bind to IL-15 (e.g. human IL-15 complexed with IL-15Rα), and comprise a light chain variable region comprising the amino acid sequence of any one of SEQ ID NO:5, SEQ ID NO:8, SEQ ID NO:455, SEQ ID NO:457, SEQ ID NO:459, SEQ ID NO:503, SEQ ID NO:505, SEQ ID NO:506, SEQ ID NO:507, SEQ ID NO:509, or SEQ ID NO:510 and a heavy chain variable region.

The antibodies may specifically bind to IL-15 (e.g. human IL-15 complexed with IL-15Rα), and comprise VH and VL or light chain pair comprising the amino acid sequence shown in the following table:

| | VH | VL | L |
|---|---|---|---|
| SEQ ID NO: | 7 | 8 | 9 |
| SEQ ID NO: | 454 | 8 | 9 |
| SEQ ID NO: | 4 | 455 | 456 |
| SEQ ID NO: | 4 | 457 | 458 |
| SEQ ID NO: | 4 | 459 | 460 |
| SEQ ID NO: | 4 | 5 | 6 |

The antibodies may specifically bind to IL-15 (e.g. human IL-15 complexed with IL-15Rα), and comprise VH and VL or light chain pair comprising the amino acid sequence shown in the following table:

| | VH | VL |
|---|---|---|
| SEQ ID NO: | 4 | 8 |
| SEQ ID NO: | 4 | 503 |
| SEQ ID NO: | 4 | 505 |
| SEQ ID NO: | 4 | 509 |
| SEQ ID NO: | 4 | 510 |
| SEQ ID NO: | 454 | 455 |
| SEQ ID NO: | 454 | 503 |
| SEQ ID NO: | 454 | 457 |
| SEQ ID NO: | 454 | 505 |
| SEQ ID NO: | 454 | 406 |
| SEQ ID NO: | 454 | 507 |
| SEQ ID NO: | 454 | 5 |
| SEQ ID NO: | 454 | 509 |
| SEQ ID NO: | 454 | 510 |

Any of the antibodies described or exemplified herein bind to IL-15, which is preferably human IL-15. The antibodies may bind to uncomplexed IL-15 or IL-15 when in a complex with the IL-15 receptor alpha (IL-15R-alpha or IL-15Rα) - the IL-15 complex. In some aspects, human IL-15 comprises the amino acid sequence of SEQ ID NO: 511. In some aspects, the IL-15R-alpha comprises the amino acid sequence of SEQ ID NO: 512, without the AVI and His tags. In some aspects, the IL-15R-alpha comprises the amino acid sequence of SEQ ID NO: 520.

The antibodies may have an affinity to IL-15 (e.g. human IL-15 complexed with IL-15Rα) with a dissociation constant (KD) of less than about 1 x 10⁻² M. In some embodiments, the KD is less than about 1 x 10⁻³ M. In other embodiments, the KD is less than about 1 x 10⁻⁴ M. In some embodiments, the KD is less than about 1 x 10⁻⁵ M. In still other embodiments, the KD is less than about 1 x 10⁻⁶ M. In other embodiments, the KD is less than about 1 x 10⁻⁷ M. In other embodiments, the KD is less than about 1 x 10⁻⁸ M. In other embodiments, the KD is less than about 1 x 10⁻⁹ M. In other embodiments, the KD is less than about 1 x 10⁻¹⁰ M. In still other embodiments, the KD is less than about 1 x 10⁻¹¹ M. In some embodiments, the KD is less than about 1 x 10⁻¹² M. In other embodiments, the KD is less than about 1 x 10⁻¹³ M. In other embodiments, the KD is less than about 1 x 10⁻¹⁴ M. In still other embodiments, the KD is less than about 1 x 10⁻¹⁵ M. In some aspects, the KD is less than about 1.8 x 10⁻⁹ M. In some aspects, the KD is from about 1.2 x 10⁻¹⁰ M to about 2 x 10⁻¹⁰ M. In some aspects, the KD is from about 1.3 x 10⁻¹⁰ M to about 1.9 x 10⁻¹⁰ M. In some aspects, the KD is from about 1.33 x 10⁻¹⁰ M to about 1.93 x 10⁻¹⁰ M. In some aspects, the KD is from about 1.6 x 10⁻¹⁰ M to about 1.8 x 10⁻¹⁰ M. In some aspects, the KD is about 1.7 x 10⁻¹⁰ M. Affinity values refer to those obtained by standard methodologies, including surface plasmon resonance (SPR) such as BIACORE^{®} analyses or analysis using an OCTET^{®} Red 96 (Forte Bio) Dip-and-Read system. In a preferred embodiment, the dissociation constant is determined by SPR.

In a general BIACORE^{®} SPR analysis, an antibody is immobilized on a sensor chip surface, and suitable concentrations of IL-15 or the IL-15 complexed with the IL-15 Receptor alpha are passed across the surface. Changes in the index of refraction are detected, and software is used to generate sensorgrams for analysis. Interaction between the immobilized antibody and the IL-15 or IL-15 complex may be carried out for any suitable length of time, including about 1 to about 2 minutes. The temperature of the interaction can be any suitable temperature, including about 25 degrees C.

Antibodies that bind to IL-15 (e.g. human IL-15 complexed with IL-15Rα) may be monoclonal antibodies. Preferably, the antibodies are full-length antibodies comprising a heavy chain and a light chain. In some aspects, the antibodies comprise derivatives or fragments or portions of antibodies that retain the antigen-binding specificity, and also preferably substantially retain the affinity, of the full-length parent antibody molecule (e.g., for IL-15). For example, derivatives may comprise a single variable region (either a heavy chain or light chain variable region). Other examples of suitable antibody derivatives and fragments include, without limitation, antibodies with polyepitopic specificity, diabodies, minibodies, FAb, F(Ab')2, Fd, Fabc, and Fv molecules, single chain (Sc) antibodies, single chain Fv antibodies (scFv), individual antibody light chains, individual antibody heavy chains, fusions between antibody chains and other molecules, heavy chain monomers or dimers, light chain monomers or dimers, dimers consisting of one heavy and one light chain, and other multimers. Single chain Fv antibodies may be multi-valent. Antibody derivatives, fragments, and/or portions may be recombinantly produced and expressed by any cell type, prokaryotic or eukaryotic.

In a full-length antibody, each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Typically, the antigen binding properties of an antibody are less likely to be disturbed by changes to FR sequences than by changes to the CDR sequences. Immunoglobulin molecules can be of any type (*e.g.,* IgG, IgE, IgM, IgD, IgA and IgY), class (*e.g.,* IgG1, IgG2, IgG3, IgG4, IgA1 and IgA2) or subclass.

The anti-IL-15 antibodies preferably are fully human. Fully human antibodies are those where the whole molecule is human or otherwise of human origin, or includes an amino acid sequence identical to a human form of the antibody. Fully human antibodies include those obtained from a human V gene library, for example, where human genes encoding variable regions of antibodies are recombinantly expressed. Fully human antibodies may be expressed in other organisms (*e.g*., mice and xenomouse technology) or cells from other organisms transformed with genes encoding human antibodies. Fully human antibodies may be expressed in an OMNIRAT^{®} rat system (OMT, Inc.), according to WO 08/151081. Fully human antibodies may nevertheless include amino acid residues not encoded by naturally occurring human sequences, *e.g.*, mutations introduced by random or site directed mutations.

In some aspects, the anti-IL-15 antibodies may comprise non-immunoglobulin derived protein frameworks. For example, reference may be made to (Ku et al. (1995) Proc. Natl. Acad. Sci. USA 92: 6552-6556), which describes a four-helix bundle protein cytochrome b562 having two loops randomized to create CDRs, which have been selected for antigen binding.

The anti-IL-15 antibodies may comprise post-translational modifications or moieties, which may impact antibody activity, circulating half-life, or shelf/storage stability. For example, the antibodies may be methylated, acetylated, glycosylated, sulfated, phosphorylated, carboxylated, and/or amidated, or may comprise other suitable moieties that are well known in the art. Moieties include any chemical group or combinations of groups commonly found on immunoglobulin molecules in circulation or otherwise added to antibodies by recombinant expression systems, including prokaryotic and eukaryotic expression systems.

Examples of side chain modifications contemplated by the disclosure include modifications of amino groups such as by reductive alkylation by reaction with an aldehyde followed by reduction with NaBH4; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH4.

The guanidine group of arginine residues may be modified by the formation of heterocyclic condensation products with reagents such as 2,3-butanedione, phenylglyoxal and glyoxal. The carboxyl group may be modified by carbodiimide activation via O-acylisourea formation followed by subsequent derivation, for example, to a corresponding amide. Sulfydryl groups may be modified by methods such as carboxymethylation with iodoacetic acid or iodoacetamide; performic acid oxidation to cysteic acid; formation of mixed disulfides with other thiol compounds; reaction with maleimide, maleic anhydride or other substituted maleimide; formation of mercurial derivatives using 4-chloromercuribenzoate, 4-chloromercuriphenylsulfonic acid, phenylmercury chloride, 2-chloromercuri-4-nitrophenol and other mercurials; carbamoylation with cyanate at alkaline pH. Tryptophan residues may be modified by, for example, oxidation with N-bromosuccinimide or alkylation of the indole ring with 2-hydroxy-5-nitrobenzyl bromide or sulfenyl halides. Tyrosine residues on the other hand, may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate.

The anti-IL-15 antibodies may include modifications that modulate serum half-life and biodistribution, including without limitation, modifications that modulate the antibody's interaction with the neonatal Fc receptor (FcRn), a receptor with a key role in protecting IgG from catabolism, and maintaining high serum antibody concentration. Serum half-life modulating modifications may occur in the Fc region of IgG1, IgG2, or IgG4, including the triple substitution of M252Y/S254T/T256E (numbering according to the EU numbering system (Edelman, GM et al. (1969) Proc. Natl. Acad. USA 63:78-85)), as described in U.S. Pat. No. 7,083,784. Other substitutions may occur at positions 250 and 428, *see e.g.,* U.S. Pat. No 7,217,797, as well as at positions 307, 380 and 434, *see, e.g.,* PCT Publ. No. WO 00/042072. Examples of constant domain amino acid substitutions which modulate binding to Fc receptors and subsequent function mediated by these receptors, including FcRn binding and serum half-life, are described in U.S. Publ. Nos. 2009/0142340, 2009/0068175, and 2009/0092599. Antibodies of any class may have the heavy chain C-terminal lysine omitted or removed to reduce heterogeneity (ΔK). The substitution of S228P (EU numbering) in the human IgG4 can stabilize antibody Fab-arm exchange *in vivo* (Labrin et al. (2009) Nature Biotechnol. 27:8; 767-773), and this substitution may be present at the same time as M252Y/S254T/T256E and/or ΔK modifications.

The anti-IL-15 antibodies preferably comprise human constant domains. The heavy chain constant domains preferably are human IgG1, IgG2, or IgG4 constant domains. The light chain constant domains preferably are human lambda constant domains.

Human heavy chain IgG1 constant regions that may be used with the anti-IL-15 antibodies may be selected from among human IgG1 (SEQ ID NO: 32), human IgG1 (ΔK) (SEQ ID NO: 33), human IgG1 252Y/254T/256E (SEQ ID NO: 34), human IgG1 252Y/254T/256E (ΔK) (SEQ ID NO: 35), human IgG1 L235A/G237A (SEQ ID NO: 36), human IgG1 L235A/G237A (ΔK) (SEQ ID NO: 37) human IgG1 L234A/L235A/G237A (SEQ ID NO: 38), and human IgG1 L234A/L235A/G237A (ΔK) (SEQ ID NO: 39). Human heavy chain IgG2 constant regions that may be used with the anti-IL-15 antibodies may be selected from among human IgG2 (SEQ ID NO: 40), human IgG2 (ΔK) (SEQ ID NO: 41), human IgG2 A330S/P331S (SEQ ID NO: 42), and human IgG (ΔK) (SEQ ID NO: 43). Human heavy chain IgG4 constant regions that may be used with the anti-IL-15 antibodies may be selected from among human IgG4 (SEQ ID NO: 44), human IgG4 (ΔK) (SEQ ID NO: 45), human IgG4 S228P (SEQ ID NO: 46), human IgG4 S228P (ΔK) (SEQ ID NO: 47), human IgG4 228P/252Y/254T/256E (SEQ ID NO: 48), human IgG4 228P/252Y/254T/256E (ΔK) (SEQ ID NO: 49), human IgG4 252Y/254T/256E (SEQ ID NO: 50), and human IgG4 252Y/254T/256E (ΔK) (SEQ ID NO: 51).

The anti-IL-15 antibodies may be labelled, bound, or conjugated to any chemical or biomolecule moieties. Labelled antibodies may find use in therapeutic, diagnostic, or basic research applications. Such labels/conjugates can be detectable, such as fluorochromes, electrochemiluminescent probes, quantum dots, radiolabels, enzymes, fluorescent proteins, and luminescent proteins, or may comprise biotin or PEG.

The antibodies may be derivatized by known protecting/blocking groups to prevent proteolytic cleavage or enhance activity or stability.

Polynucleotide sequences that encode the anti-IL-15 antibodies, their domains (*e.g*., VH and VL domains), and their subdomains (*e.g.*, FRs and CDRs) are featured in the disclosure. Polynucleotides include, but are not limited to, RNA, DNA, cDNA, hybrids of RNA and DNA, and single, double, or triple stranded strands of RNA, DNA, or hybrids thereof. The complementary nucleic acid sequences are also within the scope of the disclosure.

In some aspects, a polynucleotide comprises a first nucleic acid sequence encoding an antibody heavy chain variable region comprising the amino acid sequence of SEQ ID NO: 1, SEQ ID NO: 4, or SEQ ID NO: 7. The polynucleotide may further comprise a second nucleic acid sequence encoding an antibody light chain variable region comprising the amino acid sequence of SEQ ID NO: 2, SEQ ID NO: 5, or SEQ ID NO: 8. The polynucleotide may further comprise a second nucleic acid sequence encoding an antibody light chain comprising the amino acid sequence of SEQ ID NO: 3, SEQ ID NO: 6, or SEQ ID NO: 9. The polynucleotide may further comprise a third nucleic acid sequence encoding an antibody heavy chain constant region, such as any of the IgG1, IgG2, or IgG4 constant regions described herein.

In some aspects, a polynucleotide comprises a first nucleic acid sequence encoding an antibody heavy chain variable region comprising the amino acid sequence of SEQ ID NO: 4, SEQ ID NO: 7, or SEQ ID NO:454. The polynucleotide may further comprise a second nucleic acid sequence encoding an antibody light chain variable region comprising the amino acid sequence of SEQ ID NO: 5, SEQ ID NO: 8, SEQ ID NO:455, SEQ ID NO:457, SEQ ID NO:459, SEQ ID NO:503, SEQ ID NO:505, SEQ ID NO:506, SEQ ID NO:507, SEQ ID NO:509, or SEQ ID NO:510. The polynucleotide may further comprise a second nucleic acid sequence encoding an antibody light chain comprising the amino acid sequence of SEQ ID NO: 6, SEQ ID NO: 9, SEQ ID NO:456, SEQ ID NO:458, or SEQ ID NO:460. The polynucleotide may further comprise a third nucleic acid sequence encoding an antibody heavy chain constant region, such as any of the IgG1, IgG2, or IgG4 constant regions described herein.

In some aspects, a polynucleotide comprises a first nucleic acid sequence encoding an antibody light chain variable region comprising the amino acid sequence of SEQ ID NO: 2, SEQ ID NO: 5, or SEQ ID NO: 8. In some aspects, a polynucleotide comprises a first nucleic acid sequence encoding an antibody light chain comprising the amino acid sequence of SEQ ID NO: 3, SEQ ID NO: 6, or SEQ ID NO: 9. In some aspects, a polynucleotide comprises the nucleic acid sequence of SEQ ID NO: 517. In some aspects, a polynucleotide comprises the nucleic acid sequence of SEQ ID NO: 518.

In some aspects, a polynucleotide comprises a first nucleic acid sequence encoding an antibody light chain variable region comprising the amino acid sequence of SEQ ID NO: 5, SEQ ID NO: 8, SEQ ID NO:455, SEQ ID NO:457, SEQ ID NO:459, SEQ ID NO:503, SEQ ID NO:505, SEQ ID NO:506, SEQ ID NO:507, SEQ ID NO:509, or SEQ ID NO:510. In some aspects, a polynucleotide comprises a first nucleic acid sequence encoding an antibody light chain comprising the amino acid sequence of SEQ ID NO: 6, SEQ ID NO: 9, SEQ ID NO:456, SEQ ID NO:458, or SEQ ID NO:460.

Any such polynucleotides may be comprised within a vector. Thus, vectors comprising polynucleotides are provided as part of the disclosure. The vectors may be expression vectors. Recombinant expression vectors containing a sequence encoding a polypeptide of interest are thus provided. The expression vector may contain one or more additional sequences, such as but not limited to regulatory sequences, a selection marker, a purification tag, or a polyadenylation signal. Such regulatory elements may include a transcriptional promoter, enhancers, mRNA ribosomal binding sites, or sequences that control the termination of transcription and translation.

Expression vectors, especially mammalian expression vectors, may include one or more nontranscribed elements, such as an origin of replication, a suitable promoter and enhancer linked to the gene to be expressed, other 5' or 3' flanking nontranscribed sequences, 5' or 3' nontranslated sequences (such as necessary ribosome binding sites), a polyadenylation site, splice donor and acceptor sites, or transcriptional termination sequences. An origin of replication that confers the ability to replicate in a specific host may also be incorporated.

The vectors may be used to transform any of a wide array of host cells well known to those of skill in the art, and preferably host cells capable of expressing antibodies. Vectors include without limitation, plasmids, phagemids, cosmids, bacmids, bacterial artificial chromosomes (BACs), yeast artificial chromosomes (YACs), and baculovirus, as well as other bacterial, eukaryotic, yeast, and viral vectors. Suitable host cells include without limitation CHO cells, NS0 cells, HEK293 cells, or any eukaryotic stable cell line known or produced, and also include bacteria, yeast, and insect cells.

The antibodies may also be produced by hybridoma cells; methods to produce hybridomas being well known and established in the art.

The disclosure also provides compositions comprising the anti-IL-15 antibodies. The compositions may comprise any of the antibodies described and/or exemplified herein and an acceptable carrier such as a pharmaceutically acceptable carrier. Suitable carriers include any media that does not interfere with the biological activity of the antibody and preferably is not toxic to a host to which it is administered. The carrier may be an aqueous solution. The compositions may comprise any of the antibodies described and/or exemplified herein and a pharmaceutically acceptable excipient.

The anti-IL-15 antibodies may be used to treat an autoimmune disease, including an autoimmune disease in which IL-15 is dysregulated. The anti-IL-15 antibodies may be used to treat an inflammatory disease, including an inflammatory disease in which IL-15 is dysregulated. The anti-IL-15 antibodies may be used to treat an inflammatory disorder, including an inflammatory disorder in which IL-15 is dysregulated. In some aspects, the anti-IL-15 antibodies may be used to treat Celiac disease, refractory Celiac disease, rheumatoid arthritis, psoriasis, inflammatory bowel disease, type 1 diabetes, alopecia areata as well as certain type of cancer such as T cell large granular lymphocytic leukemia in a subject. Thus, the disclosure features treatment methods.

In some aspects, the treatment methods comprise administering an anti-IL-15 antibody, or composition thereof, to a subject in need of treatment for an autoimmune disease in which IL-15 is dysregulated, such that the autoimmune disease is treated in the subject. The anti-IL-15 antibodies are preferably administered in an amount that is effective to treat the autoimmune disease in which IL-15 is dysregulated in the subject. The effective amount may vary, for example, according to the needs or condition of the subject. Administration may be at the direction or control of a medical practitioner.

In some aspects, the treatment methods comprise administering an anti-IL-15 antibody, or composition thereof, to a subject in need of treatment for an inflammatory disease in which IL-15 is dysregulated, such that the inflammatory disease is treated in the subject. The anti-IL-15 antibodies are preferably administered in an amount that is effective to treat the inflammatory disease in which IL-15 is dysregulated in the subject. The effective amount may vary, for example, according to the needs or condition of the subject. Administration may be at the direction or control of a medical practitioner.

In some aspects, the treatment methods comprise administering an anti-IL-15 antibody, or composition thereof, to a subject in need of treatment for an inflammatory disorder in which IL-15 is dysregulated, such that the inflammatory disorder is treated in the subject. The anti-IL-15 antibodies are preferably administered in an amount that is effective to treat the inflammatory disorder in which IL-15 is dysregulated in the subject. The effective amount may vary, for example, according to the needs or condition of the subject. Administration may be at the direction or control of a medical practitioner.

In some aspects, the treatment methods comprise administering an anti-IL-15 antibody, or composition thereof, to a subject in need of treatment for Celiac disease, such that Celiac disease is treated in the subject, and the Celiac disease may be refractory. Refractory celiac disease (RCD) affects patients who have failed to heal and demonstrate continual symptoms of celiac disease, after 6-12 months of a strict gluten-free diet and when other causes of symptoms (including malignancy) have been ruled out. It may also occur in patients who previously responded to a long-term gluten-free diet, but who now display symptoms of celiac disease, while maintaining a strict gluten free diet (Rishi et al. Expert Review of Gastroenterology & Hepatology:10 537-546 (2016)). The anti-IL-15 antibodies are preferably administered in an amount that is effective to treat Celiac disease in the subject. The effective amount may vary, for example, according to the needs or condition of the subject. Administration may be at the direction or control of a medical practitioner.

The anti-IL-15 antibodies may be used to treat or inhibit one or more symptoms of gluten exposure, for example, as caused by ingestion of gluten. The one or more symptoms include muscle pain, body pain, joint pain, fatigue, bloating, gas, nausea, cramps, constipation, diarrhea, skin rash, headache, migraine headache, depression, anxiety, brain fog, and irritability. In general, the methods comprise administering an anti-IL-15 antibody, or composition thereof, to a subject having gluten sensitivity who has been exposed to gluten such that the one or more symptoms of gluten exposure are inhibited or treated in the subject. The anti-IL-15 antibodies are preferably administered in an amount that is effective to treat or inhibit the one or more symptom of gluten exposure in the subject. The effective amount may vary, for example, according to the needs or condition of the subject. Administration may be at the direction or control of a medical practitioner.

In some aspects, the treatment methods comprise administering an anti-IL-15 antibody, or composition thereof, to a subject in need of treatment for rheumatoid arthritis, such that rheumatoid arthritis is treated in the subject. The anti-IL-15 antibodies are preferably administered in an amount that is effective to treat rheumatoid arthritis in the subject. The effective amount may vary, for example, according to the needs or condition of the subject. Administration may be at the direction or control of a medical practitioner.

In some aspects, the treatment methods comprise administering an anti-IL-15 antibody, or composition thereof, to a subject in need of treatment for psoriasis, such that psoriasis is treated in the subject. The anti-IL-15 antibodies are preferably administered in an amount that is effective to treat psoriasis in the subject. The effective amount may vary, for example, according to the needs or condition of the subject. Administration may be at the direction or control of a medical practitioner.

In some aspects, the treatment methods comprise administering an anti-IL-15 antibody, or composition thereof, to a subject in need of treatment for inflammatory bowel disease, such that inflammatory bowel disease is treated in the subject. The anti-IL-15 antibodies are preferably administered in an amount that is effective to treat inflammatory bowel disease in the subject. The effective amount may vary, for example, according to the needs or condition of the subject. Administration may be at the direction or control of a medical practitioner.

In some aspects, the treatment methods comprise administering an anti-IL-15 antibody, or composition thereof, to a subject in need of treatment for type 1 diabetes, such that type 1 diabetes is treated in the subject. The anti-IL-15 antibodies are preferably administered in an amount that is effective to treat type 1 diabetes in the subject. The effective amount may vary, for example, according to the needs or condition of the subject. Administration may be at the direction or control of a medical practitioner.

In some aspects, the treatment methods comprise administering an anti-IL-15 antibody, or composition thereof, to a subject in need of treatment for alopecia areata, such that alopecia areata is treated in the subject. The anti-IL-15 antibodies are preferably administered in an amount that is effective to treat alopecia areata in the subject. The effective amount may vary, for example, according to the needs or condition of the subject. Administration may be at the direction or control of a medical practitioner.

In some aspects, the treatment methods comprise administering an anti-IL-15 antibody, or composition thereof, to a subject in need of treatment for T cell large granular lymphocytic leukemia, such that T cell large granular lymphocytic leukemia is treated in the subject. The anti-IL-15 antibodies are preferably administered in an amount that is effective to treat T cell large granular lymphocytic leukemia in the subject. The effective amount may vary, for example, according to the needs or condition of the subject. Administration may be at the direction or control of a medical practitioner.

The anti-IL-15 antibodies may be used in the manufacture of a medicament. For example, the anti-IL-15 antibodies may be used in the manufacture or preparation of a medicament for use in the treatment of Celiac disease. The anti-IL-15 antibodies may be used in the manufacture or preparation of a medicament for use in the treatment of refractory Celiac disease. The anti-IL-15 antibodies may be used in the manufacture or preparation of a medicament for use in the treatment of rheumatoid arthritis. The anti-IL-15 antibodies may be used in the manufacture or preparation of a medicament for use in the treatment of psoriasis. The anti-IL-15 antibodies may be used in the manufacture or preparation of a medicament for use in the treatment of inflammatory bowel disease. The anti-IL-15 antibodies may be used in the manufacture or preparation of a medicament for use in the treatment of type 1 diabetes. The anti-IL-15 antibodies may be used in the manufacture or preparation of a medicament for use in the treatment of alopecia areata. The anti-IL-15 antibodies may be used in the manufacture or preparation of a medicament for use in the treatment of T cell large granular lymphocytic leukemia. The anti-IL-15 antibodies may be used in the manufacture or preparation of a medicament for use in the treatment or inhibition of one or more symptoms of gluten exposure, for example, gluten exposure in a patient who has a gluten sensitivity or allergy. The one or more symptoms may include muscle pain, body pain, joint pain, fatigue, bloating, gas, nausea, cramps, constipation, diarrhea, skin rash, headache, migraine headache, depression, anxiety, brain fog, and/or irritability.

The anti-IL-15 antibodies may be for use in the treatment of Celiac disease. The anti-IL-15 antibodies may be for use in the treatment of refractory Celiac disease. The anti-IL-15 antibodies may be for use in the treatment of rheumatoid arthritis. The anti-IL-15 antibodies may be for use in the treatment of psoriasis. The anti-IL-15 antibodies may be for use in the treatment of inflammatory bowel disease. The anti-IL-15 antibodies may be for use in the treatment of type 1 diabetes. The anti-IL-15 antibodies may be for use in the treatment of alopecia areata. The anti-IL-15 antibodies may be for use in the treatment of T cell large granular lymphocytic leukemia. The anti-IL-15 antibodies may be for use in the treatment or inhibition of one or more symptoms of gluten exposure, for example, gluten exposure in a patient who has a gluten sensitivity or allergy. The one or more symptoms may include muscle pain, body pain, joint pain, fatigue, bloating, gas, nausea, cramps, constipation, diarrhea, skin rash, headache, migraine headache, depression, anxiety, brain fog, and/or irritability.

The disclosure also features kits comprising any of the anti-IL-15 antibodies, and these kits may be used to supply antibodies and other agents for use in diagnostic, basic research, or therapeutic methods, among others. In some aspects, the kits comprise any anti-IL-15 antibody described or exemplified herein and instructions for using the antibody in a method for treating Celiac disease. In some aspects, the kits comprise any anti-IL-15 antibody described or exemplified herein and instructions for using the antibody in a method for treating refractory Celiac disease. In some aspects, the kits comprise any anti-IL-15 antibody described or exemplified herein and instructions for using the antibody in a method for treating or inhibiting one or more symptoms of gluten exposure, for example, in a patient with a gluten sensitivity or allergy. In some aspects, the kits comprise any anti-IL-15 antibody described or exemplified herein and instructions for using the antibody in a method for treating rheumatoid arthritis. In some aspects, the kits comprise any anti-IL-15 antibody described or exemplified herein and instructions for using the antibody in a method for treating psoriasis. In some aspects, the kits comprise any anti-IL-15 antibody described or exemplified herein and instructions for using the antibody in a method for treating inflammatory bowel disease. In some aspects, the kits comprise any anti-IL-15 antibody described or exemplified herein and instructions for using the antibody in a method for treating type 1 diabetes. In some aspects, the kits comprise any anti-IL-15 antibody described or exemplified herein and instructions for using the antibody in a method for treating alopecia areata. In some aspects, the kits comprise any anti-IL-15 antibody described or exemplified herein and instructions for using the antibody in a method for treating T cell large granular lymphocytic leukemia.

Also provided are methods for detecting IL-15 in a tissue sample isolated from a subject. Generally, such methods comprising contacting any anti-IL-15 antibody described or exemplified herein with a tissue sample isolated from a subject to form an antibody-IL-15 complex with the IL-15 Receptor-alpha, and detecting the complex in the tissue sample. The method may further comprise isolating the tissue sample from the subject. The tissue sample may be from gastrointestinal tissue, including esophageal tissue, stomach tissue, small intestine tissue, large intestine tissue, and other tissue from the gastrointestinal tract. The antibody may be conjugated to a detectable label. The antibody may be detected with a secondary antibody that is labelled with a detectable label. Such methods may be carried out *in vivo, in vitro,* or *in situ.*

Also provided are methods for detecting a complex of IL-15 and IL-15 Receptor-alpha in a tissue sample isolated from a subject. Generally, such methods comprising contacting any anti-IL-15 antibody described or exemplified herein with a tissue sample isolated from a subject to form an antibody-antigen complex of the anti-IL-15 antibody bound to a complex of IL-15 and IL-15 Receptor-alpha, and detecting the antibody-antigen complex in the tissue sample. The method may further comprise isolating the tissue sample from the subject. The tissue sample may be from gastrointestinal tissue, including esophageal tissue, stomach tissue, small intestine tissue, large intestine tissue, and other tissue from the gastrointestinal tract. The antibody may be conjugated to a detectable label. The antibody may be detected with a secondary antibody that is labelled with a detectable label. Such methods may be carried out *in vivo, in vitro,* or *in situ.*

The following examples are provided to describe the disclosure in greater detail. They are intended to illustrate, not to limit, the disclosure. In the examples, reference to a position of a residue is a reference to the position in the relevant sequence as set forth herein, unless otherwise indicated.

### Example 1

### Generation of transgenic rats, immunization and production of hybridomas

### 1.1 IL-15 Protein and IL-15Rα Protein

Human Interleukin 15 (IL-15) was purchased (Sigma) or produced in a mammalian HEK293F expression system, using plasmids encoding human IL-15 and the soluble IL-15 receptor α (IL-15Rα) with an N-terminally located HIS and AVI tag (SEQ ID NO: 512) in a 1:1 ratio.

### 1.2 Generation of transgenic rats

Transgenic rats were generated as described in PCT Publication No. WO 08/151081. In brief, a meganuclease expression construct was integrated into the genome of a subject animal. Expression of the meganuclease in germ cells resulted in double-strand breaks in endogenous rat immunoglobulin genes. Mating of such transgenic rats resulted in offspring with mutated/inactivated endogenous rat immunoglobulin genes.

The transgenic rats are further modified to carry artificial human immunoglobulin genes such that the rats are capable of producing antibodies with fully human variable regions.

### 1.3 Immunizations

To generate fully human monoclonal antibodies to the IL-15 complex with the IL-15 Receptor-alpha, transgenic rats (generated as described above) were immunized with DNA encoding human IL-15 with and DNA encoding human IL-15Rα.

Ten animals were immunized and the immune response was monitored over the course of immunization with plasma samples obtained by submandibular bleeds. The plasma was screened for antibody expression by ELISA, and animals with sufficient titers of anti-IL-15 antibodies were selected for fusion and hybridoma generation. Animals with a high titer were boosted subcutaneously with recombinant human IL-15 complex 5 days before sacrifice.

### 1.4 Generation of hybridomas producing monoclonal antibodies to IL-15 complex

To generate hybridomas producing monoclonal antibodies to the IL-15 complex with the IL-15 Receptor-alpha, splenocytes and lymph node cells from immunized animals were isolated and fused to an immortalized cell line. Single cell suspensions of lymphocytes were fused to P3X63Ag8.653 non secreting mouse myeloma cells (ATCC, CRL-1580). Cells were plated at approximately 1x10⁵ cells/mL in flat bottom microtiter plates, followed by 2 week incubation in selective medium containing, besides usual reagents, 10% fetal clone serum and 1x HAT (Sigma). Individual wells were then screened by ELISA and BIACORE^{®} for human IL-15 IgG antibodies with high affinity.

### Example 2

### Screening of hybridomas

### 2.1 Use of ELISA to select antibodies that bind to the IL-15 complex but do not bind to uncomplexed IL-15 receptor α

Microtiter plates were coated with purified IL-15 or purified IL-15Rα or purified IL-15 complex. Briefly, microtiter plates were coated with purified protein in PBS and then blocked with irrelevant proteins such as bovine serum albumin (BSA) diluted in PBS. Dilutions of hybridoma supernatants were added to each well and incubated for 1-2 hours at 37° C. The plates were washed with PBS/TWEEN^{®} 20, and then incubated with a goat-anti-human IgG Fc-specific polyclonal reagent conjugated to a suitable detection reagent (*e.g*., horseradish peroxidase) alkaline phosphatase for 1 hour at 37° C. After washing, the plates were developed with suitable substrate (*e.g*., 3,3',5,5'-tetramethylbenzidine TMD) and analyzed at OD of 405. Hybridomas that produced antibodies showing positive reactivity with the IL-15 complex but not with IL-15Rα were selected for further characterization.

### 2.2 Use of cell based ELISA (cELISA) to select antibodies that bind to the IL-15 complex but do not bind to uncomplexed IL-15 receptor α

Each of the hybridomas tested as above were also tested in a cell-based enzyme-linked immunosorbent assay (cELISA) to select for antibodies that bind to the IL-15 complex but do not bind to uncomplexed IL-15Rα.

cELISA was carried out as follows. HEK cells were transfected with DNA encoding IL-15 and IL-15Rα such that they expressed the IL-15 complex. The transfected HEK cells were coated onto an ELISA plate and dilutions of hybridoma supernatants was applied to the plate so that it could bind to the IL-15 complex expressed on the surface of the cells. The assay was repeated using HEK cells transfected with IL-15Rα such that they expressed uncomplexed IL-15Rα. The advantage of using cELISA in addition to classical ELISA is that a native protein complex is used to screen the antibodies.

As a positive control, cell surface expression of the IL-15 complex or uncomplexed IL-15Rα was analysed using an anti-human IL-15-Phycoerythrin (PE) antibody (R&D Systems, Cat. No. IC2471P). Hybridomas producing antibodies that showed positive reactivity with the IL-15 complex but not with IL-15Rα were selected for further characterization.

A representative selection of results in shown in Figure 1. Antibody 4 bound to uncomplexed IL-15 and the IL-15 complex, but not to uncomplexed IL15Rα. Antibody 1A6 did not bind to uncomplexed IL-15, the IL-15 complex or, IL-15Rα, and was not selected for further characterization. Antibody 1B3 bound uncomplexed IL-15, the IL-15 complex and IL-15Rα. Binding to uncomplexed IL-15Rα is disadvantageous because it indicates that the clones are not IL-15 specific.

### Example 3

### Identification of Candidate Antibodies for Further Development

### 3.1 CTLL-2 cell based assay

1500 hybridoma samples that bound IL-15 complex but not to IL-15α were tested in a murine CTLL-2 cell-based assay to determine which neutralize the biological activity of IL-15. The CTLL-2 cell line is derived from cytotoxic T cell lymphoma (ATCC: TIB-214) and is responsive to both IL-2 and IL-15.

Hybridoma supernatants (unpurified antibodies) were tested for their ability to neutralize IL-15-induced proliferation of CTLL-2 cells.

CTLL-2 cells were incubated in complete media without IL-2 or IL-15 for 4 hours prior testing. CTLL-2 cells (5x10⁴/well) were incubated in 96-well plates with IL-15 complex with the IL-15 Receptor-alpha at 200 pM to induce cell proliferation. Hybridoma supernatants were added to the plates and incubated for 48 hours. Inhibition of cell proliferation was then assessed using the CELLTITER-GLO^{®} Luminescent Cell Viability Assay (Promega) according to manufacturer's instructions and read on GLOMAX^{®} 96 Microplate Luminometer (Promega). Data not shown.

### 3.2 BIACORE^{®} assays

In parallel to the cell based assays described above, the 1500 hybridomas were also tested for IL-15 complex binding activity and their affinity was measured. A surface plasmon resonance (SPR) assay was used. SPR screening was conducted using a BIACORE^{®} 4000 Biosensor (GE Healthcare) in a single concentration analyte pass assay. CM5 Series S (GE Healthcare) was docked in the machine. Normalization with Bia Normalisation solution (GE Healthcare) was used. Hydrodynamic addressing was performed on the docked chip and passed the internal quality control check.

An anti-rat Fc fragment antibody (Bethyl A110-136A) was immobilised on the surface of a CM5 sensor chip using an amine coupling kit (GE Healthcare). The antibody was diluted in sodium acetate pH 4.5 to a concentration of 50 µg/mL for immobilisation and was immobilised on Spots 1, 2, 4, 5 on flow cells 1-4 using the HBS-EP+ buffer (GE Healthcare) and a coupling time of 10 minutes. All interactions were measured at 25 °C. This resulted in an immobilisation level of between 10,000-12,000 response units for each spot on the four flow cells. Cells were regenerated using a 100 mM phosphoric acid buffer.

For hybridoma binding assessment, 70 µL of HBS-EP+ running buffer was added to 50 µL of rat hybridoma supernatant. The followed method was used:
Startup - Regeneration 3 cycles 10 secs each at 30 µL /min
Sample Run:
   Capture - Spot 1 - Flow cells 1-4 - 130 secs injection - 30 µL /min - normal injection - 4 different samples are loaded in this step onto spot 1 from each of the four flow cells Capture - Spot 5 - Flow cells 1-4 - 130 secs injection - 30 µL /min - normal injection - 4 different samples are loaded in this step onto spot 1 from each of the four flow cells Sample - All spots, All flow cells - 60 secs injection, 60 secs off-rate - 30 µL /min - normal injection - human IL-15 complex (20 µg/mL ; batch 491p90A) is injected over all flow cells and all spots.
Regeneration 1- 20 sec 100 mM phosphoric acid
Regeneration 2 - 15 sec 100 mM phosphoric acid
Regeneration 3 - 10 sec 100 mM phosphoric acid
Between every 96-well plate another regeneration cycle was performed -as per the startup cycle.

### Analysis

Using the BiaEvaluation software, capture with kinetics was used for analysis. Sensorgrams for 5-4 and 1-2 were analyzed. This allowed for the IL-15 complex signal to be subtracted from a spot that contained no antibody. Spot 3 was not used in the analysis. Each sensorgram was then analyzed and those samples that displayed no binding of antibody to complex were rejected and those that showed binding to the IL-15 complex with the IL-15 Receptor-alpha were approved. Curve fitting was performed and a table of the affinity measurements was obtained.

### 3.3 Variable Region Sequencing

The molecular identity of the antibody variable regions in the selected non-clonal hybridoma pellets was established by reverse transcription polymerase chain reaction.

Briefly, 96 well plates containing hybridoma pellets were defrosted following cryopreservation at -80°C in RNALATER^{®} (Thermo). Plates were spun at 1000 x g for 5 minutes to pellet the cells and the RNALATER^{®} buffer removed. RNA was isolated from the plates of hybridomas using a GENELUTE^{™} 96 well total RNA purification kit (Sigma #RTN9602, RTN9604) according to the manufacturer's protocol. The concentration and quality of the resulting RNA samples were determined using a NANODROP^{®} 8000 spectrophotometer (Thermo). RNA was reverse transcribed into cDNA using an oligo(dT) primer and AccuScript reverse transcriptase (Agilent #600184). The cDNA synthesis reaction was assembled according the manufacturer's protocol and cDNA synthesis carried out at 42°C for 30 minutes.

Amplification of human antibody variable regions from the transgenic rodent derived hybridomas was performed by PCR using either PfuUltrall (Agilent) or Q5 high fidelity DNA polymerases (NEB) according to the manufacturer's directions. Heavy chains were amplified using primer pairs specific to the rodent heavy chain constant region DNA sequence and the DNA sequences of the human heavy chain leader sequences. Lambda light chain variable regions were similarly amplified using primer pairs specific to the human lambda constant region DNA sequence and the DNA sequences of the human lambda chain leader sequences.

Successful amplification of the variable regions was confirmed by running a small aliquot of the PCR reaction on a gel using an e-gel electrophoresis system (Thermo). Post-PCR clean-up of the reactions was performed using a GENELUTE^{™} 96 well PCR clean-up system (Sigma #PCR9604) according to the manufacturer's protocol. The concentration of the resulting purified DNA was assessed using a Nanodrop spectrophotometer. Sanger sequencing of the PCR fragments was performed using oligos designed to bind internally to the heavy- or light chain amplicons. The resulting DNA sequences were conceptually translated into amino acid sequences for further analysis prior to their use in full length antibody chain generation. Antibody variable regions with unique amino acid sequences (with at least one amino acid change in the full sequence)were selected for conversion to full-length human antibodies.

### 3.4 Generation of plasmids for antibody production

Variable region sequences were back-translated into DNA sequences using GENEOPTIMIZER^{®} technology prior to synthesis of the resulting DNA *de novo* by assembly of synthetic oligonucleotides (GeneArt, Germany). Synthesized heavy and light chain variable region sequences were subcloned into mammalian expression vectors containing either a human IgG1 heavy chain constant region (such as Swissprot accession number PO1857) or a human lambda constant region (Swissprot accession number P0CG05) to yield full length antibody chains.

### 3.5 Expression of antibodies

Antibodies were produced by co-transfecting plasmids encoding antibody heavy and light chains into EXPI293^{®} cells (Life Technologies). The day before transfection, the number of cells needed for the experiment was determined. For each 20 mL transfection, 3.6 × 10⁷ cells were required in 20 mL of EXPI293^{®} Expression Medium. On the day prior to transfection, cells were seeded into TPP 50mL bioreactor tubes at a density of 0.9 × 10⁶ viable cells/mL and incubated overnight at 37°C in a humidified atmosphere of 8% CO₂ in air on an orbital shaker rotating at 200 rpm. On the day of transfection, the cell number and viability were determined using an automated cell counter. Only cultures with >98% viable cells were used. For each 20 mL transfection, lipid-DNA complexes were prepared by diluting 10 µg of heavy chain DNA and 10 µg of light chain DNA in OPTI-MEM^{®} I Reduced Serum Medium (Cat. no. 31985-062) to a total volume of 1.0 mL. 54 µL of EXPIFECTAMINE^{®} 293 Reagent was diluted in OPTI-MEM^{®} I medium to a total volume of 1.0 mL. Both vials were mixed gently and incubated for 5 minutes at room temperature. Following incubation, the diluted DNA was mixed with the diluted EXPIFECTAMINE^{®} 293 Reagent and the DNA-EXPIFECTAMINE^{®} 293 Reagent mixture incubated a further 20 minutes at room temperature to allow the formation of DNA-EXPIFECTAMINE^{®} 293 Reagent complexes. Following incubation, 2 mL of DNA-EXPIFECTAMINE^{®} 293 Reagent complex was added to each TPP 50mL bioreactor tube. To the negative control tube, 2 mL of OPTI-MEM^{®} I medium was added instead of DNA-EXPIFECTAMINE^{®} 293 Reagent complex. The cells were incubated in a 37°C incubator with a humidified atmosphere of 8% CO2 in air on an orbital shaker rotating at 200 rpm. Approximately 16-18 hours post-transfection, 100 µL of EXPIFECTAMINE^{®} 293 Transfection Enhancer 1 and 1.0 mL of EXPIFECTAMINE^{®} 293 Transfection Enhancer 2 were added to each tube. Antibodies were harvested approximately 72 hours post-transfection.

### 3.6 Purification of antibodies

Cultures of transfected EXPI293^{®} cells were spun down in 50 mL falcon tubes at 3000 x g for 20 minutes, and supernatants were filtered using a 0.22 µm filter (Corning). Antibody-containing supernatants were purified using a Gilson ASPEC GX274 robot by Protein A chromatography. Briefly, SPE cartridges (Agilent, 12131014) packed with 1.2 mL MABSELECT SURE^{®} protein A resin (GE Healthcare) were pre-equilibrated with 3 column volumes of 1X PBS. 18 mL of supernatant was run over the columns followed by a 4ml 1X PBS wash. Each column was pre-eluted with 0.9mL of 0.1M citric acid, pH 2.9. Purified antibodies were eluted with 2 mL 0.1 M citric acid, pH 2.9. Antibodies were desalted into Sørensens PBS (59.5mM KH₂PO₄, 7.3mM Na₂HPO₄.2H₂O, 145.4mM NaCl (pH ~5.8)) using PD-10 columns (GE Healthcare).

### 3.7 Three point dilution on CTLL-2 cells

Each purified antibody was tested at three different dilutions for its ability to inhibit IL-15 mediated proliferation of CTLL2 cells.

CTLL-2 cells were incubated in complete media without IL-2 or IL-15 for 4 hours prior to testing. Cells (5x10⁴/well) were incubated in 96-well plates with 200 pM of IL-15 complex with the IL-15 Receptor-alpha, the concentration that induced 50% of the maximum cell proliferation (EC₅₀). Antibody dilutions were added to the plates and incubated for 48 hours. Three anti-IL-15 antibody dilutions were used: 2000 pM, 200 pM and 20 pM. Inhibition of cell proliferation was then assessed using the CELLTITER-GLO^{®} Luminescent Cell Viability Assay (Promega) according to manufacturer's instructions and read on a GLOMAX^{®} 96 Microplate Luminometer (Promega). Data were expressed as relative luminescence units (the number of viable cells in culture based on quantitation of the ATP present, an indicator of metabolically active cells).

This crude dose response of the antibodies' ability to functionally neutralize the biological activity of IL-15 was used to select antibodies for further analysis. A representative selection of results is shown in Figure 2A. Antibody 4 was a highly potent antagonist of IL-15 activity.

### 3.8 Full dose response on CTLL-2 cells

Selected antibodies were run in a 10-point version of the above CTLL-2 cell assay with the aim of generating full dose response curves.

A representative selection of results is shown in Figure 2B. The relative inhibition profile of each antibody was assessed using the IC₅₀ value (the concentration of anti-IL-15 antibodies at which the cell proliferation is reduced by half). Out of the antibodies tested, the most potent antibodies were Antibody 4 and Antibody 10F.

### 3.9 Full dose response on NK-92 cells

The most potent antibodies in the CTLL-2 cell assay were subjected to a further cell assay using NK-92 cells. The cell line is derived from NK malignant non-Hodgkin's lymphoma (ATCC: CRL-2407).

NK-92 cells were incubated in complete media without IL-2 or IL-15 for 4 hours prior testing. Cells (5x10⁴/well) were incubated in 96-well plates with IL-15 complexed with the IL-15 Receptor-alpha at 25 pM (EC₅₀) to induce cell proliferation. Antibody doses were added to the plates and incubated for 48 hours. Inhibition of cell proliferation was then assessed using the CELLTITER-GLO^{®} Luminescent Cell Viability Assay (Promega) according to manufacturer's instructions and read on GLOMAX^{®} 96 Microplate Luminometer (Promega) as described above. Data were expressed as relative luminescence units.

A representative selection of results is shown in Figure 3. The relative inhibition profile of each antibody was assessed using the IC50 value. Antibody 4 had the lowest inhibitory IC50 value (0.1nM and was identified as the most potent inhibitor of IL-15 driven cell based proliferation.

### Example 4

### Modification of the antibody

Antibody 4 was altered with the aim of yielding a positive effect on the antibody's biophysical properties, as well as improving the potency.

### 4.1 Location of critical amino acids for binding

Variants of the parent antibody were generated by modifying each residue in the CDR sequences and assessing the effect on antibody potency and binding properties (CDR scanning). Nine variants at each CDR position were generated by modifying the residues to alanine (A), aspartic acid (D), histidine (H), lysine (K), leucine (L), glutamine (Q), serine (S), tryptophan (W) or tyrosine (Y). These nine amino acids were chosen due to their range of properties so that all functional properties were tested, as shown in Table 1.

**Table 1. Amino acid functional properties.**

| **Amino acid** | **Functional property** |
|---|---|
| Alanine (A) | Small size |
| Aspartic acid (D) | Acidic |
| Histidine (H) | Basic; ring structure |
| Lysine (K) | Basic |
| Leucine (L) | Hydrophobic |
| Glutamine (Q) | Amide |
| Serine (S) | Nucleophilic |
| Tryptophan (W) | Aromatic |
| Tyrosine (Y) | Aromatic |

This resulted in the selection of ~520 variants of the Antibody 4 each differing from Antibody 4 by 1 amino acid. Antibodies were generated as described previously and screening was performed using surface plasmon resonance on a Biacore T200 (GE Healthcare) system using a CM5 Protein A (GE Healthcare) chip. The running buffer used was HBS-EP+ (GE Healthcare) and all interactions were measured at 25 °C and data collection rate was set to 10 Hz. Before running the method, a start-up cycle was performed in which both flow cells of the chip were cleaned with two consecutive 60 s pulses of 0.1 M citric acid (pH 3.0).

Supernatants of EXPI293^{®} F cells co-expressing antibody variants were diluted 1 in 100 in HBS-EP+ (GE Healthcare) and human IL-15 complex was diluted to 10 µg/mL in the same buffer. Antibodies were captured onto the second flow cell of the chip and binding was measured by injection of 30 µL of human IL-15 complex at a flow rate of 30 µL/min across both flow cells and allowing 120 s of dissociation time. The chip surface was regenerated between cycles with two consecutive 60 s pulses of 0.1 M citric acid (pH 3.0) over both flow cells.

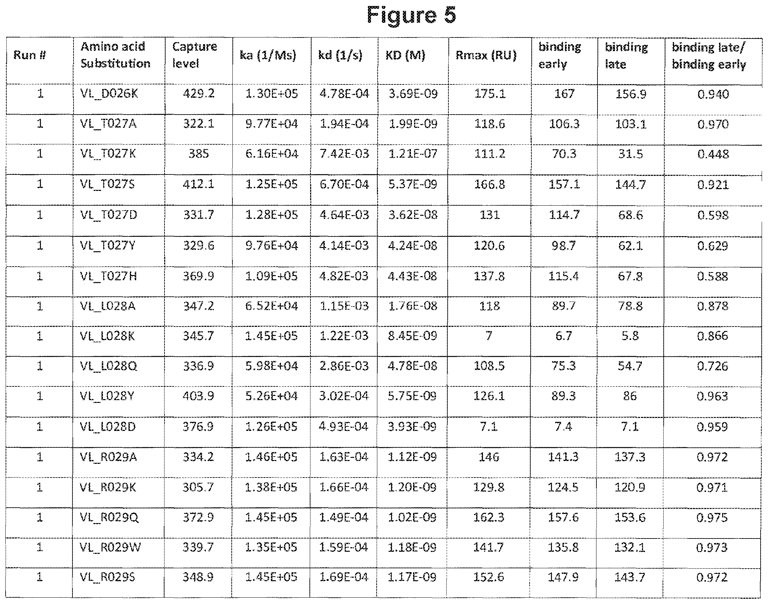

Data from FC2-1 was used for analysis. The resultant sensorgrams were analysed by creating two custom report points, each calculated for a 1 s window starting either 5 s after injection of the sample ('early binding') or 5 s before the end of dissociation ('late binding'). Antibodies were ranked based on the ratio of these two report points ('late binding'/'early binding') as an estimation of dissociation rate. Relative capture levels were used as a rough indicator of productivity. The results of the CDR modification experiments are shown in Figures 5-34. Figures 5-33 show single modifications that led to an improved antibody (shaded grey) and Figure 34 summarizes the single and multiple modifications tested that led to an improved antibody.

These experiments identified the amino acids that were critical for binding and potency as well as the amino acids that could be substituted with no change in binding or potency. Surprisingly, it was found that substitution of amino acids in position 54 or 56 in CDR2 of the heavy chain by aromatic amino acids Y or W led to an increase in potency of the antibody.

Further variants were made to test the substitution of amino acids in position 54 or 56 in CDR2 of the heavy chain by aromatic amino acid phenylalanine (F). These two variants also showed an increase in potency in the NK-92 cell proliferation assay.

Double variants were generated where both of the amino acids in position 54 or 56 of CDR2 of the heavy chain were modified to either Y, W or F. The potency of the double variants was assessed in the NK-92 cell proliferation assay as described in section 3.9. Results are shown in Table 2.

**Table 2. 54-56 double variants.**

| | | Position 54 | | |
|---|---|---|---|---|
| | | F | Y | W |
| Position 56 | F | 4.9 pM | 6.6 pM | 3.4 pM |
| | Y | 11.2 pM | 7.4 pM | 10.1 pM |
| | W | 8.3 pM | 7.6 pM | 5.1 pM |

It was found that the effect of including two aromatic amino acids at positions 54 and 56, rather than being counteractive, instead led to a cumulative improvement in potency.

Antibody 11 (comprising 54Y and 56Y) has at least a 10 fold improvement of IC₅₀ in the NK-92 cell proliferation assay compared to Antibody 4 (Figure 35 and Table 3).

### 4.2 Modification of Antibody 4 to reduce potential immunogenic epitopes

To remove potentially immunogenic epitopes in antibodies substitutions can be made to the peptide sequence to revert the sequence in this region back to germline antibody sequence. Substituting I82aS in the heavy chain (Antibody 63) resulted in a germline sequence and removed the predicted immunogenic peptide in this region. This substitution had no impact on potency in the NK-92 assay, as shown in Table 3 (Antibody 63 compared to Antibody 11).

Substitution of N30S in the light chain (Antibody 73) resulted in a germline sequence and removed the predicted immunogenic peptide in this region. This substitution had a minor impact on potency in the NK-92 assay, as shown in Table 3 (Antibody 73 compared to Antibody 11).

When both substitutions were combined into one antibody, Antibody 64, a slight reduction in potency compared to Antibody 11 was observed (Figure 35).

**Table 3. List of reduced immunogenicity variants**

| Antibody # | Heavy Chain substitutions | Light Chain substitutions | IC 50 (NK-92) in pM | VH SEQ ID NO: | VL SEQ ID NO: | Full L SEQ ID NO: |
|---|---|---|---|---|---|---|
| Antibody 4 | Wild type | WT | 148 | 7 | 8 | 9 |
| Antibody 11 | S54Y + N56Y | WT | 6.3 | 454 | 8 | 9 |
| Antibody 63 | S54Y + N56Y + I82aS | WT | 4.21075 | 4 | 8 | |
| Antibody 64 | S54Y + N56Y + I82aS | N30S | 13.5975 | 4 | 455 | 456 |
| Antibody 65 | S54Y + N56Y + I82aS | D92E | 42.48 | 4 | 503 | |
| Antibody 66 | S54Y + N56Y + I82aS | S93L | 6.0443 | 4 | 457 | 458 |
| Antibody 67 | S54Y + N56Y + I82aS | S93E | 7.688 | 4 | 505 | |
| Antibody 68 | S54Y + N56Y + I82aS | S93F | 5.338 | 4 | 459 | 460 |
| Antibody 69 | S54Y + N56Y + I82aS | N30S+092E | 162.5 | 4 | 507 | |
| Antibody 70a | S54Y + N56Y + I82aS | N30S+S93L | 18.178 | 4 | 5 | 6 |
| Antibody 71 | S54Y + N56Y + I82aS | N30S+S93E | 26.39 | 4 | 509 | |
| Antibody 72 | S54Y + N56Y + I82aS | N30S+S93F | 20.93 | 4 | 510 | |
| Antibody 73 | S54Y + N56Y | N30S | 11.842 | 454 | 455 | |
| Antibody 74 | S54Y + N56Y | D92E | 33.9 | 454 | 503 | |
| Antibody 75 | S54Y + N56Y | S93L | 3.136 | 454 | 457 | |
| Antibody 76 | S54Y + N56Y | S93E | 7.686 | 454 | 505 | |
| Antibody 77 | S54Y + N56Y | S93F | 5.694 | 454 | 506 | |
| Antibody 78 | S54Y + N56Y | N30S+092E | 168 | 454 | 507 | |
| Antibody 79 | S54Y + N56Y | N30S+S93L | 12.38 | 454 | 5 | |
| Antibody 80 | S54Y + N56Y | N30S+S93E | 22.75 | 454 | 509 | |
| Antibody 81 | S54Y + N56Y | N30S+S93F | 17.29 | 454 | 510 | |

### 4.3 Modification of Antibody 64 to improve manufacturability

Amino acid analysis of the variable heavy and light chain sequence of Antibody 64 and related antibodies identified amino acids that may undergo isomerization. Changes to these amino acids may, over time, alter the stability of the antibody. In the light chain, D92 and S93, were identified as a potential aspartic acid isomerization site. To reduce the potential impact of these predicted issues variants of Antibody 64 were produced containing conservative or semi-conservative amino acid substitutions in these positions. These substitutions and their impact on the potency of the resulting variants are listed in Table 4. Figure 35 shows a representative selection of variants tested in the NK-92 cell proliferation assay.

Modifications to enhance manufacturability by changing D92 lead to a loss in potency (see Antibody 68 compared to Antibody 11) Altering S93 to L93 surprisingly resulted in an improvement in potency of the antibody, as shown in Table 3 and in Figure 35.

A summary of the modifications made to Antibody 4 to generate Antibody 70 is given in Table 4.

**Table 4. Modification Summary**

| Amino acid position | Unmodified residue (Antibody 4) | Modified residue (Antibody 70) | Improved property |
|---|---|---|---|
| H54 | S | Y | Improved potency |
| H56 | N | Y | Improved potency |
| H82a | I | S | Removes potential immunogenic epitope by germlining |
| L30 | N | S | Removes potential immunogenic epitope by germlining |
| L93 | S | L | Reduces potential isomerization site |

### Example 5

### Receptor affinity and selectivity of Antibody 70 variants

Constant region variants of antibody 70 were generated. The heavy chain variable region of antibody 70 was synthesized in-frame with the human IgG isotype constant domains described in Table 5.

**Table 5. Antibody 70 variants**

| **Antibody 70 variants** | **SEQ ID NO** |
|---|---|
| Antibody 70a | 33 |
| Antibody 70b | 35 |
| Antibody 70e | 47 |
| Antibody 70f | 49 |

IL-15 binds to and signals through a complex composed of IL-15Rα, IL-2Rβ and IL-2Ry. Antibodies were assessed for their ability to bind IL-15Rα, as well as cytokines that share the common receptor IL-2Rβ/γ such as IL-2, IL-4, IL-7, IL-9 and IL-21. Antibody 70 variants did not bind to IL-15Rα, IL-2, IL-4, IL-7, IL-9 and IL-21.

Binding of Antibody 70 variants to human IL-15 complexed with the IL-15 Receptor-alpha were assessed using surface plasmon resonance on a BIACORE^{®} T200 (GE Healthcare) system using a Protein A Sensor Chip (GE Healthcare). Antibodies were captured onto the second flow cell to a level of 150-200 RU. Purified cytokines were diluted to 10 µg/mL in HBS-EP+. Binding was measured by injection of 45 µL of each cytokine at a flow rate of 30 µL/min across both flow cells and allowing 180 s of dissociation time. The chip surface was regenerated between cycles with two 10 sec pulses of 50 mM sodium hydroxide. The running buffer used was HBS-EP+ (GE Healthcare) and all interactions were measured at 25 °C and data collection rate set to 10 Hz.

A summary of the surface plasmon resonance data is shown on Figure 36. The affinity can be measured by the KD (the equilibrium dissociation constant between the antibody and its antigen). The Antibody 70 variants had the lowest KD values (from 0.133 to 0.193 nM), as compared to Antibody 4 (average KD = 0.629 nM) and AMG714 (average KD = 1.84 nM). The large difference in KD between variants of Antibody 70 and AMG714 is driven by the dissociation rate (kd). IL-15 dissociates from AMG714 ten times faster than the rate of dissociation of IL-15 from Antibody 70 variants. Once bound to IL-15, Antibody 70 and variants thereof remain bound for longer and therefore are superior at inhibiting IL-15 activity. This was tested in a cell based potency assay.

The potency of Antibody 70 variants and AMG714 was assessed in the NK-92 proliferation assay with IL-15 complexed with the IL-15 Receptor-alpha at 25 pM (EC50) to induce cell proliferation as described in Example 3. Figure 37 and Table 6 shows that the IC₅₀ of the Antibody 70 variants was 83 to 98 times lower than the IC₅₀ of AMG714. Therefore, Antibody 70 was superior at inhibiting IL-15 activity in a cell based potency assay.

**Table 6: IC₅₀ values for anti-IL-15 antibodies in the NK-92 proliferation assay**

| **Antibody** | **Mean IC₅₀ (pM)** | **Std. Dev.** | **Min IC₅₀ (pM)** | **Max IC₅₀ (pM)** |
|---|---|---|---|---|
| **AMG714** | 1303.2 | 666.2 | 377.8 | 2653.9 |
| **Antibody 70a** | 14.7 | 3.8 | 10.2 | 22.0 |
| **Antibody 70b** | 13.3 | 2.5 | 10.6 | 16.6 |
| **Antibody 70e** | 15.7 | 4.7 | 11.7 | 20.9 |
| **Antibody 70f** | 14.6 | 5.0 | 7.3 | 29.8 |

Subsequent studies with antibody 70F resulted in an average affinity for the epitope having a KD of 430pM. Altogether, these results suggest that Antibody 70 variants have improved binding capacity, affinity and potency to human IL-15 compared to AMG714.

### Example 6

### Epitope mapping of Antibody 70

Epitope mapping was performed using alanine scanning experiments. Modelling analysis was carried out to determine probable exposed residues on IL-15 that were not involved in IL-15Rα binding. IL-15 constructs were then designed in which each of these theoretically exposed residues was substituted with an alanine. The list of these variants is listed in Table 7

**Table 7: list of IL-15 alanine variants**

| Residue | Position | Percentage of solvent exposure of side chain |
|---|---|---|
| N | 1 | 37.7 |
| V | 3 | 52 |
| N | 4 | 55 |
| S | 7 | 55.1 |
| D | 8 | 26.8 |
| K | 10 | 66.1 |
| K | 11 | 53.6 |
| E | 13 | 35.2 |
| D | 14 | 65.5 |
| L | 15 | 35.2 |
| Q | 17 | 70.7 |
| S | 18 | 79.1 |
| H | 20 | 94.5 |
| S | 29 | 34 |
| D | 30 | 78.8 |
| H | 32 | 57.2 |
| P | 33 | 61.1 |
| S | 34 | 69.8 |
| K | 36 | 46 |
| K | 41 | 63.6 |
| Q | 48 | 61.4 |
| D | 56 | 43.7 |
| A | 57 | 66.7 |
| H | 60 | 56.4 |
| D | 61 | 61 |
| E | 64 | 70.4 |
| I | 68 | 64.6 |
| L | 69 | 31.6 |
| N | 72 | 59.6 |
| S | 75 | 70.2 |
| N | 77 | 77.9 |
| N | 79 | 96.5 |
| V | 80 | 96.9 |
| T | 81 | 91.5 |
| E | 82 | 42.9 |
| S | 83 | 93.8 |
| K | 86 | 65.7 |
| E | 92 | 64.1 |
| K | 94 | 50.5 |
| N | 95 | 59.2 |
| K | 97 | 77.1 |
| E | 98 | 37.6 |
| Q | 101 | 49.5 |
| H | 105 | 55.2 |
| Q | 108 | 60.7 |
| M | 109 | 55.4 |
| I | 111 | 41.3 |
| N | 112 | 93.3 |

These constructs were then co-expressed with IL-15Rα and supernatant from the expression cultures tested for protein expression and binding to Antibody 70a using SPR.

The running buffer used was HBS-EP+ (GE Healthcare). All interactions were measured at 25 °C and the data collection rate was set to 10 Hz. Data from FC2-1 was used for analysis. The resultant sensorgrams were analysed by creating two custom report points, each calculated for a 5 s window starting either 10 s after injection of the sample ('early binding') or 10 s before the end of dissociation ('late binding'). These values were subtracted from the values for the untransfected control and a relative dissociation rate was estimated by first, taking a ratio of these two custom report points ('late binding'/'early binding') and then, taking the resultant values as a fraction of the value calculated for wild-type human IL-15 complexed with the IL-15 Receptor-alpha (data not shown).

To confirm the results from the supernatant screen, samples which showed a faster dissociation rate compared to wild-type human IL-15 complexed with the IL-15 Receptor-alpha were purified and retested by SPR. A Protein A Sensor Chip (GE Healthcare) was used to capture Antibody 70a onto the second flow cell to a capture level of 150-200 RU. Purified IL-15 alanine scan constructs were diluted to 10 µg/mL in HBS-EP+ and then a 2-fold dilution series was made. Binding was measured by injection of 45 µL of each dilution at a flow rate of 30 µL/min across both flow cells and allowing 600 s of dissociation time. The chip surface was regenerated between cycles with a 10 sec pulse of 50 mM sodium hydroxide. The running buffer used was HBS-EP+ (GE Healthcare) All interactions were measured at 25 °C and the data collection rate was set to 10 Hz. Data from Fc2-1 was used for analysis and generated sensorgrams were fitted using a 1:1 Langmuir equation (using local Rmax fitting) to determine KD. Data are shown in Figure 38 for all IL-15 alanine variants co-expressed with IL-15Rα that showed a reduction in binding to the tested anti-IL-15 antibodies. Antibody 70a had low binding to mutated IL-15 with a Q108A substitution. AMG714 has no binding or significantly reduced binding to mutated IL-15 with the following amino acid substitutions: E98A, Q101A, H105A and Q108A. These results indicate that mutation of these 4 amino acids disrupts the binding of AMG714 to IL-15 complex with the IL-15 Receptor-alpha, and only the mutation of Q108 disrupts the binding of Antibody 70a to IL-15 complexed with the IL-15 Receptor-alpha. The results in Figure 38 show that for the residues in IL-15 that were tested, the binding of the AMG714 antibody is reduced upon alteration of the residues, whereas the binding of Antibody 70a was not reduced by alteration given the high affinity of the antibody for IL-15; only alteration of Q108 in IL-15 resulted in binding reduction by Antibody 70a.

Given both antibodies (Antibody 70a and AMG714) had low or no binding to Q108A mutant of IL-15, an additional screen was performed on Antibody 70a with another anti-IL-15 antibody, which bound Q108 and wild-type IL-15 complex with equal affinity demonstrating the Q108A mutant is correctly folded. Alanine scanning identifies residues that, when mutated, can disrupt the binding of the antibody to IL-15. To determine the exact residues that contact IL-15 and thus determine the Antibody 70a binding epitope, the interaction of Antibody 70a with human IL-15 was characterized by X-ray crystallography.

In preparation for crystallization experiments, recombinant human IL-15 was expressed and purified from bacteria. Antibody 70a Fab (where FAb is Fragment antigen binding) was prepared by papain-mediated cleavage of the antibody hinge, which separates the antibody FAb from the Fc. The FAb was purified via standard protein-A chromatography methods and complexed with IL-15. The FAb:IL-15 was purified by size exclusion chromatography and set up for crystallization screening using sparse matrix crystallization screens. Final crystals used for diffraction data collection were formed in 10% polyethyleneglycol (PEG) 20000, 20% PEG 500 monomethyl ether (MME), 30 mM CaCl₂, 30 mM MgCl₂, 100 mM of unspecified imidazole/sodium cacodylate/MES (acid)/Bis-Tris buffer mix, pH 6.5. Diffraction data were collected to 2.25 Å at beamline i04 at the Diamond Synchrotron facility. The structure was solved by molecular replacement using published structures of human IL-15 and FAb molecules as templates for model building. The structure was iteratively refined against experimental data to R/Rfree values of 23.6/28.9. The structure shows that the variable regions of Antibody 70a bind at the IL-2Ry and IL-2Rβ binding sites of human IL-15, thus blocking the interaction of those receptor units with IL-15 (Figures 39A and 39B). This is further demonstrated by looking at the interactions of the IL-15 side chains with the side chains of the FAb, comparing these to the IL-15 side chains that interact with IL-2Ry and IL-2Rβ and looking for side chains or residues in common to both interactions.

Table 8 lists the interactions between the IL-15 side chains and the side chains of the FAb fragment of Antibody 70a. The side chains of Antibody 70a FAb that contact IL-15 form the paratope of Antibody 70a and related antibodies. Interpretations presented in the table correspond to: "hydrophobic interactions" - van der Waals interactions between atom pairs; "water-mediated" - water mediated hydrogen bonds between atom pairs; "hydrogen bond" - hydrogen bonds of heteroatoms between 2.5- 3.5Å; "H-pi hydrogen bond" - hydrogen bond corresponding to a donor/acceptor atom within an aromatic

**Table 8 - Side chain interactions of IL-15 with the FAb of Antibody 70a**

| **IL-15 residue** | | **Antibody 70a Fab** | | | |
|---|---|---|---|---|---|
| **residue** | **Chemical atom name** | **CDR** | **Residue** | **Chemical atom name** | **Interpretation** |
| Ile6 | side chain | CDR_L1 | Tyr31 | Phenyl ring | Hydrophobic interaction |
| Ser7 | Backbone carbonyl | CDR_L1 | Arg29 | Backbone carbonyl | Water-mediated hydrogen bond |
| Lys10 | Amino group | CDR_L1 | Leu28 | Backbone carbonyl | Hydrogen bond |
| Lys10 | Amino group | CDR_L1 | Arg29 | Backbone carbonyl | Hydrogen bond |
| Lys10 | Backbone carbonyl | CDR_L1 | Tyr32 | Hydroxyl group | Hydrogen bond |
| Lys10 | Aliphatic part of side chain | CDR_L1 | Tyr32 | Phenyl ring | Hydrophobic interaction |
| Glu13 | Carboxylate | CDR_H3 | Trp99 | Indole nitrogen | Hydrogen bond |
| Glu13 | Carboxylate | CDR_L2 | Lys51 | Backbone amide | Water-mediated hydrogen bond |
| Glu13 | Carboxylate | CDR_L2 | Asn53 | Amide nitrogen | Water-mediated hydrogen bond |
| Asp14 | Carboxylate group | CDR_L1 | Tyr32 | Hydroxyl group | Hydrogen bond |
| Asp14 | Carboxylate group | CDR_L2 | Lys51 | Amino group | Hydrogen bond |
| Ser29 | Backbone carbonyl | CDR_H1 | Ser32 | Hydroxyl group | Hydrogen bond |
| Val31 | Backbone carbonyl | CDR_H2 | Tyr52 | Hydroxyl group | Hydrogen bond |
| Pro81 | Side chain | CDR_H2 | Tyr54 | Phenyl rings | Hydrophobic interaction |
| Gln101 | Amide nitrogen | CDR_H3 | IIe97 | Backbone carbonyl | Hydrogen bond |
| VaI104 | Side chain | CDR_H3 | Trp99 | Indole group | Hydrophobic interaction |
| His105 | Side chain Nitrogen | CDR_H3 | Gly98 | Backbone amide | Hydrogen bond |
| His105 | Side chain Nitrogen | CDR_H1 | Ser32 | Backbone carbonyl | Hydrogen bond |
| Gln108 | Amide nitrogen | CDR_L3 | Gly95 | Backbone carbonyl | Hydrogen bond |
| Gln108 | Amide oxygen | CDR_L1 | Tyr31 | Hydroxyl group | Hydrogen bond |
| Gln108 | Amide oxygen | CDR_H3 | Gly99 | Backbone carbonyl | Water-mediated hydrogen bond |
| Ile111 | Backbone carbonyl | CDR_L3 | Lys95A | Amino group | Hydrogen bond |
| Asn112 | Amide nitrogen | CDR_H1 | Trp34 | Indole group | H-pi hydrogen bond |
| Asn 112 | Backbone carbonyl | CDR_H2 | Asn58 | Amide nitrogen | Hydrogen bond |
| Thr113 | Side chain Methyl | CDR_H2 | Tyr56 | Phenyl ring | Hydrophobic interaction |
| Ser114 | C-terminus | CDR_L3 | Lys95A | Amino group | Hydrogen bond |

A similar analysis was performed on the X-ray structure of the quaternary IL-15 receptor complex (pdb code 4GS7). Table 9 shows the IL-15 residues important for binding of the IL-15 complex to IL-2Ry and IL-2Rβ based on the hydrogen bonding that occurs between the side chains of IL-15 and the side chains of IL-2Ry and IL-2Rβ.

**Table 9 - Side chain interactions of IL-15 with IL-2Ry and IL-2Rβ**

| | **IL-15 residue** | | **IL-2Rγ residue** | |
|---|---|---|---|---|
| **Residue** | **Chemical atom name** | **Residue** | **Chemical atom name** | **Interpretation** |
| Asp30 | Amide oxygen | Asn71 | Side chain nitrogen | Hydrogen bond |
| Asp30 | Carboxylate group | Thr105 | Hydroxyl group | Hydrogen bond |
| His32 | Side chain nitrogen | Asp73 | Carobxylate group | Hydrogen bond |
| Gln108 | Amide oxygen | Tyr103 | Hydroxyl group | Hydrogen bond |
| Gln108 | Amino group | Pro207 | Amide oxygen | Hydrogen bond |
| Gln108 | Amino group | Ser211 | Hydroxyl group | Hydrogen bond |
| Asn112 | Carboxylate group | Cys160 | Sulfide | Hydrogen bond |
| Asn112 | Amino group | Tyr103 | Hydroxyl group | Hydrogen bond |

| | **IL-15 residue** | | **IL-2Rβ residue** | |
|---|---|---|---|---|
| **Residue** | **Chemical atom name** | **Residue** | **Chemical atom name** | **Interpretation** |
| Asn1 | Carboxylate group | Thr74 | Hydroxyl group | Hydrogen bond |
| Asp8 | Carboxylate group | His133 | Side chain nitrogen | Hydrogen bond |
| Asp8 | Carboxylate group | Tyr134 | Hydroxyl group | Hydrogen bond |
| Glu64 | Carboxylate group | Arg42 | Amino group | Hydrogen bond |
| Asn65 | Carboxylate group | Arg42 | Amino group | Hydrogen bond |
| Asn65 | Carboxylate group | Arg42 | Amino group | Hydrogen bond |
| Ser7 | Hydroxyl group | Glu136 | Carboxylate group | Hydrogen bond |
| Asn65 | Side chain nitrogen | Gln70 | Backbone carbonyl | Hydrogen bond |

The solvent accessible surface area, formed on interaction of antibody 70 with human IL-15, is 2270.9 Å2. This value was calculated in PYMOL using the method of Strake and Rupley (1973) J. Mol. Biol. 79: 351-71.

Comparison of the side chain interactions of Antibody 70a FAb with IL-15 and the side chain interactions of the IL2R beta and gamma chains with IL-15 identifies several common residues. S7 (Ser7) forms a hydrogen bond with IL-2Rβ, and Q108 (GIn108) and N112 (Asn112) form hydrogen bonds with IL-2Ry, (Figures 39C-39E). These 3 residues also form the epitope on IL-15 to which Antibody 70a FAb binds and forms hydrogen bonds, thereby preventing the interaction of IL-15 with IL-2Rβ and IL-2Ry.

The triple tyrosine motif comprising Y52/54/56 in CDRH2, discovered during the affinity and potency maturation of the Antibody 70a, is a key binding determinant of the antibody with human IL-15. Examining the crystal structure (Figures 39F-39H) it can be seen that this motif veils and protects hydrophobic residues around helix-4 of IL-15 preventing solvation and stabilizing the structure.

### Example 7

### Flow Cytometry Detection of variants binding to IL-15 on primary human cells

To further characterize antibody, their ability to bind IL-15 on primary human cells was tested. Human Peripheral Mononuclear Cells (PBMCs) were isolated and purified from buffy coats using Lymphoprep (Axis-Shield, Lymphoprep) and the binding of the lead Ab was assessed by flow cytometry analysis.

1 x 10⁶ viable PBMCs were initially seeded per well, in a 96-well polypropylene plate (Sigma/Corning) and stained with Zombie Violet dye (Biolegend) for 20 min at 4°C. Cells were further stained with TruStain FcX Fc block (Biolegend) diluted in FACS buffer for 10 min at room temperature. For surface staining, PBMC were stained with 10 µg/mL of test or isotype control antibodies (listed in Table 10), and incubated for 20 min at 4°C. Following immunostaining, samples were then fixed with BD cytofix/cytoperm (BD Biosciences) according to the manufacturer's instructions and stored at 4°C until analysed. For intracellular staining, PBMC were fixed with BD cytofix/cytoperm (BD Biosciences) prior staining. Samples were analysed using a BD FACSCanto II (BD Biosciences).

Initial doublet discrimination was performed on all cell events to remove cell aggregates from analysis. Live cells were selected after exclusion of dead cells using a viability dye. Initial gating of leukocytes separated cells into the following: CD3⁻CD8⁻ cells, CD3⁺CD8⁺ T cells, and CD3⁺CD8⁻ (putatively CD4⁺) T cells. Further analysis of the CD3-population separated cells into CD19+ B cells and a CD19- cell population. The latter population was again additionally gated to select for CD56dimCD16+ and CD56briCD16dim/- NK cells. Further, based on levels of CD14 and CD16 expression, monocytes (Hi SSC population) were subsequently subdivided into the three major monocyte subsets: classical (CD14+ CD16-), intermediate (CD14intCD16int), and non-classical (CD14dimCD16+).

### Data analysis was performed using FLOWJO^{®} analysis software V.10.

**Table 10. List of antibodies and conjugates.**

| **Specificity** | **Conjugate** | **Clone** | **Species & Isotype** | **Supplier** |
|---|---|---|---|---|
| Anti-Human Antibodies | | | | |
| CD3 | APC-Vio770 | REA613 | Recombinant Human IgG1 | Miltenyi Biotec |
| CD8 | VioGreen | BW135/80 | Mouse IgG2a | Miltenyi Biotec |
| CD14 | PerCP | TUK4 | Mouse IgG2a κ | Miltenyi Biotec |
| CD16 | FITC | REA423 | Recombinant Human IgG1 | Miltenyi Biotec |
| CD19 | PE | LT19 | Mouse IgG1 κ | Miltenyi Biotec |
| CD56 | PE Vio770 | AF12-7H3 | Mouse IgG1 | Miltenyi Biotec |
| IL-15 | iFluor647 | Antibody 70a | Human IgG1 λ | In-House |
| | iFluor647 | Antibody 70b | Human IgG1 λ | In-House |
| | iFluor647 | Antibody 70e | Human IgG4 λ | In-House |
| | iFluor647 | Antibody 70f | Human IgG4 λ | In-House |

| Isotype Control Antibodies | | | | |
|---|---|---|---|---|
| Anti-KLH | iFluor647 | | Human IgG1 | In-House |

| **Specificity** | **Conjugate** | **Clone** | **Species & Isotype** | **Supplier** |
|---|---|---|---|---|
| C3 IgG1 | | | | |
| Anti-KLH C3 IgG4 | iFluor647 | | Human IgG4 | In-House |
| APC: Allophycocyanin; FITC: Fluorescein isothiocyanate; PE: Phycoerythrin; PerCP: Peridinin chlorophyll protein | | | | |

A representative binding of Antibody 70 variants is on monocytes is shown in Figure 40. Moderate binding of the Antibody 70 variants was detected on the cell surface of all monocyte subsets (classical, intermediate and non-classical). Higher binding levels were reported in intracellular binding in all monocyte subsets. Marginal differences in binding detection levels were found between both Antibody 70 variants. No or low binding was detected at the surface on T CD4⁺ and CD8⁺ cells and all subsets of NK cells, data not shown. These results indicate that the Antibody 70 variants were able to bind human IL-15 on primary cells.

### Example 8

### Efficacy of Anti-IL-15 Antibodies in Animal Models

### 8.1 In vivo neutralization of human IL-15 and IL-15 complexed with the IL-15 Receptor-alpha

This study was undertaken to determine the extent to which recombinant human IL-15 or IL-15/IL-15Rα complex could induce NK and NKT cell expansion in C57BL/6 mice, and the extent to which an exemplar antibody of the present invention could neutralize such induction.

Groups of 8 male C57BI/6 mice were given a single dose of Antibody 70f (at 10, 3, 1, 0.3 or 0.1 mg/kg) or isotype control (10 mg/kg) i.p. on Day 1 (1 hour before first cytokine injection). NK1.1+ cells were induced by i.p. injection of recombinant IL-15 complex (where IL-15Rα is an Fc chimera) (1.5 µg /mouse) daily for 3 days from Day 1 to Day 3. On Day 4, spleens from mice were harvested. Cell suspensions were prepared from the total spleen of each mouse and counted on an automated cell counter. NK1.1+ numbers were assessed from cell suspensions by flow cytometry based on % of total splenocytes. Phycoerythrin-conjugated anti-mouse NK-1.1 (BD553165) was used. 50,000 events/sample were acquired on the cytometer.

As shown in Figure 41, injection of IL-15/IL-15Rα complex induced NK1.1+ cell accumulation in mouse spleen. This accumulation could be significantly inhibited by treatment with Antibody 70f from 0.3 mg/kg but not with a human isotype control antibody.

### 8.2 Effect of anti-IL-15 antibodies on circulating NK cell numbers in non-human primates

Administration of anti-IL-15 antibodies has previously been shown to decreased circulating NK cell numbers in cynomolgus monkeys (Lebrec et al. (2013) J. Immunol. 191:5551-5558). To further characterize Antibody 70, the consequence of antagonising IL-15 activity on circulating NK cells was tested *in vivo.* Groups of 4 male cynomologus monkeys received a single intravenous injection of Antibody 70f at 1 or 10 mg/kg or Antibody 70b at 10 mg/kg. Circulating numbers of NK cells were quantified by expression of the NK cell marker CD159a (NKG2A), determined by flow cytometry analysis of whole blood samples pre-dose and at study days 2, 5, 8, 15, 30, 45, 60, 75, 90, 102, 120 and 150.

Individual timepoints for each monkey and the median (solid line) peripheral blood NK cell counts are shown in Figure 42. Administration of Antibody 70f at 1 or 10 mg/kg or Antibody 70b at 10 mg/kg resulted in a significant decrease below pre-dose circulating NK cell numbers from study day 7 which was sustained to study day 120 in the majority of animals.

### 8.3 Effect of anti-IL-15 antibodies in rhesus model of celiac disease in non-human primates

A chronic diarrheal disease named "Gluten-Sensitive Enteropathy" has been described in a subset of captive rhesus monkeys fed gluten-containing chow. When fed with a gluten-containing diet, gluten-sensitive macaques showed signs and symptoms of celiac disease including presence of intestinal tissue transglutaminase autoantibodies, anti-gliadin serum antibodies, decreased resorption of nutrients, decreased xenobiotic metabolism, villous atrophy, lowered diversity of gut microbiome, chronic diarrhea, weight loss, cancer predisposition and immunogenetic (MHC II-linked) association (Bethune MT et al. (2008) PLoS ONE. 3(2):e1614). A gluten-free diet reversed these clinical, histological and serological features, while reintroduction of dietary gluten caused rapid relapse. Interestingly, biopsies from gluten sensitive macaques showed IL-15 overexpression in jejunum tissues (Sestak K et al. (2016) Nutrients. 8(7):401.)

The capacity of an anti-IL-15 antibody of the invention to inhibit the gluten induced symptoms was tested in rhesus macaques with gluten-Sensitive enteropathy. The gluten-free (GFD) and gluten-containing (GD) diets were administered to all 6 gluten sensitive macaques to induce the stages of immunological remission and relapse, respectively, characterized by anti-gliadin (AGA) and anti-transgluataminase 2(TG2) positive and negative plasma antibody responses as described in Sestak et al., 2015; 2016. After inducing AGA/TG2 antibody relapse, Antibody 70f was intravenously (i.v.) administered weekly in a dose of 10 mg/kg (BW) to three animals for 28 days (Group 1) and three animals for 90 days (Group 2) while macaques were still fed gluten containing diet. Intestinal biopsies were taken at stages of the study to measure the villus height to crypt depth ratio, and intraepithelial lymphocyte (IELs) counts. Presence of auto-antibodies against transglutaminase-2 and anti-gliadin antibodies were measured from serum samples. The study design is shown in Figure 45A.

The evaluation of AGA and TG2 plasma (IgG) antibodies, GS enteropathy including morphometric evaluation of small intestinal villous heights versus crypt depths, i.e. V/C ratios, was done according to previously established protocols Sestak et al., 2015; 2016. Small intestinal biopsies were collected at times corresponding to immunological relapse (GD) and remission (GFD), as well as the beginning and end of the Anti-IL-15 antibody treatment period. Biopsies were collected and processed for intra-epithelial lymphocyte (IEL) counts as described by Sestak et al., 2016.

### Results:

Assessment of jejunum biopsy tissues collected from gluten sensitive rhesus macaques prior, during and after the anti-IL15 antibody treatment revealed amelioration of enteropathy upon morphological evaluation of small intestinal tissue architecture, i.e., villous heights vs. crypt depths (V/C) ratios. On a gluten diet, macaques in both groups had significant loss of villus height, crypt depth tissue architecture (Figure 45B). The V/C ratios improved significantly with anti-IL15 antibody treatment (*p*<0.0001) benefiting both groups of celiac macaques, as all treated animals exhibited increased heights of their small intestinal villi, i.e., V/C ratios to an extent comparable with healthy, age-matched controls (Figure 45B).

To evaluate the efficacy of Anti-IL-15 antibody treatment in gluten sensitive rhesus macaques the counts of small intestinal IELs were compared between biopsy samples taken at time points representing the GD diet (6 months), GFD (3 months), and Anti-IL-15 antibody treatment (days 35 and 61) while on GD (Figure 45C). Compared to the IEL count taken at an earlier timepoint when the animals were on a GD, both Anti-IL15 antibody treatment groups had significantly lower IEL counts (*p*<0.0001). Despite being fed a gluten containing diet, anti-IL15 antibody treatment measured at post-treatment day 35 (TD35) in Group 1 and at TD61 in Group 2 macaques, resulted in a greater decrease of IELs (*p*<0.0001) than that associated with 3 months of GFD (Figure 45C).

Prior to treatment, the levels of anti-gliadin antibodies increased on exposure to gluten and decrease on a gluten free diet in 5/6 animals (animal 1B having no AGA response), indicating the animals were gluten sensitive (Figure 45D). Anti-IL-15 treatment reduced anti-gliadin (AGA) antibodies in 5/5 animals that had high AGA levels prior to treatment, which was surprising given these animals were still exposed to a GD.

In summary anti-IL15 antibody treatment attenuated gluten-induced small intestinal mucosal injury (improved V/C ratio), attenuated gluten-induced small intestinal mucosal inflammation (reduced IEL counts) and attenuated gluten-induced serum antibodies (reduced anti-gliadin antibodies) as measured in a rhesus macaque model of celiac disease.

The disclosure is not limited to the embodiments described and exemplified above, but is capable of variation and modification within the scope of the appended claims.

### EMBODIMENTS

1. An antibody, comprising a variable heavy chain and a variable light chain, wherein the antibody specifically binds to an epitope comprising the Q108 residue of human IL-15, wherein the IL-15 is complexed with IL-15 Receptor alpha (IL-15Rα).
2. The antibody of embodiment 1, wherein the antibody has an affinity for the epitope that includes a dissociation constant (KD) of less than 1.8 x 10⁻⁹ M as determined by surface plasmon resonance.
3. The antibody according to embodiment 1 or 2, wherein the epitope further comprises the S7 and N112 residues of human IL-15.
4. The antibody according to any one of embodiments 1-3, wherein the dissociation constant (KD) is less than 1x 10⁻⁹ M.
5. An antibody, comprising an HCDR1 comprising the amino acid sequence of SEQ ID NO: 16, an HCDR2 comprising the amino acid sequence of SEQ ID NO: 17, an HCDR3 comprising the amino acid sequence of SEQ ID NO: 20, an LCDR1 comprising the amino acid sequence of SEQ ID NO: 25, an LCDR2 comprising the amino acid sequence of SEQ ID NO: 28, and an LCDR3 comprising the amino acid sequence of SEQ ID NO: 29, wherein the antibody specifically binds to human IL-15, wherein the IL-15 is complexed with IL-15 Receptor alpha (IL-15Rα).
6. The antibody according to embodiment 5, wherein the antibody comprises an HCDR2 comprising the amino acid sequence of SEQ ID NO: 18.
7. The antibody according to embodiment 5 or 6 wherein the antibody comprises an LCDR1 comprising the amino acid sequence of SEQ ID NO: 26 and an LCDR3 comprising the amino acid sequence of SEQ ID NO: 30.
8. The antibody according to embodiment 7, wherein the antibody comprises an HCDR2 comprising the amino acid sequence of SEQ ID NO: 18, a heavy chain FR3 comprising the amino acid sequence of SEQ ID NO: 13.
9. The antibody according to embodiment 7 or 8, wherein the antibody comprises a heavy chain variable region comprising the amino acid sequence of SEQ ID NO: 4.
10. The antibody according to embodiment 9, wherein the antibody comprises a light chain variable region comprising the amino acid sequence of SEQ ID NO: 5.
11. The antibody according to embodiment 9 or 10, wherein the antibody comprises a light chain comprising the amino acid sequence of SEQ ID NO: 6.
12. The antibody according to embodiment 7 or 8, wherein the antibody comprises a light chain variable region comprising the amino acid sequence of SEQ ID NO: 5.
13. The antibody according to embodiment 12, wherein the antibody comprises a light chain comprising the amino acid sequence of SEQ ID NO: 6.
14. The antibody according to embodiment 5 or 6, wherein the antibody comprises an LCDR1 comprising the amino acid sequence of SEQ ID NO: 27 and an LCDR3 comprising the amino acid sequence of SEQ ID NO: 31.
15. The antibody according to embodiment 14, wherein the antibody comprises a heavy chain variable region comprising the amino acid sequence of SEQ ID NO: 454 and a light chain variable region comprising the amino acid sequence of SEQ ID NO: 8.
16. The antibody according to any one of embodiments 5, 6, 14, and 15, wherein the antibody comprises a light chain comprising the amino acid sequence of SEQ ID NO: 9.
17. The antibody according to embodiment 5 or 6, wherein the heavy chain FR3 comprises the amino acid sequence of SEQ ID NO: 12.
18. The antibody according to embodiment 17, wherein the heavy chain FR3 comprises the amino acid sequence of SEQ ID NO :13.
19. The antibody according to embodiment 5 or 6, wherein the antibody comprises an LCDR1 comprising the amino acid sequence of SEQ ID NO: 26 and an LCDR3 comprising the amino acid sequence of SEQ ID NO: 31.
20. The antibody according to embodiment 5 or 19, wherein the antibody comprises a heavy chain variable region comprising the amino acid sequence of SEQ ID NO: 4 and a light chain variable region comprising the amino acid sequence of SEQ ID NO: 455.
21. The antibody according to embodiment 19 or 20, wherein the antibody comprises a light chain comprising the amino acid sequence of SEQ ID NO: 456.
22. The antibody according to embodiment 5 or 6, wherein the antibody comprises an LCDR1 comprising the amino acid sequence of SEQ ID NO: 27 and an LCDR3 comprising the amino acid sequence of SEQ ID NO: 30.
23. The antibody according to embodiment 22, wherein the antibody comprises a heavy chain variable region comprising the amino acid sequence of SEQ ID NO: 4 and a light chain variable region comprising the amino acid sequence of SEQ ID NO: 457.
24. The antibody according to embodiment 22 or 23, wherein the antibody comprises a light chain comprising the amino acid sequence of SEQ ID NO: 458.
25. The antibody according to embodiment 5 or 6, wherein the antibody comprises an LCDR1 comprising the amino acid sequence of SEQ ID NO: 27, and an LCDR3 comprising the amino acid sequence of SEQ ID NO: 519.
26. The antibody according to embodiment 25, wherein the antibody comprises a heavy chain variable region comprising the amino acid sequence of SEQ ID NO: 4 and a light chain variable region comprising the amino acid sequence of SEQ ID NO: 459.
27. The antibody according to embodiment 26, wherein the antibody comprises a light chain comprising the amino acid sequence of SEQ ID NO: 460.
28. The antibody according to embodiment 5 or 17, wherein the antibody comprises an HCDR2 comprising the amino acid sequence of SEQ ID NO: 19, an LCDR1 comprising the amino acid sequence of SEQ ID NO: 27, an LCDR3 comprising the amino acid sequence of SEQ ID NO: 31, and a heavy chain FR3 comprising the amino acid sequence of SEQ ID NO: 14.
29. The antibody according to embodiment 28, wherein the antibody comprises a heavy chain variable region comprising the amino acid sequence of SEQ ID NO: 7 and a light chain variable region comprising the amino acid sequence of SEQ ID NO: 8.
30. The antibody according to embodiment 29, wherein the antibody comprises a light chain comprising the amino acid sequence of SEQ ID NO: 9.
31. An antibody, comprising a heavy chain variable region comprising the amino acid sequence of SEQ ID NO: 1 and a light chain variable region comprising the amino acid sequence of SEQ ID NO: 2, wherein the antibody specifically binds to human IL-15, wherein the IL-15 is complexed with IL-15 Receptor alpha (IL-15Rα).
32. The antibody according to embodiment 31, wherein the antibody comprises
   (a) a heavy chain variable region comprising the amino acid sequence of SEQ ID NO: 4 and a light chain variable region comprising the amino acid sequence of SEQ ID NO: 5;
   (b) a heavy chain variable region comprising the amino acid sequence of SEQ ID NO: 454 and a light chain variable region comprising the amino acid sequence of SEQ ID NO: 8;
   (c) a heavy chain variable region comprising the amino acid sequence of SEQ ID NO: 4 and a light chain variable region comprising the amino acid sequence of SEQ ID NO: 455;
   (d) a heavy chain variable region comprising the amino acid sequence of SEQ ID NO: 4 and a light chain variable region comprising the amino acid sequence of SEQ ID NO: 457; or
   (e) a heavy chain variable region comprising the amino acid sequence of SEQ ID NO: 4 and a light chain variable region comprising the amino acid sequence of SEQ ID NO: 459.
33. An antibody that specifically binds to human IL-15, wherein the IL-15 is complexed with IL-15 Receptor alpha (IL-15Rα) wherein the antibody comprises
   (a) a heavy chain variable region comprising the amino acid sequence of SEQ ID NO:4 and a light chain variable region comprising the amino acid sequence of SEQ ID NO:8;
   (b) a heavy chain variable region comprising the amino acid sequence of SEQ ID NO:4 and a light chain variable region comprising the amino acid sequence of SEQ ID NO:503;
   (c) a heavy chain variable region comprising the amino acid sequence of SEQ ID NO:4 and a light chain variable region comprising the amino acid sequence of SEQ ID NO:505;
   (d) a heavy chain variable region comprising the amino acid sequence of SEQ ID NO:4 and a light chain variable region comprising the amino acid sequence of SEQ ID NO:507;
   (e) a heavy chain variable region comprising the amino acid sequence of SEQ ID NO:4 and a light chain variable region comprising the amino acid sequence of SEQ ID NO:509;
   (f) a heavy chain variable region comprising the amino acid sequence of SEQ ID NO:4 and a light chain variable region comprising the amino acid sequence of SEQ ID NO:510;
   (g) a heavy chain variable region comprising the amino acid sequence of SEQ ID NO:454 and a light chain variable region comprising the amino acid sequence of SEQ ID NO:455;
   (h) a heavy chain variable region comprising the amino acid sequence of SEQ ID NO:454 and a light chain variable region comprising the amino acid sequence of SEQ ID NO:503;
   (i) a heavy chain variable region comprising the amino acid sequence of SEQ ID NO:454 and a light chain variable region comprising the amino acid sequence of SEQ ID NO:457;
   (j) a heavy chain variable region comprising the amino acid sequence of SEQ ID NO:454 and a light chain variable region comprising the amino acid sequence of SEQ ID NO:505;
   (k) a heavy chain variable region comprising the amino acid sequence of SEQ ID NO:454 and a light chain variable region comprising the amino acid sequence of SEQ ID NO:506;
   (l) a heavy chain variable region comprising the amino acid sequence of SEQ ID NO:454 and a light chain variable region comprising the amino acid sequence of SEQ ID NO:507;
   (m) a heavy chain variable region comprising the amino acid sequence of SEQ ID NO:454 and a light chain variable region comprising the amino acid sequence of SEQ ID NO:5;
   (n) a heavy chain variable region comprising the amino acid sequence of SEQ ID NO:454 and a light chain variable region comprising the amino acid sequence of SEQ ID NO:509; or
   (o) a heavy chain variable region comprising the amino acid sequence of SEQ ID NO:454 and a light chain variable region comprising the amino acid sequence of SEQ ID NO:510.
34. The antibody according to any one of embodiments 1 to 33, wherein the antibody comprises a IgG constant domain.
35. The antibody according to any one of embodiments 1 to 34, wherein the antibody comprises a IgG4 constant domain.
36. The antibody according to embodiment 35, wherein the IgG4 constant domain comprises an amino acid sequence selected from the group consisting of SEQ ID NO: 49, SEQ ID NO: 44, SEQ ID NO: 45, SEQ ID NO: 46, SEQ ID NO: 47, SEQ ID NO: 48, SEQ ID NO: 50 and SEQ ID NO: 51.
37. The antibody according to any one of embodiments 1 to 34, wherein the antibody comprises a IgG1 constant domain.
38. The antibody according to embodiment 37, wherein the IgG1 constant domain comprises an amino acid sequence selected from the group consisting of SEQ ID NO: 32, SEQ ID NO: 33, SEQ ID NO: 34, SEQ ID NO: 35, SEQ ID NO: 36, SEQ ID NO: 37, SEQ ID NO: 38 and SEQ ID NO: 39.
39. The antibody according to any one of embodiments 1 to 34, wherein the antibody comprises a IgG2 constant domain.
40. The antibody according to embodiment 39, wherein the IgG2 constant domain comprises an amino acid sequence selected from the group consisting of SEQ ID NO: 40, SEQ ID NO: 41, SEQ ID NO: 42 and SEQ ID NO: 43.
41. The antibody according to any one of embodiments 1 to 40, wherein the antibody inhibits proliferation of Natural Killer (NK) cells at an IC₅₀ of from about 0.1 pM to about about 900 pM in an NK cell proliferation assay.
42. The antibody according to any one of embodiments 1 to 41, wherein the antibody inhibits proliferation of NK cells at an IC₅₀ of from about 1 pM to about 60 pM in an NK cell proliferation assay.
43. The antibody according to any one of embodiments 1 to 42, wherein the antibody inhibits proliferation of NK cells at an IC₅₀ of from about 5 pM to about 35 pM in an NK cell proliferation assay.
44. The antibody according to any one of embodiments 1 to 43, wherein the antibody is capable of neutralizing IL-15.
45. The antibody according to any one of embodiments 1 to 44, wherein the antibody is capable of decreasing circulating NK cells.
46. The antibody according to any one of embodiments 5 to 45, wherein the antibody binds to an epitope comprising the Q108 residue of human IL-15.
47. The antibody according to embodiment 46, wherein the epitope further comprises the S7 and N112 residues of human IL-15.
48. The antibody according to any one of embodiments 5 to 46, wherein the antibody has an affinity for human IL-15 that includes a dissociation constant (KD) of less than about 1.8 x 10⁻⁹ M as determined by surface plasmon resonance or wherein the dissociation constant (KD) is less than 1 x 10⁻⁹ M.
49. An antibody that binds to IL-15 wherein the IL-15 is complexed with IL-15 Receptor alpha (IL-15Rα)comprising a heavy chain variable region and a light chain variable region, wherein the heavy chain variable region comprises the amino acid sequence of SEQ ID NO: 4.
50. An antibody that binds to IL-15 wherein the IL-15 is complexed with IL-15 Receptor alpha (IL-15Rα) comprising a heavy chain variable region and a light chain variable region, wherein the light chain variable region comprises the amino acid sequence of SEQ ID NO: 5.
51. The antibody of embodiment 50 or 51, wherein the heavy chain variable region comprises the amino acid sequence of SEQ ID NO: 4 and the light chain variable region comprises the amino acid sequence of SEQ ID NO: 5.
52. The antibody of any one of embodiments 49-51, wherein the antibody comprises a light chain comprising the amino acid sequence of SEQ ID NO:6.
53. The antibody of any one of embodiments 49-51, further comprising heavy chain constant region comprising the amino acid sequence of SEQ ID NO:49.
54. The antibody of any one of embodiments 49-51, further comprising heavy chain constant region comprising the amino acid sequence of SEQ ID NO:49, wherein the antibody comprises a light chain comprising the amino acid sequence of SEQ ID NO:6.
55. A composition, comprising the antibody according to any one of embodiments 1 to 54 and a pharmaceutically acceptable carrier or excipient.
56. A method of treating Celiac disease in a subject in need thereof, comprising administering to the subject an antibody that binds to IL-15, wherein the IL-15 is complexed with IL-15 Receptor alpha (IL-15Rα).
57. A method of treating Celiac disease in a subject in need thereof, comprising administering to the subject the antibody according to any one of embodiments 1 to 54.
58. A method for repairing the mucosa of a small intestine in a subject having gluten sensitivity, gluten allergy, or Celiac disease comprising administering to the subject the antibody according to any one of embodiments 1 to 54.
59. A method for increasing the mean villous height vs. crypt depths (V/C) ratio in a subject having gluten sensitivity, gluten allergy, or Celiac disease comprising administering to the subject the antibody according to any one of embodiments 1 to 54.
60. A method for increasing the height of small intestinal villi in a subject having gluten sensitivity, gluten allergy, or Celiac disease comprising administering to the subject the antibody according to any one of embodiments 1 to 54.
61. A method for decreasing anti-gliadin antibodies in a subject in need thereof comprising administering to the subject the antibody according to any one of embodiments 1 to 54.
62. A method for repairing gluten-induced small intestinal mucosal injury comprising administering to the subject the antibody according to any one of embodiments 1 to 54.
63. The method according to embodiment 61 or 62, wherein the subject has gluten sensitivity, a gluten allergy, or Celiac disease.
64. The method according to any one of embodiments 56 to 59 or 63, wherein the Celiac disease is refractory.
65. A method for inhibiting one or more symptoms of gluten exposure in a subject having a gluten sensitivity or gluten allergy and in need thereof, comprising administering to the subject the antibody according to any one of embodiments 1 to 54.
66. The method according to embodiment 65, wherein the one or more symptoms include one or more of muscle pain, body pain, joint pain, fatigue, bloating, gas, nausea, cramps, constipation, diarrhea, skin rash, headache, migraine headache, depression, anxiety, brain fog, and irritability.
67. The method according to any one of embodiments 56-66, wherein the subject consumes a gluten-containing diet.
68. A method of treating rheumatoid arthritis in a subject in need thereof, comprising administering to the subject the antibody according to any one of embodiments 1 to 54.
69. A method of treating psoriasis in a subject in need thereof, comprising administering to the subject the antibody according to any one of embodiments 1 to 54.
70. A method of treating inflammatory bowel disease in a subject in need thereof, comprising administering to the subject the antibody according to any one of embodiments 1 to 54.
71. A method of treating type 1 diabetes in a subject in need thereof, comprising administering to the subject the antibody according to any one of embodiments 1 to 54.
72. A method of treating alopecia areata in a subject in need thereof, comprising administering to the subject the antibody according to any one of embodiments 1 to 54.
73. A method of treating T cell large granular lymphocytic leukemia in a subject in need thereof, comprising administering to the subject the antibody according to any one of embodiments 1 to 54.
74. A method of treating an autoimmune disease in a subject in need thereof, comprising administering to the subject the antibody according to any one of embodiments 1 to 54.
75. The method according to embodiment 74, wherein the autoimmune disease is an autoimmune disease in which IL-15 is dysregulated.
76. A method of treating or inhibiting an inflammatory disease or inflammatory condition in a subject in need thereof, comprising administering to the subject the antibody according to any one of embodiments 1 to 54.
77. The method according to embodiment 76, wherein the inflammatory disease or inflammatory condition is an inflammatory disease or inflammatory condition in which IL-15 is dysregulated.
78. The method according to any one of embodiments 56 to 77, wherein the antibody is in a composition comprising a pharmaceutically acceptable carrier or excipient.
79. The antibody according to any one of embodiments 1 to 54 for use in treating Celiac disease, refractory Celiac disease, rheumatoid arthritis, psoriasis, inflammatory bowel disease, type 1 diabetes, alopecia areata, T cell large granular lymphocytic leukemia, an autoimmune disease, an autoimmune disease in which IL-15 is dysregulated, an inflammatory disease or inflammatory condition, or inflammatory disease or inflammatory condition in which IL-15 is dysregulated.
80. The antibody according to any one of embodiments 1 to 54 for use in treating or inhibiting one or more symptoms of gluten exposure.
81. Use of the antibody according to any one of embodiments 1 to 54 for the manufacture of a medicament for treating Celiac disease, refractory Celiac disease, rheumatoid arthritis, psoriasis, inflammatory bowel disease, type 1 diabetes, alopecia areata, T cell large granular lymphocytic leukemia, an autoimmune disease, an autoimmune disease in which IL-15 is dysregulated, an inflammatory disease or inflammatory condition, or an inflammatory disease or inflammatory condition in which IL-15 is dysregulated.
82. Use of the antibody according to any one of embodiments 1 to 54 for the manufacture of a medicament for treating or inhibiting symptoms of gluten exposure.
83. The use according to embodiment 82, wherein the symptoms include one or more of muscle pain, body pain, joint pain, fatigue, bloating, gas, nausea, cramps, constipation, diarrhea, skin rash, headache, migraine headache, depression, anxiety, brain fog, and irritability.
84. An *in vitro* method for detecting IL-15 in a tissue sample isolated from a subject, comprising contacting the antibody according to any one of embodiments 1 to 54 with a tissue sample isolated from a subject to form an antibody-IL-15 complexed with the IL-15Receptor-alpha, and detecting the complex in the tissue sample.
85. An *in vitro* method for detecting a complex of IL-15 and IL-15 receptor alpha in a tissue sample isolated from a subject, comprising contacting the antibody according to any one of embodiments 1-54 with a tissue sample isolated from a subject to form an antibody-antigen complex of the antibody with IL-15 and IL-15 receptor alpha, and detecting the antibody-antigen complex in the tissue sample.
86. A transformed cell that expresses the antibody according to any one of embodiments 1 to 54.
87. The transformed cell according to embodiment 86, wherein the cell is a mammalian cell.
88. The transformed cell according to embodiment 87, wherein the mammalian cell is a Chinese Hamster Ovary cell.
89. A method of producing the antibody of any one of embodiments 1-54 comprising culturing the cell of any one of embodiments 86-88.
90. A polynucleotide comprising a nucleic acid sequence encoding an antibody variable heavy chain comprising the amino acid sequence of SEQ ID NO: 1.
91. The polynucleotide according to embodiment 90, wherein the nucleic acid sequence encodes an antibody variable heavy chain comprising the amino acid sequence of SEQ ID NO: 4.
92. The polynucleotide according to embodiment 90 or 91, wherein the polynucleotide further comprises a second nucleic acid sequence encoding an antibody light chain variable region comprising the amino acid sequence of SEQ ID NO: 2.
93. The polynucleotide according to embodiment 92, wherein the second nucleic acid sequence encodes an antibody variable light chain comprising the amino acid sequence of SEQ ID NO: 5.
94. A polynucleotide comprising a nucleic acid sequence encoding an antibody variable light chain comprising the amino acid sequence of SEQ ID NO: 2.
95. A polynucleotide comprising a nucleic acid sequence encoding an antibody variable light chain comprising the amino acid sequence of SEQ ID NO: 5.
96. A polynucleotide, comprising a nucleic acid sequence encoding the antibody according to any one of embodiments 5 to 54.
97. A polynucleotide, comprising the nucleic acid sequence of SEQ ID NO: 517.
98. A polynucleotide, comprising the nucleic acid sequence of SEQ ID NO: 518.
99. A vector comprising the polynucleotide according to any one of embodiments 90 to 98.
100. A cell transfected with the vector according to embodiment 99.
101. A cell comprising a polynucleotide comprising a nucleic acid that encodes the variable heavy chain of antibody of any one of embodiments 1-54 and a polynucleotide comprising a nucleic acid that encodes the variable light chain of the antibody.
102. The cell of embodiment 101, wherein the nucleic acid that encodes the variable heavy chain and the nucleic acid that encodes the variable light chain are on the same vector.
103. The cell of embodiment 102, wherein the nucleic acid that encodes the variable heavy chain and the nucleic acid that encodes the variable light chain are different vectors.
104. The cell according to any one of embodiments 100-103, wherein the cell is a mammalian protein expression cell.

## Claims

1. An antibody, comprising an HCDR1 comprising the amino acid sequence of SEQ ID NO: 16, an HCDR2 comprising the amino acid sequence of SEQ ID NO: 17, an HCDR3 comprising the amino acid sequence of SEQ ID NO: 20, an LCDR1 comprising the amino acid sequence of SEQ ID NO: 25, an LCDR2 comprising the amino acid sequence of SEQ ID NO: 28, and an LCDR3 comprising the amino acid sequence of SEQ ID NO: 29, wherein the antibody specifically binds to human IL-15.

2. The antibody according to claim 1, wherein the antibody comprises an HCDR2 comprising the amino acid sequence of SEQ ID NO: 18.

3. The antibody according to claim 1 or 2 wherein the antibody comprises:
(a) an LCDR1 comprising the amino acid sequence of SEQ ID NO: 26 and an LCDR3 comprising the amino acid sequence of SEQ ID NO: 30, optionally wherein the antibody comprises a heavy chain variable region comprising the amino acid sequence of SEQ ID NO: 4 and optionally wherein the antibody comprises a light chain variable region comprising the amino acid sequence of SEQ ID NO: 5 and/or optionally wherein the antibody comprises a light chain comprising the amino acid sequence of SEQ ID NO: 6;
(b) an LCDR1 comprising the amino acid sequence of SEQ ID NO: 27 and an LCDR3 comprising the amino acid sequence of SEQ ID NO: 31, optionally wherein the antibody comprises a heavy chain variable region comprising the amino acid sequence of SEQ ID NO: 454 and a light chain variable region comprising the amino acid sequence of SEQ ID NO: 8 and/or optionally wherein the antibody comprises a light chain comprising the amino acid sequence of SEQ ID NO: 9;
(c) an LCDR1 comprising the amino acid sequence of SEQ ID NO: 26 and an LCDR3 comprising the amino acid sequence of SEQ ID NO: 31, optionally wherein the antibody comprises a heavy chain variable region comprising the amino acid sequence of SEQ ID NO: 4 and a light chain variable region comprising the amino acid sequence of SEQ ID NO: 455 and/or optionally wherein the antibody comprises a light chain comprising the amino acid sequence of SEQ ID NO: 456;
(d) an LCDR1 comprising the amino acid sequence of SEQ ID NO: 27 and an LCDR3 comprising the amino acid sequence of SEQ ID NO: 30, optionally wherein the antibody comprises a heavy chain variable region comprising the amino acid sequence of SEQ ID NO: 4 and a light chain variable region comprising the amino acid sequence of SEQ ID NO: 457 and/or optionally wherein the antibody comprises a light chain comprising the amino acid sequence of SEQ ID NO: 458; or
(e) an LCDR1 comprising the amino acid sequence of SEQ ID NO: 27, and an LCDR3 comprising the amino acid sequence of SEQ ID NO: 519, optionally wherein the antibody comprises a heavy chain variable region comprising the amino acid sequence of SEQ ID NO: 4 and a light chain variable region comprising the amino acid sequence of SEQ ID NO: 459, and optionally wherein the antibody comprises a light chain comprising the amino acid sequence of SEQ ID NO: 460.

4. The antibody according to claim 1, wherein the antibody comprises an HCDR2 comprising the amino acid sequence of SEQ ID NO: 19, an LCDR1 comprising the amino acid sequence of SEQ ID NO: 27, an LCDR3 comprising the amino acid sequence of SEQ ID NO: 31, and a heavy chain FR3 comprising the amino acid sequence of SEQ ID NO: 14.

5. The antibody according to claim 4, wherein the antibody comprises a heavy chain variable region comprising the amino acid sequence of SEQ ID NO: 7 and a light chain variable region comprising the amino acid sequence of SEQ ID NO: 8, optionally wherein the antibody comprises a light chain comprising the amino acid sequence of SEQ ID NO: 9.

6. An antibody, comprising a heavy chain variable region comprising the amino acid sequence of SEQ ID NO: 1 and a light chain variable region comprising the amino acid sequence of SEQ ID NO: 2, wherein the antibody specifically binds to human IL-15, optionally wherein the antibody comprises
(a) a heavy chain variable region comprising the amino acid sequence of SEQ ID NO: 4 and a light chain variable region comprising the amino acid sequence of SEQ ID NO: 5;
(b) a heavy chain variable region comprising the amino acid sequence of SEQ ID NO: 454 and a light chain variable region comprising the amino acid sequence of SEQ ID NO: 8;
(c) a heavy chain variable region comprising the amino acid sequence of SEQ ID NO: 4 and a light chain variable region comprising the amino acid sequence of SEQ ID NO: 455;
(d) a heavy chain variable region comprising the amino acid sequence of SEQ ID NO: 4 and a light chain variable region comprising the amino acid sequence of SEQ ID NO: 457; or
(e) a heavy chain variable region comprising the amino acid sequence of SEQ ID NO: 4 and a light chain variable region comprising the amino acid sequence of SEQ ID NO: 459.

7. An antibody that specifically binds to human IL-15, wherein the antibody comprises
(a) a heavy chain variable region comprising the amino acid sequence of SEQ ID NO:4 and a light chain variable region comprising the amino acid sequence of SEQ ID NO:8;
(b) a heavy chain variable region comprising the amino acid sequence of SEQ ID NO:4 and a light chain variable region comprising the amino acid sequence of SEQ ID NO:503;
(c) a heavy chain variable region comprising the amino acid sequence of SEQ ID NO:4 and a light chain variable region comprising the amino acid sequence of SEQ ID NO:505;
(d) a heavy chain variable region comprising the amino acid sequence of SEQ ID NO:4 and a light chain variable region comprising the amino acid sequence of SEQ ID NO:507;
(e) a heavy chain variable region comprising the amino acid sequence of SEQ ID NO:4 and a light chain variable region comprising the amino acid sequence of SEQ ID NO:509;
(f) a heavy chain variable region comprising the amino acid sequence of SEQ ID NO:4 and a light chain variable region comprising the amino acid sequence of SEQ ID NO:510;
(g) a heavy chain variable region comprising the amino acid sequence of SEQ ID NO:454 and a light chain variable region comprising the amino acid sequence of SEQ ID NO:455;
(h) a heavy chain variable region comprising the amino acid sequence of SEQ ID NO:454 and a light chain variable region comprising the amino acid sequence of SEQ ID NO:503;
(i) a heavy chain variable region comprising the amino acid sequence of SEQ ID NO:454 and a light chain variable region comprising the amino acid sequence of SEQ ID NO:457;
(j) a heavy chain variable region comprising the amino acid sequence of SEQ ID NO:454 and a light chain variable region comprising the amino acid sequence of SEQ ID NO:505;
(k) a heavy chain variable region comprising the amino acid sequence of SEQ ID NO:454 and a light chain variable region comprising the amino acid sequence of SEQ ID NO:506;
(l) a heavy chain variable region comprising the amino acid sequence of SEQ ID NO:454 and a light chain variable region comprising the amino acid sequence of SEQ ID NO:507;
(m) a heavy chain variable region comprising the amino acid sequence of SEQ ID NO:454 and a light chain variable region comprising the amino acid sequence of SEQ ID NO:5;
(n) a heavy chain variable region comprising the amino acid sequence of SEQ ID NO:454 and a light chain variable region comprising the amino acid sequence of SEQ ID NO:509; or
(o) a heavy chain variable region comprising the amino acid sequence of SEQ ID NO:454 and a light chain variable region comprising the amino acid sequence of SEQ ID NO:510.

8. The antibody according to any one of claims 1 to 7, wherein the antibody comprises an IgG constant domain, optionally wherein the antibody comprises:
(a) an IgG4 constant domain, optionally wherein the IgG4 constant domain comprises an amino acid sequence selected from the group consisting of SEQ ID NO: 49, SEQ ID NO: 44, SEQ ID NO: 45, SEQ ID NO: 46, SEQ ID NO: 47, SEQ ID NO: 48, SEQ ID NO: 50 and SEQ ID NO: 51;
(b) an IgG1 constant domain, optionally wherein the IgG1 constant domain comprises an amino acid sequence selected from the group consisting of SEQ ID NO: 32, SEQ ID NO: 33, SEQ ID NO: 34, SEQ ID NO: 35, SEQ ID NO: 36, SEQ ID NO: 37, SEQ ID NO: 38 and SEQ ID NO: 39; or
(c) an IgG2 constant domain, optionally wherein the IgG2 constant domain comprises an amino acid sequence selected from the group consisting of SEQ ID NO: 40, SEQ ID NO: 41, SEQ ID NO: 42 and SEQ ID NO: 43.

9. The antibody according to any one of claims 1 to 8, wherein:
(i) the antibody inhibits proliferation of Natural Killer (NK) cells at an IC₅₀ of from about 0.1 pM to about about 900 pM in an NK cell proliferation assay, optionally
wherein the antibody inhibits proliferation of NK cells at an IC₅₀ of from about 1 pM to about 60 pM in an NK cell proliferation assay, and/or
wherein the antibody inhibits proliferation of NK cells at an IC₅₀ of from about 5 pM to about 35 pM in an NK cell proliferation assay; and/or
(ii) the antibody is capable of neutralizing IL-15; and/or
(iii) the antibody is capable of decreasing circulating NK cells.

10. The antibody according to any one of claims 1 to 9, wherein the antibody binds to an epitope comprising the Q108 residue of human IL-15, optionally wherein the epitope further comprises the S7 and N112 residues of human IL-15.

11. The antibody according to any one of claims 1 to 10, wherein the antibody has an affinity for human IL-15 that includes a dissociation constant (KD) of less than about 1.8 x 10⁻⁹ M as determined by surface plasmon resonance or wherein the dissociation constant (KD) is less than 1 x 10⁻⁹ M.

12. An antibody that binds to IL-15 wherein the IL-15 is complexed with IL-15 Receptor alpha (IL-15Rα)comprising a heavy chain variable region and a light chain variable region, wherein the heavy chain variable region comprises the amino acid sequence of SEQ ID NO: 4 and/or wherein the light chain variable region comprises the amino acid sequence of SEQ ID NO: 5.

13. The antibody of claim 12, wherein the antibody:
(i) comprises a light chain comprising the amino acid sequence of SEQ ID NO:6; or
(ii) further comprises a heavy chain constant region comprising the amino acid sequence of SEQ ID NO:49; or
(iii) further comprises a heavy chain constant region comprising the amino acid sequence of SEQ ID NO:49, and wherein the antibody comprises a light chain comprising the amino acid sequence of SEQ ID NO:6.

14. A composition, comprising the antibody according to any one of claims 1 to 13 and a pharmaceutically acceptable carrier or excipient.

15. The antibody according to any one of claims 1 to 13 for use in treating Celiac disease, refractory Celiac disease, rheumatoid arthritis, psoriasis, inflammatory bowel disease, type 1 diabetes, alopecia areata, T cell large granular lymphocytic leukemia, an autoimmune disease, an autoimmune disease in which IL-15 is dysregulated, an inflammatory disease or inflammatory condition, or an inflammatory disease or inflammatory condition in which IL-15 is dysregulated.
